(19) Europäisches Patentamt
European Patent Office
Office européen des brevets

(11) **EP 4 050 329 A1**

(12) **EUROPEAN PATENT APPLICATION**
published in accordance with Art. 153(4) EPC

(43) Date of publication:
**31.08.2022 Bulletin 2022/35**

(21) Application number: **21890366.4**

(22) Date of filing: **09.11.2021**

(51) International Patent Classification (IPC):
$G01N\ 25/00$ (2006.01)     $G01N\ 3/18$ (2006.01)
$G01N\ 3/32$ (2006.01)     $G01N\ 3/54$ (2006.01)
$G06F\ 30/20$ (2020.01)     $G06F\ 119/04$ (2020.01)

(86) International application number:
**PCT/CN2021/129423**

(87) International publication number:
**WO 2022/142742 (07.07.2022 Gazette 2022/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.12.2020 CN 202011645694**

(71) Applicants:
• **Shenzhen Goldlink Tongda Electronics Co., Ltd.**
**Shenzhen**
**Guangdong 518101 (CN)**

• **Dongguan Goldlink Tongda Thermal Conductive Materials MFG Co., Ltd.**
**Dalingshan Town**
**Dongguan**
**Guangdong 532820 (CN)**

(72) Inventor: **SHORE, Yorway**
**Shenzhen, Guangdong 518101 (CN)**

(74) Representative: **Sun, Yiming**
**HUASUN Patent- und Rechtsanwälte**
**Friedrichstraße 33**
**80801 München (DE)**

(54) **NEW ENERGY THERMAL MANAGEMENT COMPOSITE MATERIAL AGING LIFE TEST METHOD AND ALGORITHM, AND USE**

(57) Disclosed in the present invention are a test method and algorithm for an aging life of a composite, and a use thereof. The test method and algorithm includes: respectively placing specimens in four temperature environments to undergo damp and hot, high and low temperature impact and high and low temperature alternating cycle for a specified time; testing the physical, chemical and electrical properties of the specimens by using laminated combined test pieces; fitting parameters in a micro-gasification expansion oscillation equation; fitting constants in a kinetic correlation equation (2) of the parameters; calculating new values of the parameters in any temperature environment by using the constant equation (2); and substituting the new values of the parameters back into the equation (1), so as to evaluate or predict the physical, chemical and electrical properties of the specimens at any time. The highest aging test temperature can reach 298°C, not only the limitations of the GB/T 20028, ASTM G 166, ASTM G 169, ISO 2578 and UL 746B standards are broken through, but a linear correlation coefficient $R^2$ is also two "9" accuracy levels higher, so that the prediction is more accurate.

Fig. 10

EP 4 050 329 A1

**Description**

**TECHNICAL FIELD**

The present invention belongs to the field of test methods and algorithms for predicting long-term aging lives of polymer matrix composites, and proposes a method suitable for evaluating and predicting the long-term reliability, safety and environmental adaptability of polymer matrix composites under actual service working conditions. Specific application cases involve the evaluation or prediction of service lives of insulating and heat conducting materials applied to heat management interfaces of new energy power battery packs and 5G/6G equipment, interfaces of chips and heat sinks, and interfaces of heat sources and cold sinks.

**BACKGROUND ART**

In terms of the rise of new energy vehicle industry and 5G/6G technology, the whole world is at the same starting line. The same is true for the indispensable technology of insulating and heat conducting materials of heat management interfaces that matches these industrial power battery packs. Although there are already many varieties of interface insulating and heat conducting materials at home and abroad, in the entire industry chain of design, production and application of the interface insulating and heat conducting materials, no corresponding service life evaluation or prediction standard method has been established.

At present, including the academia, complete machine plants of new energy vehicles, 5G/6G complete machine plants, and research institutions and manufacturers of the interface insulating and heat conducting materials, reliability evaluation methods used for the time being are all borrowed from the IEC 60068-2 series of standards. The Chinese translation of this series of standard equivalents (IDT) is GB/T 2423. Taking the IEC 60068-2 standard as an "emergency plan", although it can pass the routine test of three aging conditions of "damp and hot, high and low temperature impact, and high and low temperature alternating cycle", it does not solve the difficulty in evaluating or predicting the long-term service lives of the interface insulating and heat conducting materials under actual service working conditions.

If a scientific evaluation standard method system for the reliability, safety and environmental adaptability of interface heat conducting materials is not established, there will be a worldwide long-term hidden danger.

In fact, the IEC 60068-2 standard is only applicable to the evaluation of short-term aging performance of small electrical and electronic components, and is not applicable to the evaluation or prediction of long-term reliability, safety and environmental adaptability of insulating and heat conducting materials of heat management interfaces of new energy power battery packs and 5G/6G equipment. This is because, among the electrical products involved in IEC 60068-2, the life targets of the electrical and electronic components are generally designed to be (8-10) years, while the design lives of fast consumer electronic component products, such as mobile phones and computers, are generally less than 8 years, so it is permissible and sufficient to evaluate the aging trend within (8-10) years according to the IEC 60068-2 series of standards.

However, the interface insulating and heat conducting materials belong to polymer matrix composites, not only their aging behaviors are essentially different from those of the electrical and electronic components, but also the design requirements and the service life targets have also changed. Take a new energy power battery pack as an example:

It is of a CMP structure in the past: battery cells are assembled into battery modules by mechanical fastening parts, and then the modules are integrated into a battery pack by using the mechanical fastening parts, that is, the so-called Cell to Model to Pack structure;

now and in the future, it is of a CTP structure: the battery cells are directly bonded into a complete battery pack in one step by using heat-conducting structural adhesive, that is, the so-called Cell to Pack structure. This path can reduce the number of mechanical parts of the battery pack by about 40%, increase the volume utilization rate by (15-20)%, increase the cruising range per unit volume by (15-17)%, increase the manufacturing efficiency by nearly 50%, and can greatly reduce the manufacturing cost;

in addition, more importantly, the power battery packs of new energy vehicles require that the reliability, safety and environmental adaptability of the interface insulating and heat conducting materials need to be reflected in six specific targets:

1) functional mission: high strength, high toughness, high thermal conductivity and high insulation, for example, among a battery cell, a water cooling plate and a heating belt, the interface insulating and heat conducting material can replace a metal fastening member to the greatest extent, and is directly bonded and sealed to form a CTP battery pack;

2) service life: the service life in a 45°C environment is more than 50 years, including 25 years of road operation and 25 years of energy storage operation;

3) service temperature: -45°C to 60°C alternating cycle, continuous normal service for 50 years or more;

4) disaster impact: under the conditions of 12 m free fall and 45° inclined driving superimposed impact, it is ensured that positive and negative electrodes of the battery cell generate no short circuit or detonation;

5) flame retardancy: it will extinguish after leaving the fire, and the flame retardancy is higher than the most stringent V0 standard of UL 94; and

6) withstand voltage: when an adhesive layer is as thin as 0.28 mm, it is not broken down by a voltage of 2500V.

Obviously, if the IEC 60068-2 series of standards are stilled borrowed, the states of physical, chemical and electrical properties of the interface insulating and heat conducting material after 50 years cannot be given in advance within a very short period of time.

In addition, although there are recognized framework standards and cases for predicting half-life periods of the polymer matrix composites under a high-temperature accelerated aging condition in the academic circles, such as GB/T 20028, ASTM G 166, ASTM G 169, ISO 2578, and UL 746B, for the applications of specific molded products in engineering, due to the many installation structural factors involved in the engineering environments, the aging factors will be far more complicated than pure materials under laboratory test conditions. These framework standards cannot directly provide a method for predicting the service lives of molding materials in specific application states. It was once found in a research project of predicting the service life of a certain type of composite solid propellant [1] that, the currently recognized serious defect of the framework standards of predicting the long-term aging lives of the polymer matrix composites is: the Arrhenius Equation using the activation energy of a single substance is used for prediction, which has a higher linear correlation coefficient $R^2$ for the prediction of the aging trends of higher purity, single-component materials and single crystal phase regions, but for the prediction of the aging trends of composites composed of multiple components in complex installation structure environments, the linear correlation coefficient $R^2$ is lower, and the prediction deviation will exceed the allowable boundary.

Therefore, there has been no report on an evaluation test method and algorithms for evaluating the long-term aging trend and reliability of an interface insulating and heat conducting material with persistent and constant compression set, in the service environment of a "double-sided metal plate sandwich biscuit structure". Furthermore, countries at home and abroad are on the same starting line, and there is no direct test method, algorithm or conclusion that can be cited.

Therefore, a test method and algorithm for long-term aging service lives of polymer matrix composites, especially interface insulating and heat conducting materials, is a worldwide technical issue that needs to be resolved in advance for the long-term safety of new energy power battery packs, and it is also a difficult problem.

Therefore, it is necessary to invent a test method and algorithm for aging lives of polymer matrix composites, especially interface insulating and heat conducting materials.

References

[1] YORWAY, Shore; Migration and volatilization of tert-butyl ferrocene and its effect on burning rate, Chinese Astronautical Society (North Sea Fleet Command) Conference Paper, September 1984; "Propulsion Technology" 1985, 6 (2): 49-60.

## SUMMARY OF THE INVENTION

The purpose of the present invention is to provide a test method and algorithm for aging lives of polymer matrix composites, especially interface insulating and heat conducting materials, and an application thereof, so as to solve the technical problem of evaluating or predicting 50-year aging trends of physical, chemical and electrical properties of power battery packs of new energy vehicles.

In order to achieve one of the above objectives, a test method and algorithm for an aging life of a new energy heat management composite provided by the present invention includes: preparing a target specimen into any one or a combined specimen of any two of an open specimen, a closed specimen and a fixture compression specimen, so as to serve as a standard specimen for an aging life test; respectively placing the standard specimens in at least four specified constant temperature environments, and making the standard specimens respectively undergo at least one condition of damp and hot, high and low temperature impact, and high and low temperature alternating cycle for a specified time or an accumulative number of cycles in each temperature environment; testing the physical, chemical and electrical properties of the target specimen by using the standard specimens or laminated combined test pieces; fitting fifteen parameters in a micro-gasification expansion oscillation equation (1) by using measured values of the physical, chemical and electrical properties; fitting three constants in a kinetic correlation equation (2) of the fifteen parameters; substituting the fitted constants back into the kinetic correlation equation (2) one by one, so as to calculate new values of the fifteen parameters in any specified constant temperature environment; and substituting the new values of the fifteen parameters back into the equation (1), so as to evaluate or predict the physical, chemical and electrical properties of the target specimen at any specified time under the at least one condition of damp and hot, high and low temperature impact and high and low temperature alternating cycle for the specified time or the accumulative number of cycles.

In order to achieve the second objective described above, a use of the test method and algorithm for the aging life of the new energy heat management composite provided by the present invention includes: by using the test method and algorithm for the aging life, evaluating or predicting the physical, chemical and electrical properties of the target specimen in any specified constant temperature environment for the specified time or the accumulative number of cycles, or evaluating or predicting a half-life period of any one of the physical, chemical and electrical properties in the specified constant temperature environment, or evaluating or predicting a rated temperature of any one of the physical, chemical and electrical properties at a specified service time of 20,000 hours; wherein the physical, chemical and electrical properties further include at least one of color, density, thermal conductivity, oil separation rate, compression set rate, specific heat, hardness, tensile strength, elongation at break, butt joint tension bonding strength, lap joint shear bonding strength, glass transition temperature, linear expansion coefficient, breakdown strength, DC or AC electric leakage resistance, volume resistivity, dielectric constant, loss factor, oxygen index, flame retardancy, vacuum volatiles, hydroscopicity, mold resistance, fumes density, fumes index, and toxicity index of burned gas.

Further, the composite includes any one of solid, fluid and melt of a polymer matrix composite, or a mixture of any two states of solid, fluid and melt, or any one or a compound of rubber, plastic, fibers and thermosetting materials, or any one or a compound of elastomers, adhesives, sealants and foam materials.

Further, the target specimen includes: the composite material is made into a specimen that conforms to a shape specified by corresponding test standards for physical, chemical and electrical properties.

Further, the open specimen includes: the target specimen is not coated, wrapped, clamped or closed by using materials, wraps or containers that are different from the chemical components of the target specimen, but the target specimen is exposed to an aging environment.

Further, the closed specimen includes: a part or all of the superficial area of the target specimen is isolated from the aging environment by using materials, wraps or containers that are different from the chemical components of the target specimen, in any manner of coating, wrapping, clamping or closing.

Further, the fixture compression specimen includes: the target specimen is clamped into a "sandwich biscuit" structure by using at least two rigid plates, and the distance between the two rigid plates is adjusted to a specified thickness or compression ratio or pressure by using fasteners; the shape of the edge contour line of the rigid plate includes any one of a camber line, a straight line and a broken line, or the edge contour line is formed by connecting and enclosing any two of the camber line, the straight line and the broken line end to end; and the size of the rigid plate is correspondingly set according to the size of the target specimen required by the test requirements of the physical, chemical and electrical properties, and when the rigid plate is liable to generate warping deformation under stress, any one or a combined body of any two of "+, ×, =, ⋈, , ⊕, #"-shaped stiffeners are arranged on one surface of the rigid plate to resist the warping deform.

Further, the combined specimen includes: on the superficial area of the target specimen, a part of the superficial area is in the state of the open specimen, and the other part of the superficial area is in the state of the closed specimen; or the fixture compression specimen is made into the state of the closed specimen again.

Further, the specified constant temperature includes: within an allowable temperature measurement error range, a constant temperature required for the experiment is set at a temperature below 400°C at least in an oven or a drying room or a warehouse; or a temperature curve is taken as a vertical coordinate, the time is taken as an abscissa, and an average temperature of ratios of areas below the temperature curve to corresponding times is taken as the constant temperature.

Further, the damp and hot includes: in the specified constant temperature environment, the moisture content of any one or a mixed medium of an air atmosphere, an oxidizing atmosphere, a reducing atmosphere and an inert gas atmosphere is controlled in the oven or the drying room or the warehouse, so as to control the relative humidity to (5-100)%.

Further, the high and low temperature impact includes: after a specified time in a specified higher temperature environment, the target specimen is transitioned to a lower temperature environment for the specified time according to a specified cooling rate; or, after a specified time in a specified lower temperature environment, the target specimen is transitioned to a higher temperature environment for the specified time according to a specified heating rate.

Further, the high and low temperature alternating cycle includes: according to a specified cooling rate and a heating rate, the target specimen is alternately transitioned for a specified time or an accumulative number of cycles between a higher specified constant temperature and a lower specified constant temperature environment; and the alternating transition is that the temperature curve is taken as the vertical coordinate, the time is taken as the abscissa, and the contour shape of the temperature curve includes any one of a straight line, a broken line and a cambered line, or a cyclic reciprocating and high-low undulating wave state formed by connecting any two lines end to end.

Further, the specified time or the accumulative number of cycles includes: the standard specimen is placed in the temperature-controlled oven or the drying room or the warehouse, is taken out from the oven or the drying room or the warehouse after a certain period of time or an accumulative number of times in accordance with established test procedures, and is placed in another specified constant temperature environment.

Further, the laminated combined test piece includes: during a constant temperature process, or when the physical, chemical and electrical properties are tested, at least one layer of materials or parts with known performance indicators and known dimensions is attached to the upper surface and the lower surface of the rigid plate of the fixture compression specimen, so that the instrument can accurately measure the physical, chemical and electrical properties.

Further, the measured value includes: data of the physical, chemical and electrical properties measured by an instrument or equipment that meets the requirements of test standards for the physical, chemical and electrical properties, in accordance with actions and conditions specified by corresponding standards.

Further, the micro-gasification expansion oscillation includes: with the observation of an oscillation phenomenon of the physical, chemical and electrical properties of the target specimen as a basis, and the superposition mechanism of micro-gasification, expansion, migration, volatilization and chemical reaction of low molecular substances generated by the target specimen as a mathematical model, a general equation (1) for the aging oscillation trend of the physical, chemical and electrical properties of the target specimen is mathematically deduced.

Further, the mathematical model further includes: aging failure model analysis, physical aging simplified treatment, and chemical aging simplified treatment.

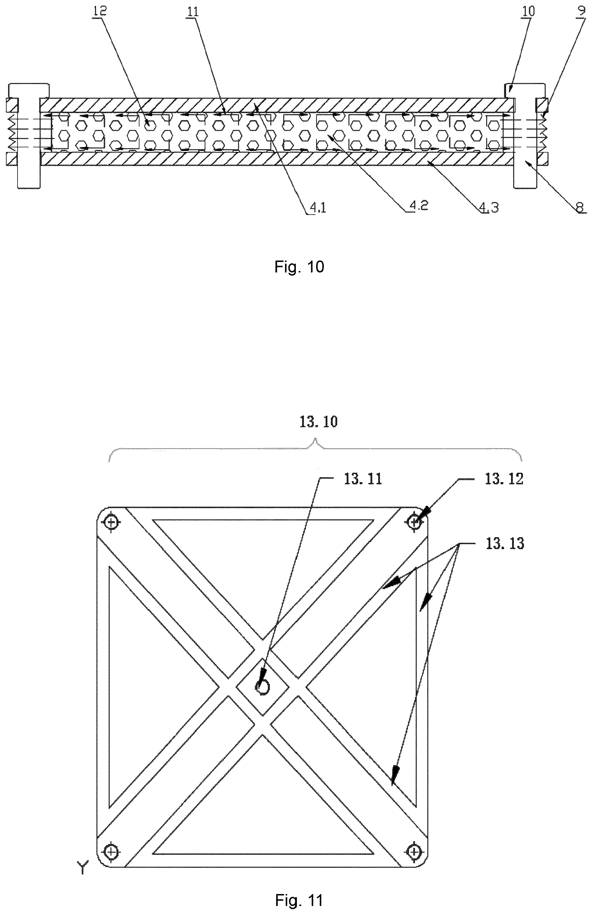

The aging failure model analysis (Aging-DFEAM) further includes: as shown in Fig. 10, a target specimen 4.2 is clamped on both sides of a metal upper rigid plate 4.1 and a lower rigid plate 4.3, and is fastened by a metal screw rod 8 to form a "sandwich biscuit" structure, and the compression ratio of the target specimen 4.2 is adjusted to a specified value within the range of (0-40)%, for example, three types of fixture compression specimens with compression ratios of 10%, 20%, and 30% respectively; and the average thickness of an air layer sandwiched among the upper rigid plate 4.1, the lower rigid plate 4.3 and the target specimen 4.2 is reduced to less than one-half of an average particle size of powder fillers inside the target specimen 4.2, the average particle size dso of heat conducting powder or fillers is (1.5-15) $\mu o$ in general, especially grading composited powders, and the thickness of the target specimen 4.2 is (0.25-5)mm.

The physical aging simplified treatment further includes:

1) at room temperature, low molecular substances generate no gasification expansion, but only migrate and volatilize in the form of free molecules, as shown in Fig. 10-

a) in addition to the metal upper rigid plate 4.1 and the lower rigid plate 4.3 of the "sandwich biscuit" structure, the low molecular substances including air, sulfides, oxynitrides, ozone and moisture are "breathing" due to seasonal temperature cycles, and are mainly transferred to the inside and outside of the target specimen 4.2 through two "gap" channels with the minimum interface resistance in contact with the metal upper rigid plate 4.1, the target specimen 4.2, and the lower rigid plate 4.3, which is regarded as a transfer process of inert substances, and the influence on the chemical aging speed of the target specimen 4.2 is negligible due to a short time; and although the outdoor seasonal temperature cycle can reach (-45-65)°C, due to the existence of an installation pre-tightening pressure, the periodical increase and decrease in the thickness of an air film on the interference is negligible, so the influence on the interface heat resistance and other physical, chemical and electrical properties is also negligible;

b) on the interface among the metal upper rigid plate 4.1, the target specimen 4.2 and the lower rigid plate 4.3, residual low molecular substances including the air, sulfides, oxynitrides, ozone and moisture only account for less than one hundred thousandth of the weight of the target specimen 4.2, so the influence on the chemical aging speed of the target specimen 4.2 is also negligible;

c) the concentration gradients of other low molecular substances contained in the target specimen 4.2 have a diffusion driving force due to spontaneous volatilization, free molecules of the low molecular substances are diffused in a phase-change-free material state, and migrate from the inside of the target specimen 4.2 along the thickness direction of the target specimen 4.2 to the "gaps" on the interface among the metal upper rigid plate 4.1, the target specimen 4.2 and the lower rigid plate 4.3, the migration and diffusion direction 11 of the low molecular substances continues to diffuse along the "gaps" to the interface between the target specimen 4.2 near a bolt 8 in a volatilization direction 9 of low molecular substances and the external air, the volatilization direction 9 of low molecular substances is further away from the target specimen 4.2, and unidirectional migration dominates. Although the influence on the chemical aging speed of the target specimen 4.2 is negligible, a positive effect is generated on improving an intrinsic thermal conductivity of the target specimen 4.2, and the physical influence on the heat resistance of the interface is negligible, resulting in a short-term and small-scale increase in an apparent thermal conductivity; and both positive and negative effects on generated on the other physical, chemical and electrical properties;

2) at a high temperatures, the low molecular substances generate micro-gasification expansion, and also migrate and volatilize, as shown in Fig. 10-

d) first, only when the service temperature is higher than the boiling points of the low molecular substances, when micro-gasification occurs inside the target specimen 4.2, the gas-phase low molecular substances further aggregate into infinitesimal volume gas clusters and produce a micro-expansion effect, which will at least significantly reduce the intrinsic thermal conductivity, the hardness, the density and the compression set rate of the target specimen 4.2;

e) then, the low molecular substances inside the target specimen 4.2 move in a gas phase material state, and driven by the gas expansion pressure gradient, the low molecular substances first migrate along the thickness direction of the target specimen 4.2 into the "gaps" on the interface among the upper metal rigid plate 4.1, the target specimen 4.2 and the lower rigid plate 4.3, and then migrate unidirectionally to the outside of the target specimen 4.2 through the "gap" channels on the interface, the volatilization direction 9 of low molecular substances is further away from the target specimen 4.2, which will significantly and irregularly increase the thickness and area of the air film on the interface among the metal upper rigid plate 4.1, the target specimen 4.2 and the lower rigid plate 4.3, and aggravate the fluctuation range of the interface heat resistance, such that the apparent thermal conductivity forms an oscillation state of peaks and valleys, and forms a time-varying chaotic system; and

f) finally, with the continuous migration and volatilization of the low molecular substances, the content of the low molecular substances inside the target specimen 4.2 is getting lower and lower, the expansion energy of micro-gasification is gradually attenuated, the micro-expansion effect gradually disappears, the intrinsic thermal conductivity of the target specimen 4.2 gradually returns to an initial value and increases, the interface heat resistance is also decreased to be close to the initial state before micro-gasification, and an upper limit peak value of the apparent thermal conductivity is formed. However, with the progress of the chemical aging time, the apparent thermal conductivity increases less and decreases more after the competition.

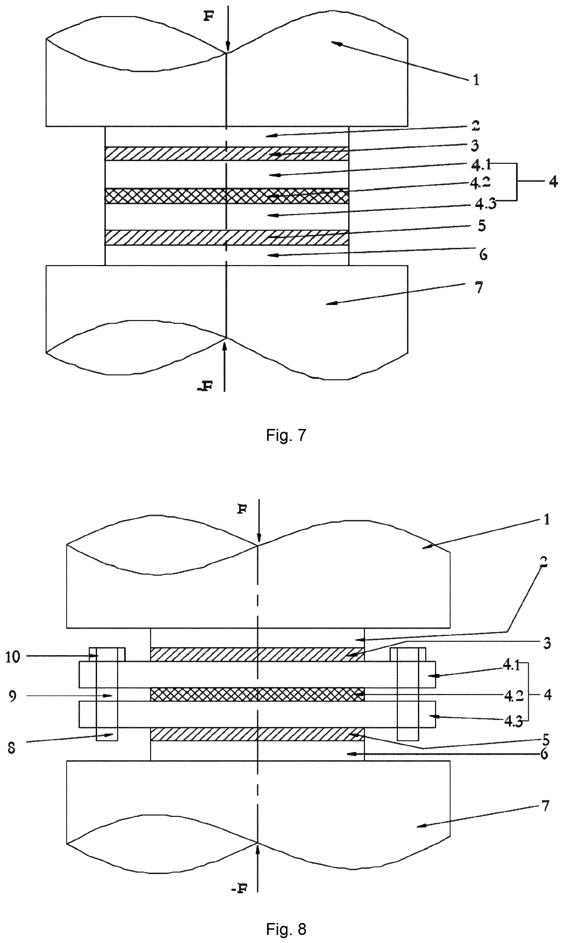

3) at any temperature, the release of mechanical compression internal stress, as shown in Fig. 7 and Fig. 8-including the metal upper rigid plate 4.1, the target specimen 4.2 and the lower rigid plate 4.3. Due to the active or passive thermodynamic movement of the material of the non-detachable combined specimen 4, no matter the micro-gasification, the migration and the volatilization produced by the microscopic movements of the low molecular substances, or the macroscopic stress produced by heat expansion and contraction, and mechanical compression, it is beneficial to accelerating the release rate of the mechanical internal stress caused by the non-detachable combined specimen 4, or to identifying the strength of the mechanical internal stress.

The chemical aging simplified treatment further includes:

As shown in Fig. 10, the external low molecular substances including the air, sulfides, oxynitrides, ozone and moisture are not only transferred to the inside of the target specimen 4.2 through the "gap" channels on the interface, but also linearly migrate to the inside from the edge surface of the target specimen 4.2, the influence on the chemical aging speed of the target specimen 4.2 is mainly controlled by the diffusion speed of the low molecular substances including the sulfides, oxynitrides, ozone, oxygen and moisture inside the target specimen 4.2, the diffusion speed is in line with the Fick's law, but the diffusion rate is also inversely proportional to the thickness of the target specimen 4.2 and is inversely proportional to the square of the diameter. When the width or diameter of the target specimen 4.2 is large enough, for example, the diameter of a laboratory specimen is greater than 30 mm, then the low molecular substances inside the target specimen 4.2 migrate outward, and the sulfides, oxynitrides, ozone, oxygen and moisture on the edge surface of the target specimen 4.2 migrate inward, the influence on the aging inside of the target specimen 4.2 is reduced to a secondary factor for treatment, and the influence on the chemical aging the target specimen 4.2 caused by the exchange of external substances is also negligible, such that the chemical aging process is simplified as heat aging dominant-competition of degradation and cross linking;

therefore, the main factors affecting the chemical aging speed of the target specimen 4.2 are:

the molecular chain structure of a high polymer base material and the chemical stability of an additive system thereof, the aging influence of this factor is sensitive to duration and temperature;
the aging of mechanical compression stress, the aging influence of this factor is sensitive to stress duration, compression ratio and temperature, and when the compression ratio is constant, the aging influence of this factor is only sensitive to the stress duration and temperature, but for elastomers, the decay rate of the influence of the mechanical stress is very fast;
the catalytic aging of chemical elements and their compounds in contact with the surfaces of the metal upper rigid plate 4.1 and the lower rigid plate 4.3, and the aging influence of this factor is sensitive to the types of the chemical elements and their compounds, the contact duration and the temperature; and
sudden changes in environmental temperature gradients and stress aging produced by heat expansion and contraction, and the aging influence of this factor is sensitive to the change rate of the temperature gradients, but is not sensitive to rubber high elastomers.

Further, the micro-gasification expansion oscillation equation (1) includes:

$$P_t = P_\infty + \left\{ P_{0\ominus} + \left[ \Delta P_1 e^{-k_1 t} \times \mathrm{Sin}\beta_1 \left( \frac{t}{t_0} - 1 \right)^{\theta_1} \pi + \Delta P_2 e^{-k_2 t} \times + \mathrm{Sin}\beta_2 \left( \frac{t}{t_0} - 1 \right)^{\theta_2} \pi + \Delta P_3 e^{-k_3 t} \right] - P_\infty \right\} e^{-kt} \ldots\ldots\ldots (1)$$

in the equation (1),

P-any one of the physical, chemical and electrical properties, a measured value, used for parameter fitting or verification;

$P_t$-the physical, chemical and electrical properties at any specified constant temperature for any service time, an evaluated or predicted value;

$P_\infty$-the physical, chemical and electrical properties at the aging end point, determined by numerical simulation of a material formula, or a fitted value;

$P_{0\ominus}$-initial physical, chemical and electrical properties before aging, a measured value;

$P_{0\oplus}$ -the physical, chemical and electrical properties at the beginning of micro-gasification expansion, a fitted value;

Sin-micro-gasification oscillation trigonometric function;

$\Delta P_1$-micro-gasification internal influence parameter, $\Delta P_1 = (P_{0\oplus} - P_{0\ominus})$;

$\Delta P_2$-micro-gasification interface influence parameter, a fitted value;

$\Delta P_3$-mechanical stress influence parameter, a fitted value;

t-aging time or service time, determined by a specified time or an accumulative number of cycles;

$t_0$-migration lag time of low molecular substances, a fitted value;

$\beta_1$-migration oscillation frequency coefficient, a fitted value;

$\beta_2$-volatilization oscillation frequency coefficient, a fitted value;

$k_1$-migration rate parameter, a fitted value;

$k_2$-volatilization rate parameter, a fitted value;

$k_3$-relaxation rate parameter, a fitted value;

k-chemical reaction rate parameter, a fitted value;

$\theta_1$-migration oscillation frequency index, a fitted value;

$\theta_2$-volatilization oscillation frequency index, a fitted value;

wherein, the equation (1) covers all the physical, chemical and electrical properties, for the sake of brevity, it is expressed as a general expression containing fifteen parameters that do not change with time, and it is not just a relational expression expressing one property; and when any one of the physical, chemical and electrical properties is evaluated or predicted, the corresponding parameters and symbols of the physical, chemical and electrical properties in the equation (1) need to be replaced one by one.

Further, the parameters include: a total of fifteen parameters $P_\infty$, $P_{0\ominus}$, $P_{0\oplus}$, $\Delta P_1$, $\Delta P_2$, $\Delta P_3$, $t_0$, $\beta_1$, $\beta_2$, $k_1$, $k_2$, $k_3$, k, $\theta_1$, $\theta_2$ in the equation (1), fourteen of which are independent parameters, the other $\Delta P_1$ is a linear correlation parameter, and the parameters do not change with time but change with temperature; and for the sake of brevity, a symbol "Q" is used for representing any one of the fifteen parameters.

Further, the constants further include: each parameter "Q" in the micro-gasification expansion oscillation equation (1) contains three constants, which neither change with time nor with temperature, and only change with the chemical components of the target specimen; for the sake of brevity, the three letters "A, B and C" are used for representing the three constants under each parameter; when any of the physical and chemical electrical properties is evaluated or predicted, each parameter and its corresponding constants in the dynamic correlation equation (2) are replaced one by one;

$$\ln Q = \frac{A}{T+C} + B \dots\dots\dots\dots\dots\dots\dots\dots\dots\dots\dots\dots\dots\dots\dots\dots\dots\dots\dots\dots \quad (2)$$

in the equation (2),

Q-any one of the fifteen parameters in the equation (1) at any temperature;

A-empirical constant associated with superposed reaction activation energy and diffusion activation energy of multiple components, a fitted value, K;

B-empirical constant associated with superposed chemical reaction rate and diffusion rate of multiple components, a fitted value, dimensionless;

C-conformal constant after Fourier series transformation associated with the activation energy of multiple components, a fitted value, K; and

T-absolute temperature, specified constant temperature +273.15, K.

Further, the parameter fitting includes: the measured value (P) of the physical, chemical and electrical properties is used as a verification specimen; an electronic calculation program or a parallax method is utilized to perform respective increase or decrease with a step pitch as small as possible, the measured value is input into the equation (1), and the respective "Q" values of the fifteen different parameters are repeatedly iterated and cycled to output calculated values $(P_t)$; when a standard deviation of a difference value between the calculated value $(P_t)$ and the measured value (P) converges to the minimum, the "Q" values corresponding to the fifteen parameters are used as optimal values; and due to a mathematical frequency doubling effect, if there is more than one optimal value among the fitted values of the fifteen parameters, only the group of fifteen smaller "Q" values closest to "1 time" is selected as the optimal parameters.

Further, the constant fitting includes: different "C" values are tentatively input, and are repeatedly iterated in the equation (2), plotting is performed by using the logarithms of the "Q" values of the fifteen optimal parameters as vertical coordinates, and using 1/(T+C) as abscissas, the points are connected into a line, and when the line is close to a straight line, "A, B and C" become the optimal fitted values; or a least square method electronic calculation program or a parallax method is utilized to perform increase or decrease with a step pitch as small as possible, different "C" values are input and are repeatedly iterated in the equation (2), when $R^2$ output by a calculation program system is ≥ 0.990, it is considered that the line has been a straight line; the "A, B and C" in one-to-one correspondence with the obtained fifteen parameters become the optimal constants, wherein the minimum boundary of the value "C" is -273.

Further, the material of the rigid plate is selected from any one of ore, stainless steel, carbon steel, copper alloy, aluminum alloy, ceramics, polytetrafluoroethylene, polyimide, and polyphenylene sulfide, or the two rigid plates respectively select any two of the materials for permutation and combination.

The test method and algorithm for the aging life of the new energy heat management composite, and the use thereof in the present invention have the following beneficial technical effects:

(1) the highest aging test temperature reaches 400°C, which shortens the laboratory aging test time by 90% from over 1,000 hours;

(2) it is suitable for predicting the aging lives of materials with the coexistence of three-phase material state of solid, liquid and gas, which breaks through the limitation that the "width of an extended prediction temperature is less than 0.8 times of the difference between the highest test temperature and the lowest temperature" in the GB/T 20028, ASTM G 166, ASTM G 169, ISO 2578 and UL 746B standards;

(3) it is suitable for evaluating or predicting the long-term service lives of all polymer matrix composites; and

(4) the linear correlation coefficient $R^2$ is two "9" accuracy levels higher than GB/T 20028, ASTM G 166, ASTM G 169, ISO 2578 and UL 746B, so that the prediction is more accurate.

**BRIEF DESCRIPTION OF THE DRAWINGS**

Fig. 1 is a schematic diagram of mathematical conversion between a bulk thermal conductivity and an intrinsic thermal conductivity of a predetermined thickness of the present invention.

Fig. 2 shows one of physical examples of an upper rigid plate, a target specimen and a lower rigid plate of a circular fixture compression specimen of the present invention.

Fig. 3 is a schematic diagram of measuring the bulk thermal conductivity of an equal-diameter elastic target specimen of the present invention.

Fig. 4 is a schematic diagram of measuring an equivalent thermal conductivity of a non-equal-diameter elastic target specimen of the present invention.

Fig. 5 is a schematic diagram of measuring an apparent thermal conductivity of the present invention, when equal-diameter upper and lower elastomers cooperate with a rigid target specimen.

Fig. 6 is a schematic diagram of measuring the apparent thermal conductivity of the present invention, when non-equal-diameter upper and lower elastomers cooperate with the rigid target specimen.

Fig. 7 is a schematic diagram of measuring the apparent thermal conductivity of the present invention, when the equal-diameter upper and lower elastomers cooperate with a non-detachable fixture compression specimen.

Fig. 8 is a schematic diagram of measuring the apparent thermal conductivity of the present invention, when the non-equal-diameter upper and lower elastomers cooperate with the non-detachable fixture compression specimen.

Fig. 9 shows the circular fixture compression specimen of the present invention, "4.1A-4.2-4.3A" representing a non-detachable butt joint bonding entirety in 9a; and, "4.1-4.2-4.3" being detachable in 9b.

Fig. 10 is a schematic diagram of a physical model of migration, diffusion and volatilization of low molecular substances and their infinitesimal volume cavitations according to the present invention.

Fig. 11 is a top view of one example of an upper square clamping plate of the present invention.

Fig. 12 is an upward view of one example of a lower square clamping plate of the present invention.

Fig. 13 is a schematic diagram of a lap joint bonding pair and a non-detachable bonding pair for measuring shear bonding strength according to international standards.

Fig. 14 is a schematic diagram of the present invention, in which a square fixture compression specimen and the lap joint bonding pair are compressed.

Fig. 15 is a front view of an upper electrode tip used for measuring the breakdown strength according to the international standards.

Fig. 16 is a front view of a lower electrode tip used for measuring the breakdown strength according to the international standards.

Fig. 17 is a front view of the present invention, in which the square fixture compression specimen, an electrode tip for a breakdown strength test and the target specimen are compressed.

Fig. 18 is a front view and a partial axial cross-sectional view of the present invention, in which the square fixture compression specimen, an electrode tip for a volume resistivity test and the target specimen are compressed.

Fig. 19 shows the comparison between measured values and predicted values of the intrinsic thermal conductivity of P20 under three compression ratios at 298°C in a first embodiment.

Fig. 20 shows the comparison between the measured values and the predicted values of the intrinsic thermal conductivity of P40 under three compression ratios at 298°C in the first embodiment.

Fig. 21 shows the comparison between the measured values and the predicted values of the intrinsic thermal conductivity of P20 under three compression ratios at 272°C in the first embodiment.

Fig. 22 shows the comparison between the measured values and the predicted values of the intrinsic thermal conductivity of P40 under three compression ratios at 272°C in the first embodiment.

Fig. 23 shows the comparison between the measured values and the predicted values of the intrinsic thermal conductivity of P20 under three compression ratios at 245°C in the first embodiment.

Fig. 24 shows the comparison between the measured values and the predicted values of the intrinsic thermal conductivity of P40 under three compression ratios at 245°C in the first embodiment.

Fig. 25 shows the comparison between the measured values and the predicted values of the intrinsic thermal conductivity of P20 under three compression ratios at 218°C in the first embodiment.

Fig. 26 shows the comparison between the measured values and the predicted values of the intrinsic thermal conductivity of P40 under three compression ratios at 218°C in the first embodiment.

Fig. 27 shows a first application example, in which an equation (1.1) is used for predicting long-term aging trends of the intrinsic thermal conductivitys of P20 and P40 at 195°C.

Fig. 28 shows the first application example, in which the equation (1.1) is used for predicting the long-term aging trends of the intrinsic thermal conductivitys of P20 and P40 at 160°C.

Fig. 29 shows the first application example, in which the equation (1.1) is used for predicting the long-term aging trends of the intrinsic thermal conductivitys of P20 and P40 at 125°C.

Fig. 30 shows the first application example, in which the equation (1.1) is used for predicting the long-term aging trends of the intrinsic thermal conductivitys of P20 and P40 at 95°C.

Fig. 31 shows the first application example, in which the equation (1.1) is used for predicting the long-term aging trends of the intrinsic thermal conductivitys of P20 and P40 at 75°C.

Fig. 32 shows the first application example, in which the equation (1.1) is used for predicting the long-term aging trends of the intrinsic thermal conductivitys of P20 and P40 at 50°C.

Fig. 33 shows the first application example, in which the equation (1.1) is used for predicting the long-term aging trends of the intrinsic thermal conductivitys of P20 and P40 at 37°C.

Fig. 34 is a schematic diagram of a method for evaluating a rated temperature of P40 by using the equation (1.1) in an eighth application example.

Fig. 35 shows the comparison between the measured value and the predicted value of a compression set rate of S20 under the compression ratio of 30% at 245°C in a second embodiment.

Fig. 36 shows the comparison between the measured value and the predicted value of the compression set rate of S20 under the compression ratio of 30% at 218°C in the second embodiment.

Fig. 37 shows the comparison between the measured value and the predicted value of the compression set rate of S20 under the compression ratio of 30% at 195°C in the second embodiment.

Fig. 38 shows the comparison between the measured value and the predicted value of the compression set rate of S20 under the compression ratio of 30% at 150°C in the second embodiment.

Fig. 39 shows the comparison between the measured value and the predicted value of the compression set rate of S20 under the compression ratio of 30% at 97°C in the second embodiment.

Fig. 40 shows the comparison between the measured value and the predicted value of the compression set rate of S20 under the compression ratio of 30% at 85°C in the second embodiment.

Fig. 41 shows a second application example, in which an equation (1.2) is used for predicting the long-term aging trend of the compression set rate of S20 at three service temperatures.

Fig. 42 shows partial enlargement of a time axis indicator in Fig. 41.

Fig. 43 shows the comparison between the measured value and the predicted value of the hardness of S20 under the compression ratio of 30% at 245°C in a third embodiment.

Fig. 44 shows the comparison between the measured value and the predicted value of the hardness of S20 under the compression ratio of 30% at 218°C in the third embodiment.

Fig. 45 shows the comparison between the measured value and the predicted value of the hardness of S20 under the compression ratio of 30% at 195°C in the third embodiment.

Fig. 46 shows the comparison between the measured value and the predicted value of the hardness of S20 under the compression ratio of 30% at 150°C in the third embodiment.

Fig. 47 shows the comparison between the measured value and the predicted value of the hardness of S20 under the compression ratio of 30% at 97°C in the third embodiment.

Fig. 48 shows the comparison between the measured value and the predicted value of the hardness of S20 under the compression ratio of 30% at 85°C in the third embodiment.

Fig. 49 shows a third application example, in which an equation (1.3) is used for predicting the long-term aging trend of the hardness of S20 at three service temperatures.

Fig. 50 shows partial enlargement of the time axis indicator in Fig. 49.

Fig. 51 shows the comparison between the measured value and the predicted value of the tensile strength of S20 under the compression ratio of 30% at 245°C in a fourth embodiment.

Fig. 52 shows the comparison between the measured value and the predicted value of the tensile strength of S20 under the compression ratio of 30% at 218°C in the fourth embodiment.

Fig. 53 shows the comparison between the measured value and the predicted value of the tensile strength of S20 under the compression ratio of 30% at 195°C in the fourth embodiment.

Fig. 54 shows the comparison between the measured value and the predicted value of the tensile strength of S20 under the compression ratio of 30% at 150°C in the fourth embodiment.

Fig. 55 shows the comparison between the measured value and the predicted value of the tensile strength of S20 under the compression ratio of 30% at 97°C in the fourth embodiment.

Fig. 56 shows the comparison between the measured value and the predicted value of the tensile strength of S20 under the compression ratio of 30% at 85°C in the fourth embodiment.

Fig. 57 shows a fourth application example, in which an equation (1.4) is used for predicting the long-term aging trend of the tensile strength of S20 at three service temperatures.

Fig. 58 shows partial enlargement of the time axis indicator in Fig. 57.

Fig. 59 shows the comparison between the measured value and the predicted value of the shear bonding strength of S20 under the compression ratio of 30% at 245°C in a fifth embodiment.

Fig. 60 shows the comparison between the measured value and the predicted value of the shear bonding strength of S20 under the compression ratio of 30% at 218°C in the fifth embodiment.

Fig. 61 shows the comparison between the measured value and the predicted value of the shear bonding strength of S20 under the compression ratio of 30% at 195°C in the fifth embodiment.

Fig. 62 shows the comparison between the measured value and the predicted value of the shear bonding strength of S20 under the compression ratio of 30% at 150°C in the fifth embodiment.

Fig. 63 shows the comparison between the measured value and the predicted value of the shear bonding strength of S20 under the compression ratio of 30% at 97°C in the fifth embodiment.

Fig. 64 shows the comparison between the measured value and the predicted value of the shear bonding strength of S20 under the compression ratio of 30% at 85°C in the fifth embodiment.

Fig. 65 shows a fifth application example, in which an equation (1.5) is used for predicting the long-term aging trend of the shear bonding strength of S20 at three service temperatures.

Fig. 66 shows partial enlargement of the time axis indicator in Fig. 65.

Fig. 67 shows the comparison between the measured value and the predicted value of the breakdown strength of S20 under the compression ratio of 30% at 245°C in a sixth embodiment.

Fig. 68 shows the comparison between the measured value and the predicted value of the breakdown strength of S20 under the compression ratio of 30% at 218°C in the sixth embodiment.

Fig. 69 shows the comparison between the measured value and the predicted value of the breakdown strength of S20 under the compression ratio of 30% at 195°C in the sixth embodiment.

Fig. 70 shows the comparison between the measured value and the predicted value of the breakdown strength of S20 under the compression ratio of 30% at 150°C in the sixth embodiment.

Fig. 71 shows the comparison between the measured value and the predicted value of the breakdown strength of S20 under the compression ratio of 30% at 97°C in the sixth embodiment.

Fig. 72 shows the comparison between the measured value and the predicted value of the breakdown strength of S20 under the compression ratio of 30% at 85°C in the sixth embodiment.

Fig. 73 shows a sixth application example, in which an equation (1.6) is used for predicting the long-term aging trend of the breakdown strength of S20 at three service temperatures.

Fig. 74 shows partial enlargement of the time axis indicator in Fig. 73.

Fig. 75 shows the comparison between the measured value and the predicted value of a logarithm of the volume resistivity of S20 under the compression ratio of 30% at 245°C in a seventh embodiment.

Fig. 76 shows the comparison between the measured value and the predicted value of the logarithm of the volume resistivity of S20 under the compression ratio of 30% at 218°C in the seventh embodiment.

Fig. 77 shows the comparison between the measured value and the predicted value of the logarithm of the volume resistivity of S20 under the compression ratio of 30% at 195°C in the seventh embodiment.

Fig. 78 shows the comparison between the measured value and the predicted value of the logarithm of the volume resistivity of S20 under the compression ratio of 30% at 150°C in the seventh embodiment.

Fig. 79 shows the comparison between the measured value and the predicted value of the logarithm of the volume resistivity of S20 under the compression ratio of 30% at 97°C in the seventh embodiment.

Fig. 80 shows the comparison between the measured value and the predicted value of the logarithm of the volume resistivity of S20 under the compression ratio of 30% at 85°C in the seventh embodiment.

Fig. 81 shows a seventh application example, in which an equation (1.7) is used for predicting the long-term aging trend of the logarithm of the volume resistivity of S20 at three service temperatures.

Fig. 82 shows partial enlargement of the time axis indicator in Fig. 81.

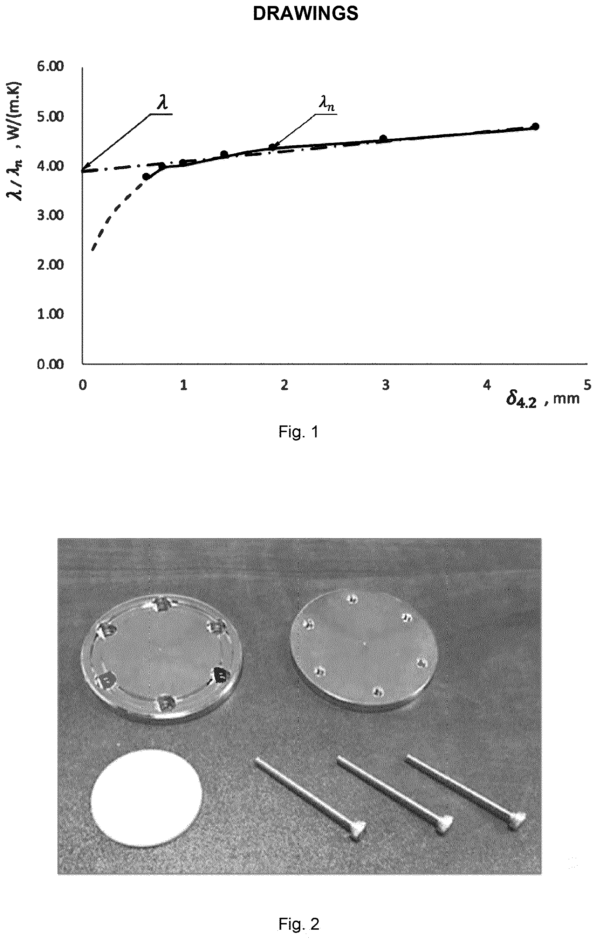

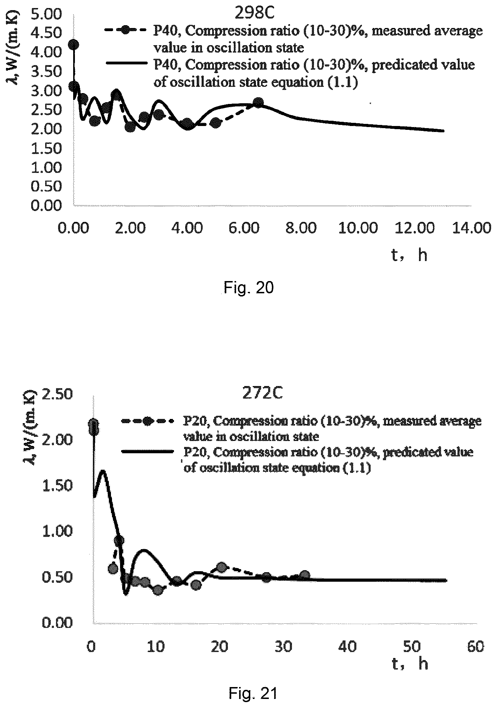

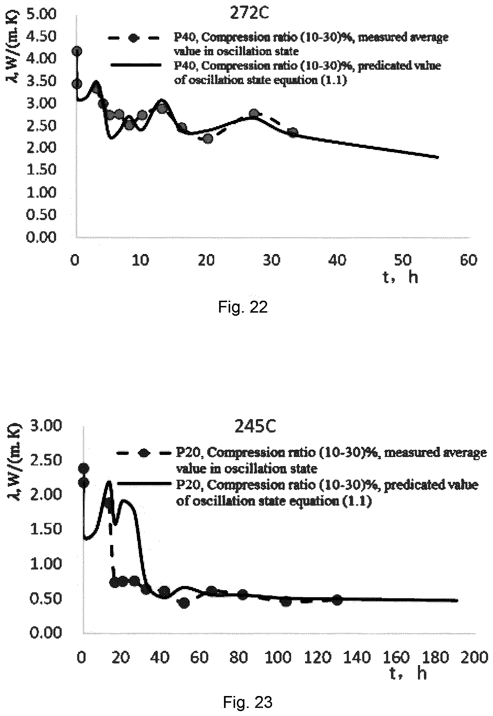

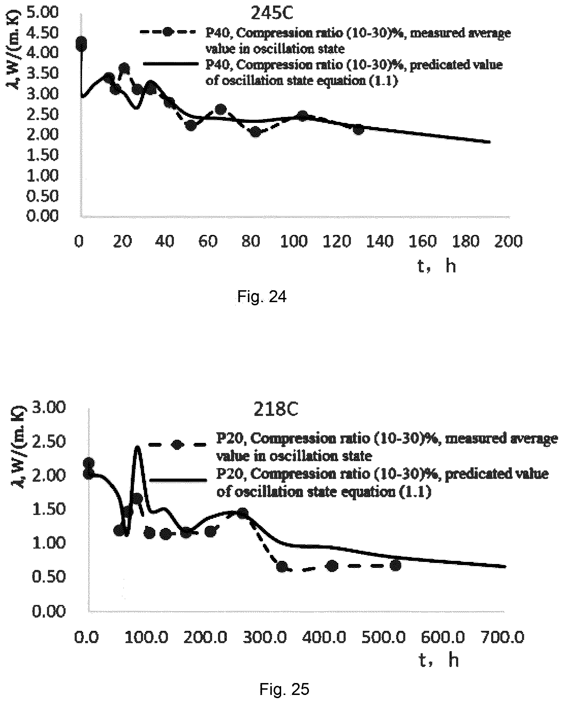

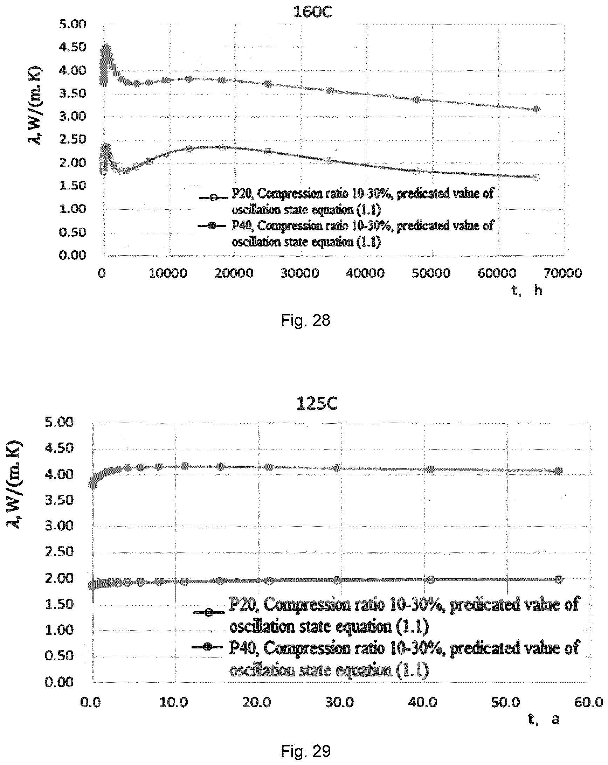

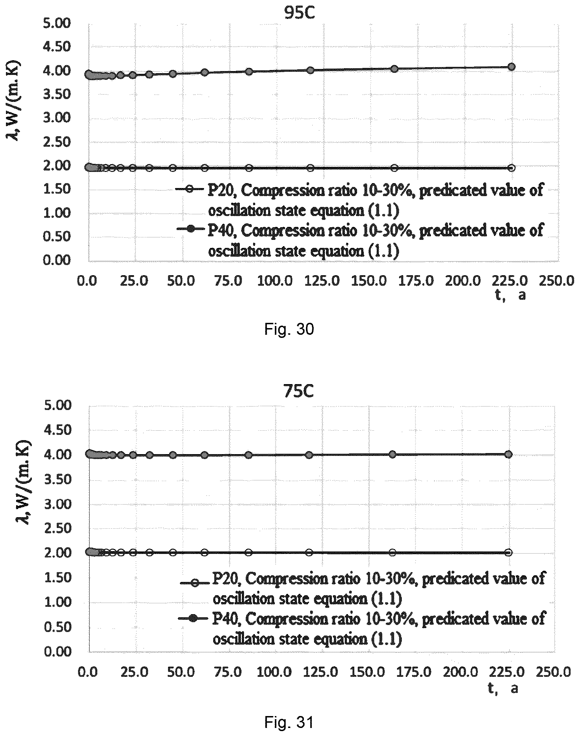

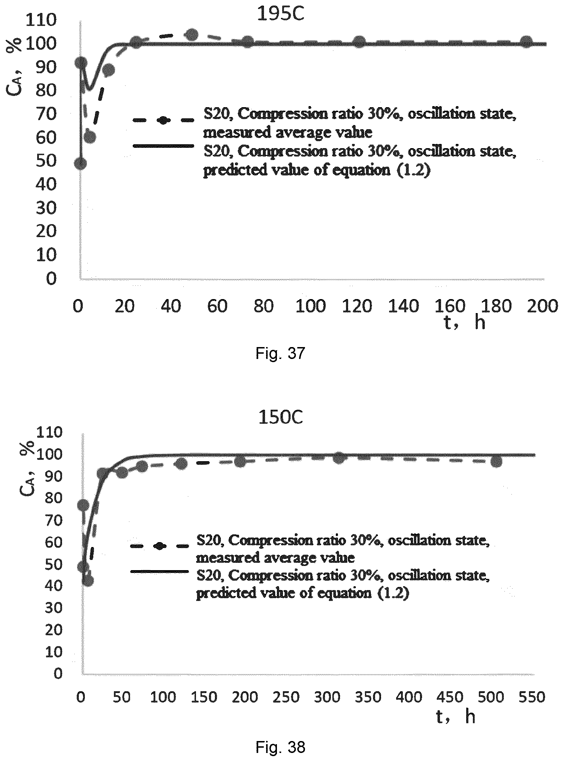

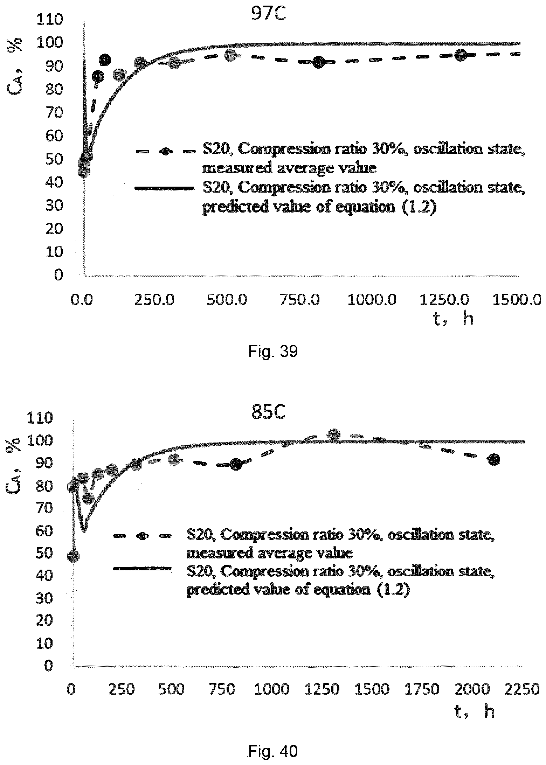

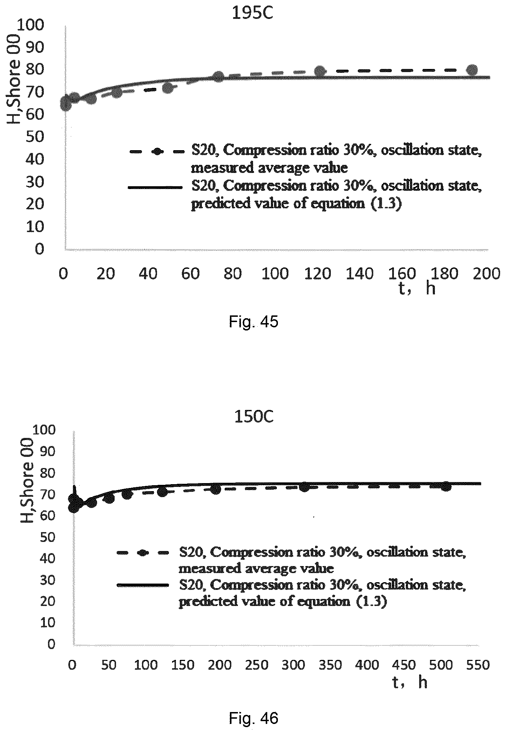

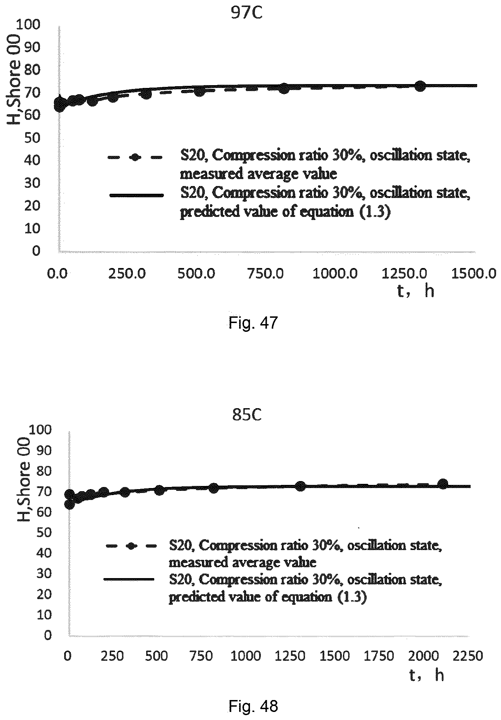

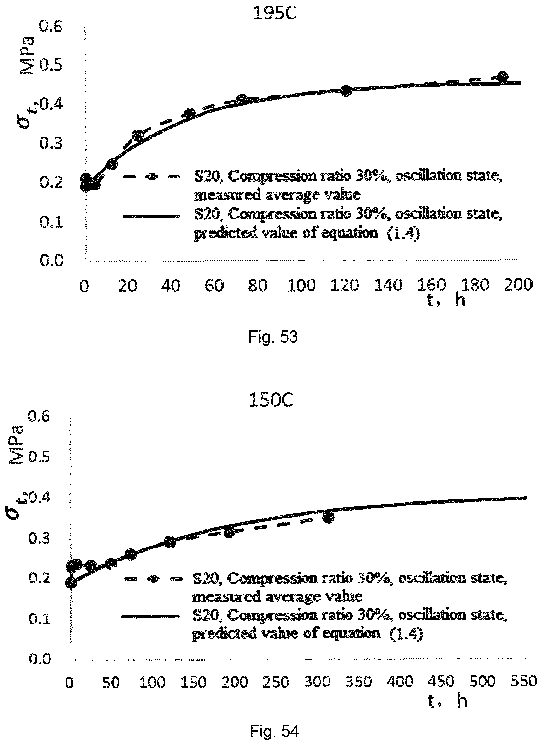

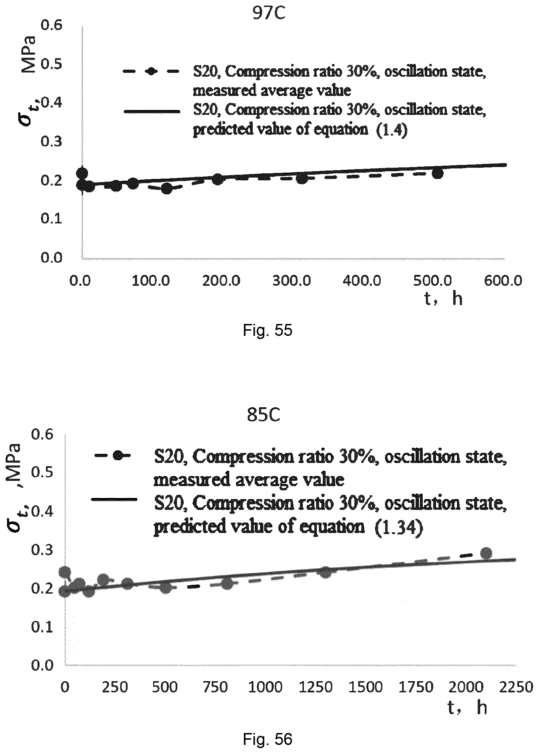

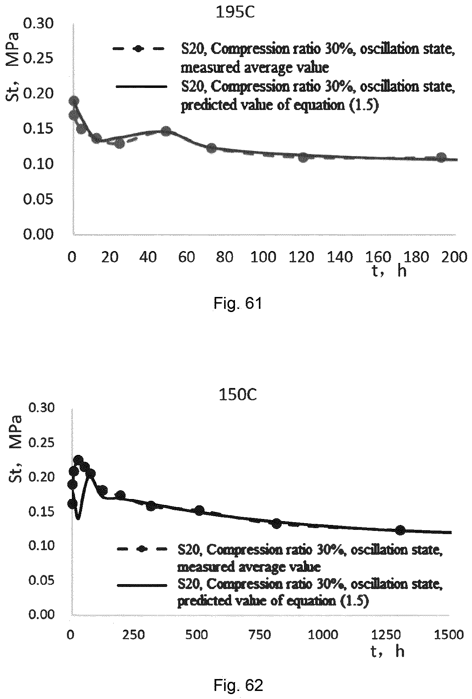

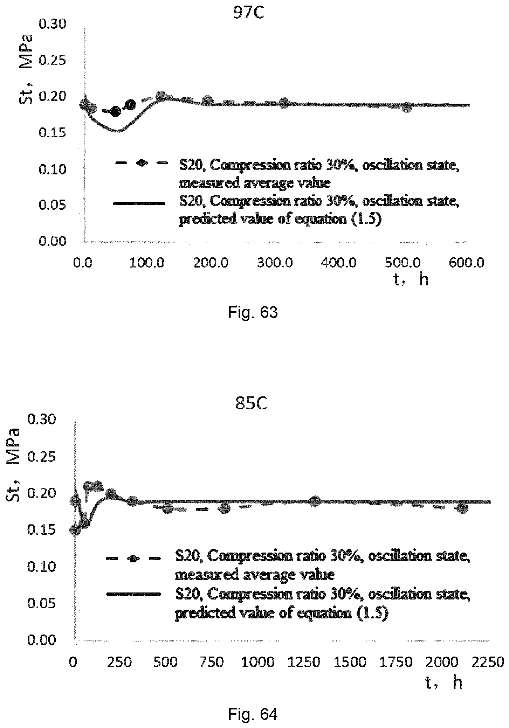

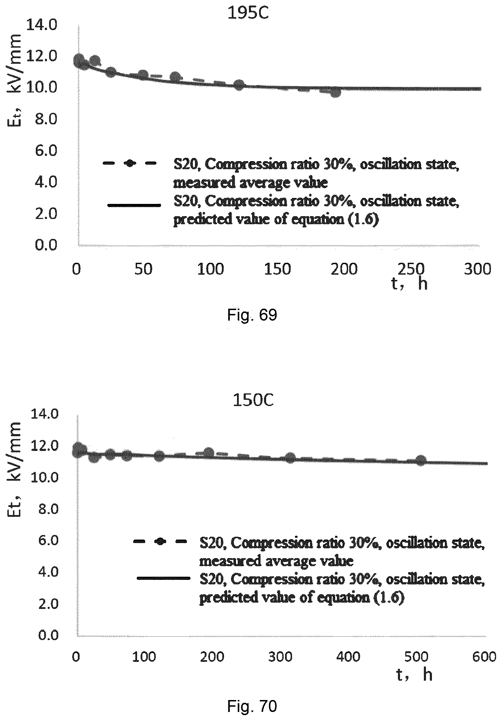

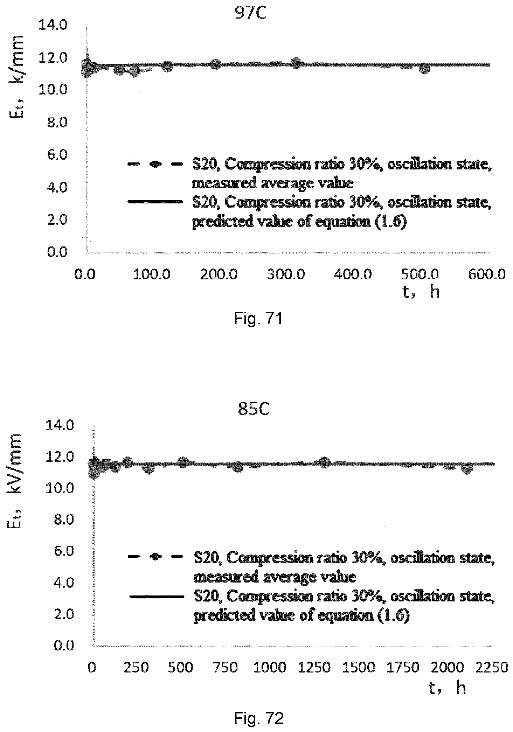

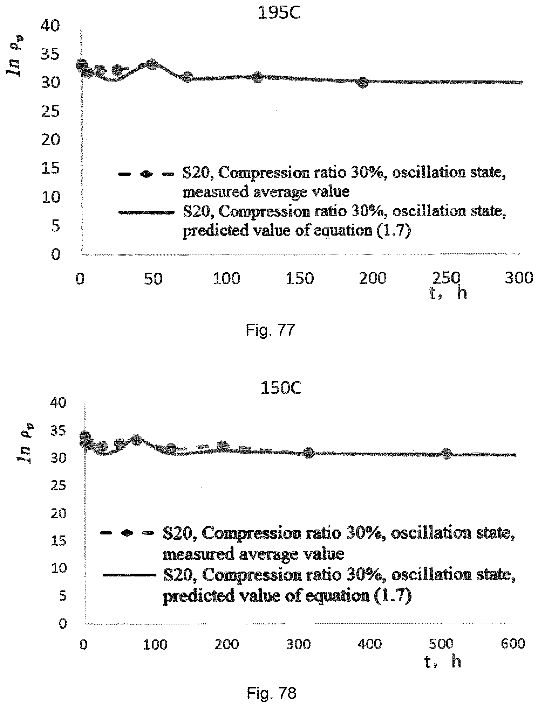

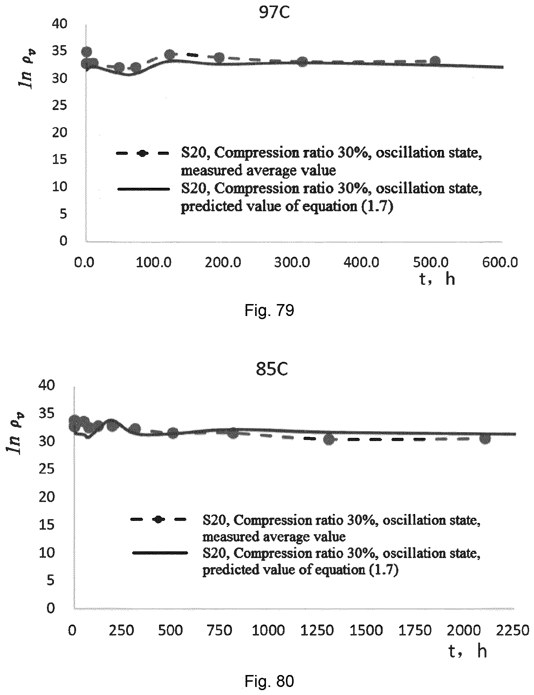

In Fig. 1 to Fig. 82, the parts with the same functions and the same structures have the same reference signs. For the sake of brevity of the drawings, the reference signs of the parts on symmetrical positions or the same series positions are omitted.

1-thermoae constant temperature cylinder,
2-thermoae pressure head,
3-upper auxiliary elastic sheet,
4-non-detachable combined specimen,

    4.1-upper rigid plate,
    4.1A-upper butt joint bonding plate,
    4.2-target specimen,
    4.3-lower rigid plate,
    4.3A-lower butt joint bonding plate,

5-lower auxiliary elastic sheet,
6-cold electrode pressure head,
7-cold electrode constant temperature cylinder,
8-screw rod,
9-volatilization direction of low molecular substances,
10-nut,
11-migration and diffusion direction of low molecular substances,
12-infinitesimal volume cavitation,
13.10-upper square clamping plate,
13.11-threaded hole A,
13.12-through hole,
13.13-protruding stiffener,

13.20-lower square clamping plate,

13.21-threaded hole B,

13.22-threaded hole C,

13.23-protruding stiffener,

14.10-upper lap joint bonding sheet,

14.20-lower lap joint bonding sheet,

15.1-upper positioning sheet A,

15.2-lower positioning sheet A,

15.3-upper positioning sheet B,

15.4-lower positioning sheet B,

16.10-upper electrode,

16.11-upper electrode tip,

16.12-upper electrode plate,

16.20-lower electrode,

16.21-lower electrode tip,

16.22-lower electrode plate,

17-high temperature insulation sheet resistant to 245 °C or above,

18-protected electrode,

19-protection electrode,

20-non-protection electrode,

21-positioning screw (electrode tip),

22-nylon bolt,

23-high temperature insulation ring resistant to 245 °C or above,

F-automatically applied force of instrument,

-F-own reaction force of the instrument itself,

t-service time,

a-year,

$\delta_{4.2}$-the thickness of a target specimen on a thermal conductivity instrument.

## DETAILED DESCRIPTION OF THE EMBODIMENTS

Hereinafter, in conjunction with eight embodiments and the drawings thereof, a test method and algorithm for an aging life of a new energy heat management composite, and a use thereof in the present invention will be further explained in terms of technical contents, structural characteristics of test pieces, and achieved objectives and effects.

The eight use embodiments of the present invention list the application of the present invention in evaluating or predicting eight physical, chemical and electrical properties, that is, a thermal conductivity, a compression set rate, hardness, tensile strength, shear bonding strength, breakdown strength, volume resistivity and rated temperature, but the content and use of the present invention are not limited thereto.

In the eight use embodiments of the present invention, damp and hot, high and low temperature impact, and high and low temperature alternating cycle conditions of a short-term accelerated aging test are all completed in an air atmosphere, but the test atmosphere is not limited thereto.

1. First embodiment: evaluation or prediction of the service life of thermal conductivity

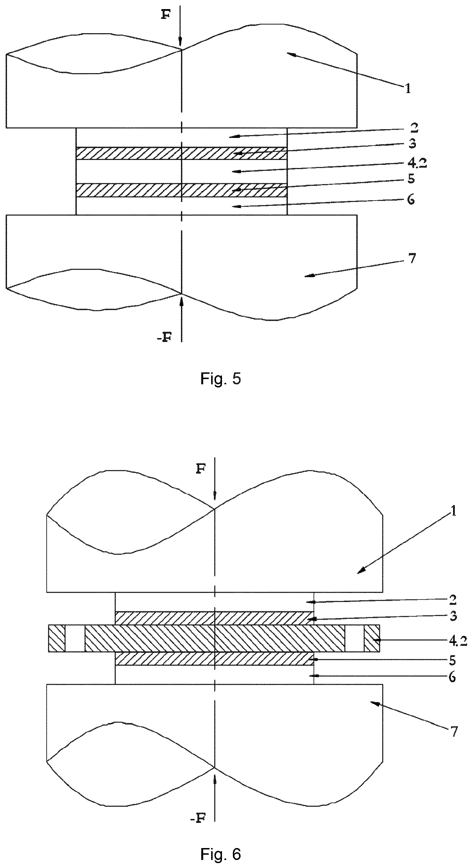

The first embodiment discloses one of the test method and algorithm for the aging life of the new energy heat management composite, and the use thereof in the present invention. The long-term change trend of the thermal conductivity of an interface target specimen during long-term service under actual working conditions is evaluated or predicted by using a short-term accelerated aging test method, including: using fixture compression specimens with three compression ratios of 10%, 20% and 30%; making the group of fixture compression specimens respectively undergo twelve specified times in four constant temperature environments within a temperature range of (218-298)°C under specified damp and hot conditions; in accordance with test procedures specified in ASTM D 5470, using a laminated combined test piece shown in Fig. 5 to Fig. 8 to test the thermal conductivity of the target specimen in the fixture compression specimens; using measured values of the thermal conductivity to fit corresponding fifteen parameters ($\lambda_\infty$, $\lambda_{0\ominus}$, $\lambda_{0\oplus}$, $\Delta\lambda_1$, $\Delta\lambda_2$, $\Delta\lambda_3$, $t_0$, $\beta_1$, $\beta_2$, $k_1$, $k_2$, $k_3$, $k$, $\theta_1$, $\theta_2$) in a micro-gasification expansion oscillation equation (1.1), and using each fitted parameter value to further fit corresponding three constants "A, B and C" in a dynamic correlation equation (2.1); substituting the three fitted constant values back into the dynamic correlation equation (2.1), so as to calculate new values of each parameter at the service temperatures of 195°C, 160°C, 125°C, 95°C, 75°C, 50°C and 37°C; and substituting the new values of this group of parameters back into the equation (1.1), so as to evaluate or predict the time-varying long-term change trends of the thermal conductivity of the target specimen after a specified service time under the damp and hot conditions at 195°C, 160°C, 125°C, 95°C, 75°C, 50°C and 37°C. The details of the implementation steps will be further disclosed in the following seven chapters 1.1 to 1.7-

1.1 Preparation of the fixture compression specimen

As shown in Fig. 2 and Fig. 10.

1.1.1 Interface heat conducting material for new energy power battery packs, a target specimen 4.2 has two products:

1) silicone rubber heat conducting sheet: with a nominal thermal conductivity 2 W/(m.K) and a thickness 2.5 mm, referred to as P20; and

2) silicone rubber heat conducting sheet: with a nominal thermal conductivity 4 W/(m.K) and a thickness 1.5 mm, referred to as P40.

1.1.2 Preparation of the fixture compression specimen, including:

a) cutting the silicone rubber heat conducting sheet P20 by using a circular cutting die with an inner diameter of 30 mm into round cakes, clamping the P20 round cakes between an upper rigid plate 4.1 and a lower rigid plate 4.3 (actually two 304 stainless steel plates) to serve as the target specimens 4.2, fastening the upper rigid plate 4.1 and the lower rigid plate 4.3 by using three metal screw rods 8, and respectively controlling the compression ratios of initial thicknesses of the three groups of target specimens 4.2 to 10%, 20% and 30%, wherein the total number of the target specimens 4.2 is not less than 432, that is, 3 parallel sub-specimens of thermal conductivityx3 groups of compression ratios×4 aging temperaturesx12 aging time points are used for testing an apparent thermal conductivity of a laminated combined test piece that clamps the target specimens 4.2 during an aging process;

b) cutting the silicone rubber heat conducting sheet P40 by using the circular cutting die with the inner diameter of 30 mm into round cakes, clamping the P40 round cakes between the upper rigid plate 4.1 and the lower rigid plate 4.3 (actually two copper plated nickel plates) to serve as the target specimens 4.2, fastening the upper rigid plate 4.1 and the lower rigid plate 4.3 by using three metal screw rods 8, and respectively controlling the compression ratios of the initial thicknesses of the three groups of target specimens 4.2 to 10%, 20% and 30%, wherein the total number of the target specimens 4.2 is not less than 432, that is, 3 parallel sub-specimens of thermal conductivityx3 groups of compression ratios×4 aging temperatures×12 aging time points are used for testing the apparent thermal conductivity of the laminated combined test piece that clamps the target specimens 4.2 during the aging process;

c) forming on a plane of the upper rigid plate 4.1 an annular ditch, in which at least six countersunk through holes arranged equally in the circumference are formed, wherein three holes are in immovable fitting with three metal screw rods 8 during the aging process, after the other three holes form immovable fitting with the other three engineering plastic screw rods 8 during the test process of the thermal conductivity after aging, the three metal screw rods 8 are removed, and the nuts 10 of the screw rods 8 should sink into the annular ditch;

d) forming at least six threaded holes, which are arranged equally in the circumference and are in immovable fitting with six screw rods 8, on the plane of the lower rigid plate 4.3; and

e) since the rigid plate is relatively small, it will not generate warping deformation under the experimental stress conditions of this embodiment, so the rigid plate does not need to be provided with a stiffener.

1.2 Aging test procedure

1.2.1 Aging equipment

In the first embodiment, four standard aging boxes conforming to the ISO 188 specification are used.
In the first embodiment, two DRL-III and DRL-V thermal conductivity instruments conforming to the ASTM D 5470 specification are used.

1.2.2 Aging procedure

In the first embodiment, four large temperature groups are used, each large group is divided into 12 intermediate aging time groups, the fixture compression specimens in each large group are respectively placed in four standard aging boxes conforming to the constant temperatures specified in Table 1 in advance, each fixture compression specimen in each compression ratio small group of each intermediate time group contains three parallel sub-specimens, and large temperature group, intermediate time group and compression ratio small group marks and operation circulation records are made for the fixture compression specimens in advance.

When the constant temperatures of the fixture compression specimens in each intermediate group reach the temperatures and times specified in Table 1, the fixture compression specimens in the intermediate group are taken out of the large group of high temperature aging boxes and are placed at room temperature for (16-96)h, wherein the fixture compression specimens are placed under standard inspection conditions for not less than 30 minutes, and apparent thermal conductivitys and entire heat resistance of the fixture compression specimens after aging in a five-layer coaxial laminated combined test piece are tested, in accordance with the method shown in Fig. 8; and the apparent thermal conductivity ($\lambda_a$) of the five-layer coaxial laminated combined test piece is converted into an equivalent thermal conductivity ($\lambda_{e4.2}$) of a corresponding diameter according to an equation (7) by using commercial software or an electronic calculation form, and then is converted into a bulk thermal conductivity ($\lambda_{n4.2}$) of a corresponding thickness, and the bulk thermal conductivity ($\lambda_{n4.2}$) of the corresponding thickness is extended to an intrinsic thermal conductivity ($\lambda$) by using the least square method according to Fig. 1.

1.2.3 Aging temperature and time

The aging temperature and the aging time in the first embodiment are executed according to Table 1.

Table 1   Plan on aging temperature and aging time

| Time sequence | 0 | 1 | 2 | 3 | 4 | 5 |
|---|---|---|---|---|---|---|
| Aging temperature T$_c$±1℃ | Continuous accumulative constant temperature time t, h | | | | | |
| 298 | 1min | 20min | 45min | 70min | 1.5 | 2.0 |
| 272 | 2min | 3.0 | 4.0 | 5.0 | 6.5 | 8.0 |

| 245 | 3min | 13.0 | 16.0 | 20.0 | 26.0 | 32.0 |
| 218 | 5min | 51.0 | 65.0 | 81.0 | 102 | 129 |

(Continue) Table 1    Plan on aging temperature and aging time

| Time sequence | 6 | 7 | 8 | 9 | 10 | 11 |
|---|---|---|---|---|---|---|
| Aging temperature $T_c \pm 1\,°C$ | Continuous accumulative constant temperature time t, h | | | | | |
| 298 | 2.5 | 3.0 | 4.0 | 5.0 | 6.5 | 8.0 |
| 272 | 10.0 | 13.0 | 16.0 | 20.0 | 27.0 | 33.0 |
| 245 | 41.0 | 51.0 | 65.0 | 81.0 | 103 | 129 |
| 218 | 163 | 205 | 258 | 325 | 410 | 516 |

1.3 Aging inspection objects of parameters

In the first embodiment, the intrinsic thermal conductivity is preferably used as an object for inspection, evaluation and prediction of the aging test, but the use is not limited thereto. Under the established conditions that are convenient for each laboratory test, any one of the apparent thermal conductivity, the equivalent thermal conductivity, the bulk thermal conductivity, and the intrinsic thermal conductivity can be selected.

1.4 Test and conversion of the thermal conductivity

1.4.1 Terms and Definitions

For the clarity of technical concepts, the present invention provides the following terms and definitions:

1) Apparent thermal conductivity ($\lambda_a$)

The total equivalent thermal conductivity of the laminated combined test piece is measured by using the DRL thermal conductivity instrument.
Intuitively, as shown in Fig. 5 and Fig. 6, an upper auxiliary elastic sheet 3 with a known thermal conductivity, a rigid target specimen 4.2 and a lower auxiliary elastic sheet 5 with a known thermal conductivity constitute a three-layer laminated combined test piece; and as shown in Fig. 7 and Fig. 8, the upper rigid plate 4.1, the target specimen 4.2 and the lower rigid plate 4.3 are tightened and compressed into a non-detachable combined specimen 4 at first, and then the upper auxiliary elastic sheet 3 with the known thermal conductivity, the non-detachable combined specimen 4 and the lower auxiliary elastic sheet 5 with the known thermal conductivity constitute a five-layer combined test piece; and the total equivalent thermal conductivity of the three-layer laminated combined test piece and the five-layer combined test piece, that is, the apparent thermal conductivity ($\lambda_a$), is measured by using the DRL thermal conductivity instrument.

2) Equivalent thermal conductivity ($\lambda_e$)

At a given temperature, pressure, thickness and any specimen diameter, the thermal conductivity of a single-layer target specimen 4.2 is measured by using the DRL thermal conductivity instrument.

3) Bulk thermal conductivity ($\lambda_n$)

The thermal conductivitys of the single-layer target specimen 4.2 with different thicknesses at a given temperature and pressure.

4) Intrinsic thermal conductivity ($\lambda$)

The thermal conductivity of the single-layer target specimen 4.2 at a given temperature and pressure that does not change with the size or the shape of the specimen.

Intuitively, as shown in Fig. 1, in the first embodiment, the DRL thermal conductivity instrument is used for testing the equivalent thermal conductivitys ($\lambda_e$) of homogeneous target specimens 4.2 with various thicknesses at the same pressure, after the equivalent thermal conductivitys ($\lambda_e$) are converted into bulk thermal conductivitys ($\lambda_n$) according to an equation (3), then plotting is performed by using the bulk thermal conductivitys ($\lambda_n$) as vertical coordinates and using the corresponding thicknesses ($\delta_{4.2}$) of the target specimens 4.2 as abscissas, linear fitting is performed by using the bulk thermal conductivitys ($\lambda_n$) corresponding to the thicknesses ($\delta_{4.2}$) greater than 0.75mm as specimens, and linear extension is performed to obtain an intersection of the thickness ($\delta_{4.2}$) tending to zero and the vertical coordinates, and the intersection is considered as the intrinsic thermal conductivity ($\lambda$).

5) No phase change

On an interface of the inside of the target specimen 4.2 and the laminated combined test piece, there is no new infinitesimal volume cavitation or no new infinitesimal volume air film during the aging process, or the influence of infinitesimal volume cavitations and infinitesimal volume air films is negligible.

6) Oscillation state

On the interface of the inside of the target specimen 4.2 and the laminated combined test piece, there are dispersive new infinitesimal volume cavitations and dispersive new infinitesimal volume air films within a certain period of time, or non-negligible influence of the infinitesimal volume cavitations and the infinitesimal volume air films are left and will change with time.

7) Micro-gasification

On the interface of the inside of the target specimen 4.2 and the laminated combined test piece, there are dispersive new infinitesimal volume cavitations and dispersive new infinitesimal volume air films all the time, or non-negligible influence of the infinitesimal volume cavitations and the infinitesimal volume air films is left.

8) Contact heat resistance

When discrete materials are in contact with each other, additional heat transfer resistance is generated on the contact interface.

9) Rated temperature

In a constant temperature environment for 20,000 hours of continuous service, when a physical, chemical and electrical property is reduced by half or doubled, the highest temperature it can withstand.

10) Standard inspection conditions

Laboratory ambient temperature (25±2)°C and relative humidity (55±15)%.

11) Phase state

When the temperature or pressure of a macro-scale material changes, one of physical states such as solid crystal transformation, solid-liquid mutual solution, solid-liquid-gas mutual solution, and solid-liquid-gas coexistence (micro-gasification) exists on the micro or meso scale.

1.4.2 Test method of the thermal conductivity

In the first embodiment, the thermal conductivity is tested by using the laminated combined test piece, including:

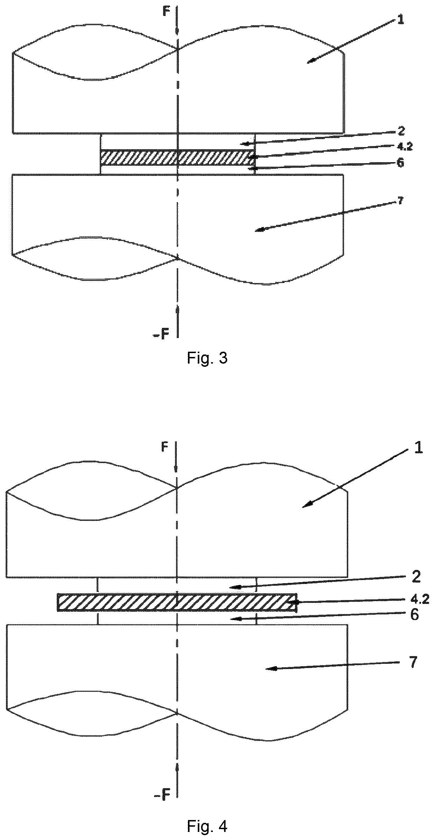

a) a single-layer specimen, as shown in Fig. 3 and Fig. 4, is used for measuring the equivalent thermal conductivity ($\lambda_e$) of an elastic specimen or a precisely made rigid standard specimen.

b) A three-layer specimen, as shown in Fig. 5 and Fig. 6, is used for calibrating the equivalent thermal conductivitys ($\lambda_e$) of the upper rigid plate 4.1 and the lower rigid plate 4.3 in Fig. 7 and Fig. 8 by using the upper auxiliary elastic sheet 3 and the lower auxiliary elastic sheet 5.

c) A five-layer specimen, as shown in Fig. 7 and Fig. 8, is used for measuring the apparent thermal conductivity ($\lambda_a$) of the non-detachable combined specimen 4 by using the upper auxiliary elastic sheet 3 and the lower auxiliary elastic sheet 5.

1.4.3 Conversion of the thermal conductivity

In the first embodiment, a conversion relationship of the thermal conductivitys between different definitions is proposed, including:

1) for the single-layer specimen, the equivalent thermal conductivity ($\lambda_e$) is corrected into the bulk thermal conductivity ($\lambda_n$)

As shown in Fig. 3 and Fig. 4, when the target specimen 4.2 is of the same material and thickness, but the diameter is inconsistent with the diameter of a thermoae pressure head 2 and a cold electrode pressure head 6 of the DRL thermal conductivity instrument, the deviation between the measured equivalent thermal conductivity ($\lambda_e$) and the bulk thermal conductivity ($\lambda_n$) will exceed an allowable random error range, and the equivalent thermal conductivity ($\lambda_e$) needs to be corrected into the bulk thermal conductivity ($\lambda_n$) by using an equation (3)

$$\lambda_e = \left(\frac{30}{\psi}\right)^2 \lambda_n$$

, single-layer specimen..................................................................... (3)

in the equation (3),

$\lambda_e$-the equivalent thermal conductivity of the target specimen 4.2, W/(m.K);

$\lambda_n$-the bulk thermal conductivity of the target specimen 4.2, W/(m.K);

$\Psi$-the diameter of the target specimen 4.2, mm; and

30-the diameter of the thermoae pressure head 2 and the cold electrode pressure head 6 of the DRL thermal conductivity instrument, mm.

2) for the three-layer specimen, the apparent thermal conductivity ($\lambda_a$) is converted into the equivalent thermal conductivity ($\lambda_e$)

As shown in Fig. 5 and Fig. 6, when the thicknesses of the upper auxiliary elastic sheet 3 and the lower auxiliary elastic sheet 5 are respectively $\delta_3$ and $\delta_5$, and are known, the corresponding diameters $\psi_3$ and $\psi_5$ are known, the corresponding bulk thermal conductivitys $\lambda_{n3}$ and $\lambda_{n5}$ are known, and the thickness $\delta_{4.2}$ and the diameter $\psi_{4.2}$ of the target specimen 4.2 to be tested are known, after the apparent thermal conductivity ($\lambda_a$) of the three-layer coaxial laminated combined test piece is measured, the bulk thermal conductivity $\lambda_{n4.2}$ of the target specimen 4.2 is calculated according to an equation (4) and an equation (5). According to the heat transfer theory:

$$\lambda_a = \cfrac{\delta_t}{\cfrac{\delta_3}{\left(\frac{30}{\psi_3}\right)^2 \lambda_{n3}} + \cfrac{\delta_{4.2}}{\left(\frac{30}{\psi_{4.2}}\right)^2 \lambda_{n4.2}} + \cfrac{\delta_5}{\left(\frac{30}{\psi_5}\right)^2 \lambda_{n5}}}$$

,the three-layer specimen.................(4)

in the equation (4),

$\lambda_a$-the apparent thermal conductivity of the three-layer material measured by the DRL thermal conductivity instrument, W/(m.K);

$\lambda_{n3}$-the bulk thermal conductivity of the upper layer elastic heat conducting sheet 3 with the known thickness $\delta_3$, W/(m.K);

$\lambda_{n4.2}$-the bulk thermal conductivity of the middle layer rigid target specimen 4.2 with the corresponding thickness $\delta_{4.2}$, W/(m.K);

$\lambda_{n5}$-the bulk thermal conductivity of the lower layer elastic heat conducting sheet 5 with the known thickness $\delta_5$, W/(m.K);

$\delta_3$-the known thickness of the upper layer elastic heat conducting sheet 3, m;

$\delta_4$-the known thickness of the middle layer rigid target specimen 4.2, m;

$\delta_5$-the known thickness of the lower layer elastic heat conducting sheet 5, m;

$\delta_t$-the total thickness of the three-layer coaxial laminated combined test piece, m;

30-the diameter of the thermoae pressure head 2 and the cold electrode pressure head 6 of the DRL thermal conductivity instrument, mm;

$\psi_3$-the known diameter of the upper layer elastic heat conducting sheet 3, m;

$\psi_{4.2}$-the known diameter of the middle layer rigid target specimen 4.2, m;

$\psi_5$-the known diameter of the lower layer elastic heat conducting sheet 5, m.

Phase shift transformation is performed on the equation (4) to obtain the equivalent thermal conductivity ($\lambda_{e4.2}$) of the target specimen 4.2:

$$\lambda_{e4.2} = \left(\frac{30}{\psi_{4.2}}\right)^2 \lambda_{n4.2}$$

$$= \cfrac{\delta_{4.2}}{\cfrac{\delta_t}{\lambda_a} - \cfrac{\delta_3}{\left(\frac{30}{\psi_3}\right)^2 \lambda_{n3}} - \cfrac{\delta_5}{\left(\frac{30}{\psi_5}\right)^2 \lambda_{n5}}} \qquad \text{,the three-layer specimen...............(5)}$$

in the equation (5), the test procedures and units specified by various symbols are consistent with those in the equation (4).

3) for the five-layer specimen, the apparent thermal conductivity ($\lambda_a$) is converted into the equivalent thermal conductivity ($\lambda_e$)

As shown in Fig. 7 and Fig. 8, when the thicknesses of the upper auxiliary elastic sheet 3 and the lower auxiliary elastic sheet 5 are respectively $\delta_3$ and $\delta_5$, and are known, the corresponding diameters $\psi_3$ and $\psi_5$ are known, the corresponding bulk thermal conductivitys $\lambda_{n3}$ and $\lambda_{n5}$ are known, the thicknesses of the upper rigid plate 4.1 and the lower rigid plate 4.3 are respectively $\delta_{4.1}$ and $\delta_{4.3}$, and are known, the corresponding diameters $\psi_{4.1}$ and $\psi_{4.3}$ are known, the corresponding bulk thermal conductivitys $\lambda_{n4.1}$ and $\lambda_{n4.3}$ are known, and the thickness $\delta_{4.2}$ and the diameter $\psi_{4.2}$ of the target specimen 4.2 to be tested are known, after the apparent thermal conductivity ($\lambda_a$) of the five-layer coaxial laminated combined test piece is measured, the bulk thermal conductivity $\lambda_{n4.2}$ of the target specimen 4.2 is calculated according to an equation (6) and an equation (7). According to the heat transfer theory, since the thermal conductivity of a nylon bolt 8 is only about 0.25 W/(mK), and a product of the thermal conductivity and a heat transfer area of the nylon bolt only accounts for less than four thousandths of the target specimen 4.2 to be tested, and the heat flow only accounts for less than one thousandth of the total heat flow, so the heat flow of the nylon bolt 8 is completely negligible

in mathematics, then:

$$\lambda_a = \cfrac{\delta_f}{\cfrac{\delta_3}{\left(\cfrac{30}{\psi_3}\right)^2 \lambda_{n3}} + \cfrac{\delta_{4.1}}{\left(\cfrac{30}{\psi_{4.1}}\right)^2 \lambda_{n4.1}} + \cfrac{\delta_{4.2}}{\left(\cfrac{30}{\psi_{4.2}}\right)^2 \lambda_{n4.2}} + \cfrac{\delta_{4.3}}{\left(\cfrac{30}{\psi_{4.3}}\right)^2 \lambda_{n4.3}} + \cfrac{\delta_5}{\left(\cfrac{30}{\psi_5}\right)^2 \lambda_{n5}}}$$

, the five-layer specimen...... (6)

in the equation (6),

$\lambda_a$ the apparent thermal conductivity of the five-layer material measured by the DRL thermal conductivity instrument, W/(m.K);
$\lambda_{n3}$ the bulk thermal conductivity of the upper auxiliary elastic sheet 3 with the known thickness $\delta_3$, W/(m.K);
$\lambda_{n4.1}$ the bulk thermal conductivity of the upper rigid plate 4.1 with the known thickness $\delta_{4.1}$, W/(m.K);
$\lambda_{n4.2}$ the bulk thermal conductivity to be tested of the target specimen 4.2 with the known thickness $\delta_{4.2}$, W/(m.K);
$\lambda_{n4.3}$ the bulk thermal conductivity of the lower rigid plate 4.3 with the known thickness $\delta_{4.3}$, W/(m.K);
$\lambda_{n5}$ the bulk thermal conductivity of the lower auxiliary elastic sheet 5 with the known thickness $\delta_5$, W/(m.K);
$\delta_3$ the known thickness of the upper auxiliary elastic sheet 3, m;
$\delta_{4.1}$ the known thickness of the upper rigid plate 4.1, m;
$\delta_{4.2}$ the known thickness of the target specimen 4.2, m;
$\delta_{4.3}$ the known thickness of the lower rigid plate 4.3, m;
$\delta_5$ the known thickness of the lower auxiliary elastic sheet 5, m;
$\delta_f$ the total thickness of the five-layer coaxial laminated combined test piece, m;
30-the diameter of the thermoae pressure head 2 and the cold electrode pressure head 6 of the DRL thermal conductivity instrument, m;
$\psi_3$ the known diameter of the upper auxiliary elastic sheet 3, m;
$\psi_{4.1}$ the known diameter of the upper rigid plate 4.1, m;
$\psi_{4.2}$ the known diameter of the target specimen 4.2, m;
$\psi_{4.3}$ the known diameter of the lower rigid plate 4.3, m; and
$\psi_5$ the known diameter of the lower auxiliary elastic sheet 5, m.

Phase shift transformation is performed on the equation (6) to obtain the equivalent thermal conductivity ($\lambda_{e4.2}$) of the target specimen 4.2:

$$\lambda_{e4.2} = \left(\frac{30}{\psi_{4.2}}\right)^2 \lambda_{n4.2}$$

$$= \cfrac{\delta_{4.2}}{\cfrac{\delta_f}{\lambda_a} - \cfrac{\delta_3}{\left(\cfrac{30}{\psi_3}\right)^2 \lambda_{n3}} - \cfrac{\delta_{4.1}}{\left(\cfrac{30}{\psi_{4.1}}\right)^2 \lambda_{n4.1}} - \cfrac{\delta_{4.3}}{\left(\cfrac{30}{\psi_{4.3}}\right)^2 \lambda_{n4.3}} - \cfrac{\delta_5}{\left(\cfrac{30}{\psi_5}\right)^2 \lambda_{n5}}}$$

, the five-layer specimen...... (7)

in the equation (7), the test procedures and units specified by various symbols are consistent with those in the equation (6).

1.5 Test results

When the test results of the thermal conductivity are expressed, any one of the apparent thermal conductivity ($\lambda_a$), the equivalent thermal conductivity ($\lambda_e$), the bulk thermal conductivity ($\lambda_n$), and the intrinsic thermal conductivity ($\lambda$) is used, although all are equivalent, in this embodiment, in order to unify the concept, the apparent thermal conductivity ($\lambda_a$), the equivalent thermal conductivity ($\lambda_e$) and the bulk thermal conductivity ($\lambda_n$) are collectively converted into the intrinsic thermal conductivity ($\lambda$) during the test process. It is only one of the manners of use, but is not a limitation of the use.

1.5.1 Initial thickness

In the first embodiment, the initial thicknesses of the upper auxiliary elastic sheet 3, the upper rigid plate 4.1, the target specimen 4.2, the lower rigid plate 4.3 and the lower auxiliary elastic sheet 5 in Fig. 3 to Fig 8 are shown in Table 2.

1.5.2 Initial thermal conductivity

In the first embodiment, the initial intrinsic thermal conductivitys of the upper auxiliary elastic sheet 3, the upper rigid plate 4.1, the target specimen 4.2, the lower rigid plate 4.3 and the lower auxiliary elastic sheet 5 in Fig. 5 to Fig. 8 are shown in Table 3.

Table 2 Measurement results of initial thickness

| Fixture compression specimen | Upper rigid plate Lower rigid plate | Upper rigid plate Lower rigid plate | Upper rigid plate Lower rigid plate | Upper auxiliary elastic sheet Lower auxiliary elastic sheet | Target specimen | |
|---|---|---|---|---|---|---|
| Material | 304 Stainless steel A | 304 Stainless steel B | Copper nickel plated | P40 | P20 | P40 |
| Initial thickness mm | 4.06+0.02 | 4.06+0.02 | 4.04+0.02 | 0.69+0.02 | 2.53+0.02 | 1.57+0.02 |

Table 3 Measurement results of initial intrinsic thermal conductivity

| Fixture compression specimen | Upper rigid plate Lower rigid plate | Upper rigid plate Lower rigid plate | Upper rigid plate Lower rigid plate | Upper auxiliary elastic sheet Lower auxiliary elastic sheet | Target specimen | |
|---|---|---|---|---|---|---|
| Material | 304 Stainless steel A | 304 Stainless steel B | Copper nickel plated | P40 | P20 | P40 |
| Initial Intrinsic thermal conductivity $\lambda$, W/(m.K) | 15.0+1.4 | 50.9+4.6 | 290+26 | 4.18+0.24 | 2.18+0.13 | 4.18+0.24 |

1.5.3 Thermal conductivity after aging

In the first embodiment, the measured values of the intrinsic thermal conductivity changing with the aging time under constant temperature aging at 298°C and under three compression ratios of 10%, 20% and 30% of the fixture are listed in Table 4;

in the first embodiment, the measured values of the intrinsic thermal conductivity changing with the aging time under constant temperature aging at 272°C and under three compression ratios of 10%, 20% and 30% of the fixture are listed in Table 5;

in the first embodiment, the measured values of the intrinsic thermal conductivity changing with the aging time under constant temperature aging at 245°C and under three compression ratios of 10%, 20% and 30% of the fixture are listed in Table 6; and

in the first embodiment, the measured values of the intrinsic thermal conductivity changing with the aging time under constant temperature aging at 218°C and under three compression ratios of 10%, 20% and 30% of the fixture are listed in Table 7.

1.6 Establishment of a thermal conductivity equation (1.1)

1.6.1 T inspection

The purpose of carrying out the T inspection in the first embodiment is to investigate the difference of degrees of influence of P20 or P40 on thermal conductivity aging under the conditions of three compression ratios of 10%, 20% and 30%, respectively.
The T inspection results before aging are listed in Table 8.
The T inspection results after aging are listed in Table 9.
It can be seen from Table 8 that, under the three compression ratios of the fixture, the T values of P20 before aging are all greater than a difference boundary, which indicates that the influence of the three compression ratios on the thermal conductivity is quite different, and the measured values belong to different specimens groups. Under the three compression ratios of the fixture, one of the T values of P40 before aging is less than the difference boundary, that is, there is almost no significant difference under the compression ratios of 10% and 30%, which indicates that the two groups of data can be equivalent to the same specimen, and it also shows that P40 has better compression resistance than P20.
It can be seen from Table 9 that, under the three compression ratios of the fixture, the T values of P20 after aging are all slightly greater than the difference boundary, and two of the T values of P40 before aging are less than the difference boundary, that is, the P40 is respectively compressed by 10% and 20%, and 20% and 30%. This indicates that after the aging of P20 and P40, the difference of different compression ratios is negligible, the main difference comes from the aging effects of high temperature and time, and all the specimen data of the three compression ratios can be combined into specimens with the same compression ratio.
Therefore, during the process of fitting and predicting the long-term aging trends, the data of the three compression ratios of 10%, 20% and 30% can be merged into a group of average values for observation and processing.
Since the T inspection results of aging at the other three temperatures are consistent with the above conclusions, in order to simplify the length, the T inspection results before and after aging at the other three temperatures are omitted in the first embodiment.

Table 4 Changes in the intrinsic thermal conductivity with the aging time at 298℃ W/(m.K)

| 298℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.02 | 0.17 | 0.33 | 0.75 | 1.17 | 1.50 |

| | | | | | | | | | | |
|---|---|---|---|---|---|---|---|---|---|---|
| P20 | Oscillation state, 10% | 3 | 2.18 | - | 2.02 | - | 1.75 | 0.91 | 0.87 | 1.22 |
| | Oscillation state, 20% | 3 | 2.18 | - | 2.12 | - | 1.79 | 1.06 | 1.09 | 0.75 |
| | Oscillation state, 30% | 3 | 2.18 | - | 2.15 | - | 1.83 | 1.01 | 1.04 | 1.34 |
| | Oscillation state,10-30% average | 9 | 2.18 | - | 2.09 | - | 1.79 | 0.99 | 1.00 | 1.10 |
| | Oscillation state, predicted in the equation (1.1) | 9 | 2.18 | 1.65 | 1.01 | 1.36 | 1.50 | 1.14 | 1.19 | 1.05 |
| P40 | Oscillation state,10% | 3 | 4.18 | - | 2.78 | - | 2.65 | 2.05 | 2.39 | 2.79 |
| | Oscillation state,20% | 3 | 4.18 | - | 2.80 | - | 3.18 | 2.12 | 2.68 | 3.05 |
| | Oscillation state,30% | 3 | 4.18 | - | 3.72 | - | 2.49 | 2.40 | 2.55 | 2.79 |
| | Oscillation state, 10-30% average | 9 | 4.18 | - | 3.10 | - | 2.77 | 2.19 | 2.54 | 2.87 |
| | Oscillation state, predicted in the equation (1.1) | 9 | 4.18 | 3.44 | 2.79 | 3.10 | 2.24 | 2.80 | 2.15 | 3.00 |

(continue) Table 4 Changes in the intrinsic thermal conductivity with the aging time at

298℃ W/(m.K)

| 298℃ Model number | Phase state, compression ratio | The number of sub-specimens | Aging time t, h | | | | | | | |
|---|---|---|---|---|---|---|---|---|---|---|
| | | | 2.00 | 2.50 | 3.00 | 4.00 | 5.00 | 6.50 | 8.00 | 650 |
| P20 | Oscillation state,10% | 3 | 0.58 | 0.31 | 0.73 | 0.25 | 0.53 | 0.55 | 0.45 | - |
| | Oscillation state, 20% | 3 | 0.75 | 0.97 | 0.31 | 0.30 | 0.63 | 0.48 | 0.55 | - |
| | Oscillation state, 30% | 3 | 1.03 | 0.22 | 0.45 | 0.28 | 0.63 | 0.45 | 0.53 | - |
| | Oscillation state, 10-30% average | 9 | 0.79 | 0.50 | 0.49 | 0.27 | 0.59 | 0.49 | 0.51 | - |
| | Oscillation state, predicted in the equation (1.1) | 9 | 0.88 | 0.72 | 0.66 | 0.29 | 0.58 | 0.51 | 0.49 | 0.47 |
| P40 | Oscillation state,10% | 3 | 1.72 | 1.79 | 2.00 | 1.94 | 1.98 | 2.56 | 2.36 | - |
| | Oscillation state, 20% | 3 | 1.84 | 2.43 | 2.20 | 2.14 | 2.38 | 2.71 | 2.38 | - |
| | Oscillation state, 30% | 3 | 2.55 | 2.66 | 2.87 | 2.31 | 2.08 | 2.74 | 2.10 | - |
| | Oscillation state, 10-30% average | 9 | 2.04 | 2.29 | 2.36 | 2.13 | 2.14 | 2.67 | 2.28 | - |
| | Oscillation state, predicted in the equation | 9 | 2.32 | 2.00 | 2.71 | 1.99 | 2.52 | 2.60 | 2.25 | 0.96 |

| | (1.1) | | | | | | | | | |
|---|---|---|---|---|---|---|---|---|---|---|
| | | | | | | | | | | |

Table 5  Changes in the intrinsic thermal conductivity with the aging time at 272℃ W/(m.K)

| 272℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.0 | 1.5 | 3.0 | 4.0 | 5.0 | 6.5 |
| P20 | Oscillation state, 10% | 3 | 2.18 | - | 1.80 | - | 0.44 | 0.80 | 0.49 | 0.45 |
| | Oscillation state, 20% | 3 | 2.18 | - | 2.27 | - | 0.69 | 0.99 | 0.46 | 0.38 |
| | Oscillation state, 30% | 3 | 2.18 | - | 2.25 | - | 0.66 | 0.93 | 0.54 | 0.56 |
| | Oscillation state, 10-30% average | 9 | 2.18 | - | 2.11 | - | 0.60 | 0.91 | 0.49 | 0.46 |
| | Oscillation state, predicted in the equation (1.1) | 9 | 2.18 | 1.68 | 1.40 | 1.67 | 1.22 | 0.92 | 0.32 | 0.70 |
| P40 | Oscillation state, 10% | 3 | 4.18 | - | 3.57 | - | 3.21 | 2.46 | 2.22 | 2.64 |
| | Oscillation state, 20% | 3 | 4.18 | - | 3.54 | - | 3.07 | 2.61 | 2.89 | 2.73 |
| | Oscillation state, 30% | 3 | 4.18 | - | 3.24 | - | 3.75 | 3.92 | 3.12 | 2.93 |
| | Oscillation state, 10-30% average | 9 | 4.18 | - | 3.45 | - | 3.34 | 3.00 | 2.75 | 2.77 |

| | | | | | | | | | | |
|---|---|---|---|---|---|---|---|---|---|---|
| Oscillation state, predicted in the equation (1.1) | 9 | 4.18 | 3.50 | 3.13 | 3.14 | 3.49 | 3.05 | 2.27 | 2.38 | |

(Continue) Table 5　Changes in the intrinsic thermal conductivity with the aging time at 272℃　W/(m.K)

| 272℃ Model number | Phase state, compression ratio | The number of sub-specimens | Aging time t, h | | | | | | | |
|---|---|---|---|---|---|---|---|---|---|---|
| | | | 8.0 | 10.0 | 13.0 | 16.0 | 20.0 | 27.0 | 33.0 | 650 |
| P20 | Oscillation state, 10% | 3 | 0.38 | 0.35 | 0.38 | 0.31 | 0.54 | 0.56 | 0.46 | - |
| | Oscillation state, 20% | 3 | 0.44 | 0.46 | 0.47 | 0.46 | 0.65 | 0.49 | 0.56 | - |
| | Oscillation state, 30% | 3 | 0.54 | 0.28 | 0.53 | 0.50 | 0.64 | 0.47 | 0.55 | - |
| | Oscillation state, 10-30% average | 9 | 0.45 | 0.36 | 0.46 | 0.42 | 0.61 | 0.51 | 0.52 | - |
| | Oscillation state, predicted in the equation (1.1) | 9 | 0.80 | 0.67 | 0.43 | 0.56 | 0.50 | 0.50 | 0.48 | 0.47 |
| P40 | Oscillation state, 10% | 3 | 2.41 | 2.42 | 2.54 | 2.27 | 2.04 | 2.66 | 2.44 | - |
| | Oscillation state, 20% | 3 | 2.58 | 2.45 | 2.58 | 2.24 | 2.45 | 2.82 | 2.46 | - |
| | Oscillation state, 30% | 3 | 2.57 | 3.35 | 3.54 | 2.88 | 2.16 | 2.85 | 2.17 | - |
| | Oscillation state, | 9 | 2.52 | 2.74 | 2.89 | 2.46 | 2.22 | 2.78 | 2.35 | - |

| | 10-30% average | | | | | | | | | | |
|---|---|---|---|---|---|---|---|---|---|---|---|
| | Oscillation state, predicted in the equation (1.1) | 9 | | 2.71 | 2.41 | 3.08 | 2.39 | 2.40 | 2.67 | 2.30 | 0.96 |

Table 6  Changes in the intrinsic thermal conductivity with the aging time at 245℃ W/(m.K)

| 245℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.1 | 6.5 | 13 | 16 | 20 | 26 |
| P20 | Oscillation state, 10% | 3 | 2.18 | - | 2.31 | - | 1.77 | 0.71 | 0.69 | 0.71 |
| | Oscillation state, 20% | 3 | 2.18 | - | 2.38 | - | 1.95 | 0.76 | 0.78 | 0.66 |
| | Oscillation state, 30% | 3 | 2.18 | - | 2.47 | - | 1.92 | 0.73 | 0.78 | 0.90 |
| | Oscillation state, 10-30% average | 9 | 2.18 | - | 2.39 | - | 1.88 | 0.73 | 0.75 | 0.76 |
| | Oscillation state, predicted in the equation (1.1) | 9 | 2.18 | 1.72 | 1.41 | 1.49 | 2.19 | 1.58 | 1.92 | 1.75 |
| P40 | Oscillation state, 10% | 3 | 4.18 | - | 4.06 | - | 2.99 | 2.97 | 3.83 | 3.81 |
| | Oscillation state, 20% | 3 | 4.18 | - | 4.03 | - | 3.33 | 2.65 | 3.68 | 2.74 |
| | Oscillation | 3 | 4.18 | - | 4.77 | - | 3.90 | 3.74 | 3.40 | 2.81 |

| | | | | | | | | | | |
|---|---|---|---|---|---|---|---|---|---|---|
| P40 | state, 30% | | | | | | | | | |
| | Oscillation state, 10-30% average | 9 | 4.18 | - | 4.29 | - | 3.41 | 3.12 | 3.64 | 3.12 |
| | Oscillation state, predicted in the equation (1.1) | 9 | 4.18 | 3.56 | 2.99 | 3.26 | 3.45 | 3.14 | 3.02 | 2.67 |

(Continue) Table 6　Changes in the intrinsic thermal conductivity with the aging time at 245℃　W/(m.K)

| 245℃ Model number | Phase state, compression ratio | The number of sub-specimens | Aging time t, h | | | | | | | |
|---|---|---|---|---|---|---|---|---|---|---|
| | | | 32 | 41 | 51 | 65 | 81 | 103 | 129 | 650 |
| P20 | Oscillation state, 10% | 3 | 0.57 | 0.56 | 0.40 | 0.53 | 0.50 | 0.51 | 0.43 | - |
| | Oscillation state, 20% | 3 | 0.77 | 0.60 | 0.44 | 0.54 | 0.59 | 0.45 | 0.52 | - |
| | Oscillation state, 30% | 3 | 0.57 | 0.67 | 0.48 | 0.76 | 0.59 | 0.43 | 0.50 | - |
| | Oscillation state, 10-30% average | 9 | 0.64 | 0.61 | 0.44 | 0.61 | 0.56 | 0.46 | 0.48 | - |
| | Oscillation state, predicted in the equation (1.1) | 9 | 0.73 | 0.52 | 0.66 | 0.56 | 0.56 | 0.51 | 0.50 | 0.47 |

| | Oscillation state, 10% | 3 | 2.86 | 2.73 | 2.37 | 2.48 | 2.09 | 2.37 | 2.22 | - |
|---|---|---|---|---|---|---|---|---|---|---|
| P40 | Oscillation state, 20% | 3 | 3.16 | 2.68 | 2.25 | 2.48 | 2.22 | 2.50 | 2.23 | - |
| | Oscillation state, 30% | 3 | 3.34 | 3.00 | 2.07 | 2.91 | 1.92 | 2.54 | ±1.96 | - |
| | Oscillation state, 10-30% average | 9 | 3.12 | 2.80 | 2.23 | 2.63 | 2.08 | 2.47 | 2.14 | - |
| | Oscillation state, predicted in the equation (1.1) | 9 | 3.31 | 2.85 | 2.47 | 2.41 | 2.34 | 2.42 | 2.21 | 0.99 |

Table 7 Changes in the intrinsic thermal conductivity with the aging time at 218℃ W/(m.K)

| 218℃ Model number | Phase state, compression ratio | The Number of sub-specimens | Aging time t, h | | | | | | | |
|---|---|---|---|---|---|---|---|---|---|---|
| | | | 0.0⊖ | 0.0⊕ | 0.10 | 26 | 51 | 65 | 81 | 102 |
| P20 | Oscillation state, 10% | 3 | 2.18 | - | 1.94 | - | 1.07 | 1.40 | 1.62 | 1.02 |
| | Oscillation state, 20% | 3 | 2.18 | - | 2.00 | - | 1.25 | 1.41 | 1.77 | 1.18 |
| | Oscillation state, 30% | 3 | 2.18 | - | 2.14 | - | 1.26 | 1.58 | 1.59 | 1.27 |
| | Oscillation state, 10-30% average | 9 | 2.18 | - | 2.03 | - | 1.19 | 1.47 | 1.66 | 1.16 |
| | Oscillation state, predicted | 9 | 2.18 | 1.76 | 2.02 | 1.97 | 1.67 | 1.14 | 2.42 | 1.50 |

| | in the equation (1.1) | | | | | | | | | |
|---|---|---|---|---|---|---|---|---|---|---|
| P40 | Oscillation state, 10% | 3 | 4.18 | - | 3.44 | - | 3.18 | 3.29 | 3.09 | 3.20 |
| | Oscillation state, 20% | 3 | 4.18 | - | 3.56 | - | 3.33 | 3.31 | 3.83 | 2.91 |
| | Oscillation state, 30% | 3 | 4.18 | - | 3.99 | - | 3.20 | 3.23 | 3.74 | 3.76 |
| | Oscillation state, 10-30% average | 9 | 4.18 | - | 3.66 | - | 3.23 | 3.27 | 3.55 | 3.29 |
| | Oscillation state, predicted in the equation (1.1) | 9 | 4.18 | 3.63 | 4.28 | 4.12 | 3.58 | 3.95 | 3.28 | 3.13 |

(Continue) Table 7   Changes in the intrinsic thermal conductivity with the aging time at 218℃  W/(m.K)

| 218℃ Model number | Phase state, compression ratio | The number of sub-specimens | Aging time t, h | | | | | | | |
|---|---|---|---|---|---|---|---|---|---|---|
| | | | 129 | 163 | 205 | 258 | 325 | 410 | 516 | 700 |
| P20 | Oscillation state, 10% | 3 | 0.99 | 1.08 | 0.95 | 1.36 | 0.62 | 0.57 | 0.46 | - |
| | Oscillation state, 20% | 3 | 1.33 | 1.16 | 1.39 | 1.50 | 0.58 | 0.84 | 0.73 | - |
| | Oscillation state, 30% | 3 | 1.09 | 1.25 | 1.19 | 1.47 | 0.78 | 0.60 | 0.84 | - |
| | Oscillation state, 10-30% average | 9 | 1.14 | 1.16 | 1.18 | 1.44 | 0.66 | 0.67 | 0.67 | - |
| | Oscillation state, | 9 | 1.51 | 1.18 | 1.39 | 1.43 | 1.01 | 0.94 | 0.80 | 0.66 |

| | predicted in the equation (1.1) | | | | | | | | | |
|---|---|---|---|---|---|---|---|---|---|---|
| P40 | Oscillation state, 10% | 3 | 2.96 | 3.06 | 3.14 | 2.81 | 2.74 | 2.61 | 2.79 | - |
| | Oscillation state, 20% | 3 | 3.15 | 2.90 | 2.81 | 2.85 | 3.05 | 2.42 | 2.63 | - |
| | Oscillation state, 30% | 3 | 3.54 | 3.08 | 3.42 | 3.25 | 2.90 | 2.94 | 2.88 | - |
| | Oscillation state, 10-30% average | 9 | 3.22 | 3.01 | 3.12 | 2.97 | 2.90 | 2.65 | 2.77 | - |
| | Oscillation state, predicted in the equation (1.1) | 9 | 3.69 | 3.00 | 3.22 | 3.04 | 2.91 | 2.70 | 2.68 | 2.34 |

1.6.2 Parameter fitting in equation (1.1)

One of the use of the algorithm of the present invention is to replace a general symbol (P) of the physical, chemical and electrical properties in the equation (1) with a specific symbol ($\lambda$) of the intrinsic thermal conductivity, so as to convert the equation (1) into an equation (1.1):

$$\lambda_t = \lambda_\infty + \left\{ \lambda_{0\ominus} + \left[ \Delta\lambda_1 e^{-k_1 t} \times Sin\beta_1 \left(\frac{t}{t_0} - 1\right)^{\theta_1} \pi + \Delta\lambda_2 e^{-k_2 t} \times + Sin\beta_2 \left(\frac{t}{t_0} - 1\right)^{\theta_2} \pi + \Delta\lambda_3 e^{-k_3 t} \right] - \lambda_\infty \right\} e^{-kt} \dots\dots\dots (1.1)$$

in the equation (1.1),

$\lambda$-the intrinsic thermal conductivity of the target specimen, and the measured values are listed in Table 4 to Table 7 for fitting and verification;

$\lambda_t$-the intrinsic thermal conductivity of the target specimen at any specified constant temperature for any service time, a predicted value;

$\lambda_\infty$-the intrinsic thermal conductivity after weathering for more than 100 years, determined by numerical simulation of a material formula, or a fitted value;

$\lambda_{0\ominus}$-initial intrinsic thermal conductivity before aging, a measured value;

$\lambda_{0\oplus}$-the intrinsic thermal conductivity during micro-gasification expansion; a fitted value;

Sin -micro-gasification oscillation trigonometric function;

$\Delta\lambda_1$-micro-gasification internal influence parameter, $\Delta\lambda_1 = (\lambda_{0\oplus} - \lambda_{0\ominus})$;

$\Delta\lambda_2$-micro-gasification interface influence parameter, a fitted value;

$\Delta\lambda_3$-mechanical stress influence parameter, a fitted value;

t-aging time or service time, determined by a specified time or an accumulative number of cycles;

$t_0$-migration lag time of low molecular substances, a fitted value;

$\beta_1$-migration oscillation frequency coefficient, a fitted value;

$\beta_2$-volatilization oscillation frequency coefficient, a fitted value;

$k_1$-migration rate parameter, a fitted value;

$k_2$-volatilization rate parameter, a fitted value;

$k_3$-relaxation rate parameter, a fitted value;

k-chemical reaction rate parameter, a fitted value;

$\theta_1$-migration oscillation frequency index, a fitted value; and

$\theta_2$-volatilization oscillation frequency index, a fitted value.

In the formula (1.1) in the first embodiment, although the fifteen parameters including the thermal conductivity are difficult to be obtained by linear fitting, the average values of the measured values under three compression ratios in Table 4 to Table 7 are taken as specimens, tentatively starting with "Q=0" assignment, and with a step pitch value as small as possible, the parameter is repeatedly and iteratively input into the equation (1.1) by using a parallax method, after each parameter is iterated for more than 50 times, the standard deviation of a difference value between a calculated value ($\lambda_t$) and a measured value ($\lambda$) converges to the minimum, optimal values of the fifteen parameters "Q" of the thermal conductivity at each temperature are obtained, and the results of iterative optimization of P20 and P40 are listed in Table 10 and Table 11. Because of a mathematical frequency doubling effect, when there is more than one optimal value among the fitted values of the obtained fifteen parameters, only the smaller group of fifteen "Q" values closest to "1 time" is selected as optimal parameters.

Table 8    The intrinsic thermal conductivity, T inspection before aging

| Before 218°C Model number | Compression group | T value of the fixture under three compression ratios in use timing sequence | | | | | | Average value | Unilateral P0.975 boundary | | n=3, unilateral α=0.025 difference T value boundary |
|---|---|---|---|---|---|---|---|---|---|---|---|
| | | 0.08 | 51 | 65 | 81 | 102 | 129 | | | | |
| P20 | 10%/20% | 2.28 | 1.18 | 2.94 | 4.60 | 2.21 | 8.18 | 5.36 | 12.68 | ≥ | 3.182 |
| | 20%/30% | 1.61 | 7.67 | 0.20 | 1.02 | 0.78 | 0.18 | 1.57 | 5.64 | ≥ | 3.182 |
| | 10%/30% | 3.21 | 2.87 | 1.38 | 4.71 | 0.97 | 5.10 | 4.90 | 11.32 | ≥ | 3.182 |
| P40 | 10%/20% | 0.88 | 0.97 | 1.22 | 2.29 | 0.27 | 4.27 | 1.77 | 4.81 | ≥ | 3.182 |
| | 20%/30% | 0.24 | 0.53 | 1.27 | 0.10 | 2.28 | 2.45 | 1.23 | 3.27 | ≥ | 3.182 |
| | 10%/30% | 1.00 | 0.14 | 0.13 | 1.56 | 1.06 | 2.44 | 1.08 | 2.82 | < | 3.182 |

(Continue) Table 8    The intrinsic thermal conductivity, T inspection before aging

| Before 218°C Model number | Compression group | T value of the fixture under three compression ratios in use timing sequence | | | | | | Average value | Unilateral P0.975 boundary | | n=3, unilateral α=0.025 difference T value boundary |
|---|---|---|---|---|---|---|---|---|---|---|---|
| | | 163 | 205 | 258 | 325 | 410 | 516 | | | | |
| P20 | 10%/20% | 5.70 | 2.39 | 4.92 | 5.62 | 11.4 | 12.9 | 5.36 | 12.68 | ≥ | 3.182 |
| | 20%/30% | 2.82 | 1.43 | 0.04 | 0.40 | 1.49 | 1.21 | 1.57 | 5.64 | ≥ | 3.182 |
| | 10%/30% | 5.94 | 2.95 | 4.60 | 5.54 | 12.8 | 8.75 | 4.90 | 11.32 | ≥ | 3.182 |
| P40 | 10%/20% | 1.12 | 1.46 | 0.53 | 4.74 | 0.22 | 3.34 | 1.77 | 4.81 | ≥ | 3.182 |
| | 20%/30% | 3.30 | 1.29 | 0.27 | 0.13 | 1.33 | 1.57 | 1.23 | 3.27 | ≥ | 3.182 |

| | 10%/30% | 0.53 | 0.27 | 0.07 | 2.34 | 1.31 | 2.17 | 1.08 | 2.82 | < | 3.182 |

Table 9   The intrinsic thermal conductivity, T inspection after aging

| After 218℃ Model number | Compressi-on group | T value of the fixture under three compression ratios in use timing sequence | | | | | | Aver-age value | Unilateral P0.975 boundary | | n=3, unilateral α=0.02 difference T value boundary |
|---|---|---|---|---|---|---|---|---|---|---|---|
| | | 0.08 | 51 | 65 | 81 | 102 | 129 | | | | |
| P20 | 10%/20% | 5.20 | 0.92 | 0.14 | 0.77 | 1.81 | 2.33 | 2.80 | 9.36 | ≥ | 3.182 |
| | 20%/30% | 0.77 | 0.06 | 0.78 | 0.90 | 0.54 | 1.48 | 0.73 | 1.76 | < | 3.182 |
| | 10%/30% | 2.70 | 0.88 | 0.83 | 0.15 | 1.43 | 0.75 | 3.47 | 12.72 | ≥ | 3.182 |
| P40 | 10%/20% | 0.26 | 0.44 | 0.05 | 3.83 | 0.66 | 1.44 | 0.96 | 3.13 | < | 3.182 |
| | 20%/30% | 1.79 | 0.56 | 0.19 | 0.31 | 3.09 | 2.09 | 1.23 | 3.01 | < | 3.182 |
| | 10%/30% | 3.65 | 0.06 | 0.13 | 2.88 | 1.13 | 2.61 | 1.55 | 4.21 | ≥ | 3.182 |

(Continue) Table 9   The intrinsic thermal conductivity, T inspection after aging

| After 218℃ Model number | Compressi-on group | T value of the fixture under three compression ratios in use timing sequence | | | | | | Avera-ge value | Unilateral P0.975 boundary | | n=3,unilateral α=0.025 differenc-e T value boundary |
|---|---|---|---|---|---|---|---|---|---|---|---|
| | | 163 | 205 | 258 | 325 | 410 | 516 | | | | |
| P20 | 10%/20% | 0.90 | 3.20 | 1.45 | 2.58 | 12.5 | 1.74 | 2.80 | 9.36 | ≥ | 3.182 |
| | 20%/30% | 0.73 | 1.89 | 0.40 | 0.07 | 0.73 | 0.36 | 0.73 | 1.76 | < | 3.182 |
| | 10%/30% | 1.44 | 1.86 | 1.82 | 2.91 | 14.1 | 12.7 | 3.47 | 12.72 | ≥ | 3.182 |
| P40 | 10%/20% | 0.85 | 2.28 | 0.15 | 0.65 | 0.91 | 0.05 | 0.96 | 3.13 | < | 3.182 |

| | | | | | | | | | | |
|---|---|---|---|---|---|---|---|---|---|---|
| 20%/30% | 1.34 | 2.10 | 0.96 | 0.81 | 0.19 | 1.36 | 1.23 | 3.01 | < | 3.182 |
| 10%/30% | 3.55 | 0.91 | 1.05 | 0.00 | 0.50 | 2.15 | 1.55 | 4.21 | ≥ | 3.182 |

1.6.3 Constant fitting in equation (2.1)

In the first embodiment, for the fifteen parameters in the thermal conductivity equation (1.1) contained in Table 10 and Table 11, on mechanism, each parameter does not change with time, but only changes with temperature. Each parameter that changes with temperature further includes constants expressed by corresponding three symbols "A, B and C" in the equation (2); and referring to Table 10 and Table 11, during the fitting process, the constants, which are corresponding to the parameters in the thermal conductivity equation (1.1), in the equation (2) need to be replaced with corresponding symbols, so as to convert the equation (2) into an equation (2.1):

$$\ln Q = \frac{A}{T+C} + B \ldots\ldots\ldots\ldots\ldots\ldots\ldots\ldots\ldots\ldots\ldots\ldots\ldots\ldots\ldots\ldots\ldots\ldots\ldots\ldots\ldots（2.1）$$

in the equation (2.1),

Q-replaced into any one of the corresponding fifteen parameters in the equation (1.1) at any temperature environment;

A-empirical constant of each parameter associated with the reaction activation energy and the diffusion activation energy of multiple components, a fitted value, K;

B-empirical constant of each parameter associated with the chemical reaction rate and the diffusion rate of multiple components, a fitted value, dimensionless;

C-conformal constant of Fourier series transformation of each parameter associated with the activation energy of multiple components, a fitted value, K;

T-absolute temperature, specified constant temperature +273.15, K;

"Q" in the equation (2.1) is replaced with one of the fifteen parameters in Table 10 and Table 11, plotting is performed by respectively using the logarithms of the fifteen parameters as vertical coordinates and using 1/(T+C) as abscissas, tentatively starting with "C=0" assignment, repeated iteration is performed by using a least square method electronic calculation program or the parallax method, and different "C" values are input, when $R^2$ automatically output by the computer program system is ≥ 0.990, it is considered that the line has been a straight line, and "A, B, C" and $R^2$ in one-to-one correspondence with the fifteen parameters are optimal values thereof, which are listed in Table 10 and Table 11, respectively.

Table 10  Parameter values of the thermal conductivity of P20 in the equation (1.1)

| Serial number | P20,parameters in the micro-gasification expansion oscillation equation (1) | Parameter values of various temperatures | | | |
|---|---|---|---|---|---|
| | | 298°C | 272°C | 245°C | 218°C |
| 1 | $\lambda_\infty$ thermal conductivity after weathering for more than 100 years, W/(m.K) | 0.470 | 0.470 | 0.470 | 0.470 |
| 2 | $t_0$ migration lag time of low molecular substances, h | 1.71 | 2.11 | 2.68 | 3.40 |
| 3 | $\Delta\lambda_1$ micro-gasification internal influence constant, % | -0.530 | -0.495 | -0.457 | -0.420 |
| 4 | $\Delta\lambda_2$ micro-gasification interface influence constant, % | -5.000 | -3.000 | -1.700 | -0.900 |
| 5 | $\Delta\lambda_3$ mechanical stress influence constant, % | -0.020 | -0.020 | -0.020 | -0.020 |
| 6 | $\lambda_{0\oplus}$ thermal conductivity in micro-gasification phase state, W/(m.K) | 1.650 | 1.685 | 1.723 | 1.760 |
| 7 | $\lambda_{0\ominus}$ initial intrinsic thermal conductivity before aging, W/(m.K) | 2.18 | 2.18 | 2.18 | 2.18 |
| 8 | $\beta_1$ migration oscillation frequency coefficient, dimensionless | 5.700 | 4.100 | 2.880 | 1.980 |
| 9 | $\beta_2$ volatilization oscillation frequency coefficient, dimensionless | 0.112 | 0.103 | 0.092 | 0.082 |
| 10 | $k_1$ migration rate constant, 1/h | 3.00E-03 | 5.50E-04 | 8.00E-05 | 1.20E-05 |
| 11 | $k_2$ volatilization rate constant, 1/h | 1.23E-02 | 1.12E-02 | 9.95E-03 | 8.80E-03 |
| 12 | $k_3$ relaxation rate constant, 1/h | 1.00E-06 | 1.00E-06 | 1.00E-06 | 1.00E-06 |
| 13 | k chemical reaction rate constant, 1/h | 7.10E-01 | 1.68E-01 | 2.80E-02 | 2.80E-03 |
| 14 | $\theta_1$ migration oscillation frequency index, dimensionless | 5.30E+00 | 3.14E+00 | 1.74E+00 | 9.21E-01 |
| 15 | $\theta_2$ volatilization oscillation | 4.3 | 2.9 | 1.9 | 1.2 |

| | frequency index, dimensionless | | | | |
|---|---|---|---|---|---|

(Continue) Table 10   Parameter and constant values of the thermal conductivity of P20 in the equation (1.1)

| Serial number | P20, parameters in the micro-gasification expansion oscillation equation (1) | Constant values of various parameters | | | | |
|---|---|---|---|---|---|---|
| | | General expression formula | A | B | C | $R^2$ |
| 1 | $\lambda_\infty$ heat conductivity coefficient after weathering for more than 100 years, W/(m.K) | $\ln \lambda_\infty = \dfrac{A}{T+C} + B$ | 0.00 | -0.7550 | 0 | - |
| 2 | $t_0$ migration lag time of low molecular substances, h | $\ln t_0 = \dfrac{A}{T+C} + B$ | 12023 | -9.310 | 650 | 0.9999 |
| 3 | $\Delta\lambda_1$ micro-gasification internal influence constant, % | $\Delta\lambda_1 = \Delta\lambda_{0\oplus} - \Delta\lambda_{0\ominus}$ | -817.4 | 0.7959 | 0 | 0.9999 |
| 4 | $\Delta\lambda_2$ micro-gasification interface influence constant, % | $\ln(\Delta\lambda_2) = \dfrac{A}{T+C} + B$ | -6004 | 12.12 | 0 | 1.0000 |
| 5 | $\Delta\lambda_3$ mechanical stress influence constant, % | $\ln(\Delta\lambda_3) = \dfrac{A}{T+C} + B$ | 0.0 | -3.9120 | 0 | - |
| 6 | $\lambda_{0\oplus}$ thermal conductivity in micro-gasification phase state, W/(m.K) | $\Delta\lambda_{0\oplus} = A(1 + e^{\frac{-B}{T+C}})$ | 2.180 | 954.0 | 95 | 1.0000 |
| 7 | $\lambda_{0\ominus}$ initial intrinsic thermal conductivity before aging, W/(m.K) | $\ln \lambda_{0\ominus} = \dfrac{A}{T+C} + B$ | 0.00 | 0.7793 | 0 | - |
| 8 | $\beta_1$ migration oscillation frequency coefficient, dimensionless | $\ln \beta_1 = \dfrac{A}{T+C} + B$ | -10231 | 12.84 | 350 | 1.0000 |
| 9 | $\beta_2$ volatilization | $\ln \beta_2 = \dfrac{A}{T+C} + B$ | -1056 | -0.3064 | -10 | 0.9999 |

| | | | | | | |
|---|---|---|---|---|---|---|
| | oscillation frequency coefficient, dimensionless | | | | | |
| 10 | $k_1$ migration rate constant, 1/h | $\ln k_1 = \dfrac{A}{T + C} + B$ | -118064 | 81.90 | 775 | 0.9999 |
| 11 | $k_2$ volatilization rate constant, 1/h | $\ln k_2 = \dfrac{A}{T + C} + B$ | -1313 | -2.214 | 30 | 1.0000 |
| 12 | $k_3$ relaxation rate constant, 1/h | $\ln k_3 = \dfrac{A}{T + C} + B$ | 0 | -13.82 | 0 | - |
| 13 | k chemical reaction rate constant, 1/h | $\ln k = \dfrac{A}{T + C} + B$ | -4603 | 14.95 | -270 | 1.0000 |
| 14 | $\theta_1$ migration oscillation frequency index, dimensionless | $\ln \theta_1 = \dfrac{A}{T + C} + B$ | -8409 | 14.39 | 90 | 1.0000 |
| 15 | $\theta_2$ volatilization oscillation frequency index, dimensionless | $\ln \theta_2 = \dfrac{A}{T + C} + B$ | -6226 | 10.8 | 95 | 1.0000 |

Table 11　Parameter values of the thermal conductivity of P40 in the equation (1.1)

| Serial number | P40, parameters in the micro-gasification expansion oscillation equation (1) | Parameter values of various temperatures | | | |
|---|---|---|---|---|---|
| | | 298°C | 272°C | 245°C | 218°C |
| 1 | $\lambda_\infty$ thermal conductivity after weathering for more than 100 years, W/(m.K) | 0.960 | 0.960 | 0.960 | 0.960 |
| 2 | $t_0$ migration lag time of low molecular substances, h | 1.55 | 2.00 | 2.69 | 3.73 |
| 3 | $\Delta\lambda_1$ micro-gasification internal influence constant, % | -0.740 | -0.677 | -0.615 | -0.555 |

| 4 | $\Delta\lambda_2$ micro-gasification interface influence constant, % | -1.750 | -1.230 | -0.830 | -0.530 |
|---|---|---|---|---|---|
| 5 | $\Delta\lambda_3$ mechanical stress influence constant, % | -0.032 | -0.032 | -0.032 | -0.032 |
| 6 | $\lambda_{0\oplus}$ thermal conductivity in micro-gasification phase state, W/(m.K) | 3.440 | 3.500 | 3.563 | 3.625 |
| 7 | $\lambda_{0\ominus}$ initial intrinsic thermal conductivity before aging, W/(m.K) | 4.18 | 4.18 | 4.18 | 4.18 |
| 8 | $\beta_1$ migration oscillation frequency coefficient, dimensionless | 3.890 | 3.110 | 2.390 | 1.770 |
| 9 | $\beta_2$ volatilization oscillation frequency coefficient, dimensionless | 0.570 | 0.390 | 0.257 | 0.160 |
| 10 | $k_1$ migration rate constant, 1/h | 5.50E-02 | 2.10E-02 | 7.00E-03 | 2.00E-03 |
| 11 | $k_2$ volatilization rate constant, 1/h | 6.20E-02 | 1.80E-02 | 4.10E-03 | 8.00E-04 |
| 12 | $k_3$ relaxation rate constant, 1/h | 1.00E-06 | 1.00E-06 | 1.00E-06 | 1.00E-06 |
| 13 | k chemical reaction rate constant, 1/h | 9.80E-02 | 2.60E-02 | 7.10E-03 | 1.10E-03 |
| 14 | $\theta_1$ migration oscillation frequency index, dimensionless | 1.17E+00 | 8.60E-01 | 5.75E-01 | 4.10E-01 |
| 15 | $\theta_2$ volatilization oscillation frequency index, dimensionless | 0.410 | 0.366 | 0.322 | 0.280 |

(Continue) Table 11    Parameter and constant values of the thermal conductivity of P40 in the equation (1.1)

| Serial number | P40, parameters in the micro-gasification expansion oscillation equation (1) | Constant values of various parameters | | | | |
|---|---|---|---|---|---|---|
| | | General expression formula | A | B | C | $R^2$ |

| | | | A | B | C | |
|---|---|---|---|---|---|---|
| 1 | $\lambda_\infty$ heat conductivity coefficient after weathering for more than 100 years, W/(m.K) | $\ln \lambda_\infty = \dfrac{A}{T+C} + B$ | 0.00 | -0.0408 | 0 | - |
| 2 | $t_0$ migration lag time of low molecular substances, h | $\ln t_0 = \dfrac{A}{T+C} + B$ | 3940 | -5.719 | 69 | 0.9999 |
| 3 | $\Delta\lambda_1$ micro-gasification internal influence constant, % | $\Delta\lambda_1 = \Delta\lambda_{0\oplus} - \Delta\lambda_{0\ominus}$ | -1007 | 1.460 | 0 | 0.9995 |
| 4 | $\Delta\lambda_2$ micro-gasification Interface influence constant, % | $\ln(\Delta\lambda_2) = \dfrac{A}{T+C} + B$ | -4181 | 7.880 | 0 | 1.0000 |
| 5 | $\Delta\lambda_3$ mechanical stress influence constant, % | $\ln(\Delta\lambda_3) = \dfrac{A}{T+C} + B$ | 0.0 | -3.4420 | 0 | - |
| 6 | $\lambda_{0\oplus}$ thermal conductivity in micro-gasification phase state, W/(m.K) | $\Delta\lambda_{0\oplus} = A(1 + e^{\frac{-B}{T+C}})$ | 4.180 | 972.0 | -10 | 1.0000 |
| 7 | $\lambda_{0\ominus}$ initial intrinsic thermal conductivity before aging, W/(m.K) | $\ln \lambda_{0\ominus} = \dfrac{A}{T+C} + B$ | 0.00 | 1.4303 | 0 | - |
| 8 | $\beta_1$ migration oscillation frequency coefficient, dimensionless | $\ln\beta_1 = \dfrac{A}{T+C} + B$ | -2457 | 5.902 | -30 | 0.9999 |
| 9 | $\beta_2$ volatilization oscillation frequency coefficient, dimensionless | $\ln\beta_2 = \dfrac{A}{T+C} + B$ | -4445 | 7.217 | 0 | 1.0000 |
| 10 | $k_1$ migration rate constant, 1/h | $\ln k_1 = \dfrac{A}{T+C} + B$ | -11612 | 17.44 | 0 | 1.0000 |
| 11 | $k_2$ volatilization rate constant, 1/h | $\ln k_2 = \dfrac{A}{T+C} + B$ | -15283 | 24.00 | 0 | 0.9999 |
| 12 | $k_3$ relaxation rate constant, 1/h | $\ln k_3 = \dfrac{A}{T+C} + B$ | 0 | -13.816 | 0 | - |

| 13 | k chemical reaction rate constant, 1/h | $\ln k = \dfrac{A}{T + C} + B$ | -5103 | 12.38 | -225 | 0.9985 |
|----|----|----|----|----|----|----|
| 14 | $\theta_1$ migration oscillation frequency index, dimensionless | $\ln \theta_1 = \dfrac{A}{T + C} + B$ | -23530 | 17.32 | 800 | 0.9982 |
| 15 | $\theta_2$ volatilization oscillation frequency index, dimensionless | $\ln \theta_2 = \dfrac{A}{T + C} + B$ | -1630 | 1.711 | 55 | 1.0000 |

1.7 Prediction of changes in the thermal conductivitys of P20 and P40

In the first embodiment, it is only necessary to substitute the three one-to-one corresponding constants "A, B and C" in Table 10 and Table 11 and any service temperature below 298°C back into the "general expression formula" in Table 10 and Table 11, that is, the equation (2.1) or its shifted variant form, so as to respectively replace and figure out new "Q" values of the one-to-one corresponding fifteen parameters, and then any service time and the new "Q" values of the corresponding fifteen parameters are substituted back into the equation (1.1), so as to evaluate the change trends of the thermal conductivitys of P20 and P40 at any temperature and any time in advance.

Table 12　Compression ratio 10-30%, predict the long-term aging trend of the thermal conductivity $\lambda_t$, W/(m.K) at 195°C  by using the equation (1.1)

| 195°C | Aging time t, h | | | | | | | | | | |
|----|----|----|----|----|----|----|----|----|----|----|----|
| | 0.0⊖ | 0.0⊕ | 1 | 2 | 3 | 4 | 5 | 7 | 10 | 13 | 18 |
| P20 | 2.13 | 2.24 | 2.18 | 2.08 | 1.91 | 1.75 | 1.76 | 2.16 | 2.30 | 1.86 | 1.54 |
| P40 | 4.18 | 4.41 | 4.37 | 4.30 | 4.18 | 3.94 | 3.80 | 4.08 | 4.44 | 4.51 | 4.31 |

(Continue 1) Table 12　Compression ratio 10-30%, predict the long-term aging trend of the thermal conductivity $\lambda_t$, W/(m.K) at 195°C  by using the equation (1.1)

| 195°C | Aging time t, h | | | | | | | | | | |
|----|----|----|----|----|----|----|----|----|----|----|----|
| | 25 | 35 | 49 | 67 | 93 | 128 | 177 | 245 | 338 | 467 | 646 |

| | | | | | | | | | | | |
|---|---|---|---|---|---|---|---|---|---|---|---|
| P20 | 1.98 | 1.93 | 1.45 | 2.14 | 1.60 | 2.29 | 2.31 | 1.93 | 1.79 | 2.03 | 2.03 |
| P40 | 3.95 | 3.61 | 3.46 | 3.63 | 4.02 | 4.29 | 4.12 | 3.60 | 3.34 | 3.66 | 3.85 |

(Continue 2)  Table 12 Compression ratio 10-30%, predict the long-term aging trend of the thermal conductivity $\lambda_t$, W/(m.K) at 195°C  by using the equation (1.1)

| 195°C | Aging time t, h | | | | | | | | | | |
|---|---|---|---|---|---|---|---|---|---|---|---|
| | 893 | 1234 | 1705 | 2356 | 3256 | 4500 | 6219 | 8595 | 11878 | 16450 | - |
| P20 | 1.81 | 1.53 | 1.69 | 1.24 | 1.10 | 1.14 | 0.84 | 0.72 | 0.64 | 0.60 | - |
| P40 | 3.39 | 3.13 | 3.22 | 2.87 | 2.59 | 2.30 | 1.94 | 1.61 | 1.32 | 1.12 | - |

Table 13   Compression ratio 10-30%, predict the long-term aging trend of the thermal conductivity $\lambda_t$, W/(m.K) at 160°C  by using the equation (1.1)

| 160°C | Aging time t, h | | | | | | | | | | |
|---|---|---|---|---|---|---|---|---|---|---|---|
| | 0.0⊖ | 0.0⊕ | 4 | 6 | 8 | 11 | 15 | 20 | 28 | 39 | 53 |
| P20 | 2.13 | 1.88 | 1.84 | 1.81 | 1.83 | 1.89 | 1.95 | 2.02 | 2.08 | 2.15 | 2.21 |
| P40 | 4.18 | 3.89 | 3.82 | 3.78 | 3.72 | 3.76 | 3.86 | 3.96 | 4.06 | 4.15 | 4.24 |

(Continue 1) Table 13   Compression ratio 10-30%, predict the long-term aging trend of the thermal conductivity $\lambda_t$, W/(m.K) at 160°C  by using the equation (1.1)

| 160°C | Aging time t, h | | | | | | | | | | |
|---|---|---|---|---|---|---|---|---|---|---|---|
| | 74 | 102 | 140 | 194 | 268 | 371 | 512 | 708 | 979 | 1353 | 1869 |
| P20 | 2.27 | 2.31 | 2.35 | 2.36 | 2.35 | 2.32 | 2.25 | 2.16 | 2.06 | 1.95 | 1.87 |
| P40 | 4.33 | 4.40 | 4.46 | 4.49 | 4.50 | 4.48 | 4.43 | 4.34 | 4.22 | 4.09 | 3.95 |

(Continue 2) Table 13   Compression ratio 10-30%, predict the long-term aging trend of the thermal conductivity $\lambda_t$, W/(m.K) at 160°C  by using the equation (1.1)

| 160°C | Aging time t, h |
|---|---|

| | 2584 | 3570 | 4934 | 6819 | 9424 | 13025 | 18000 | 24876 | 34378 | 47511 | - |
|---|---|---|---|---|---|---|---|---|---|---|---|
| P20 | 1.82 | 1.83 | 1.91 | 2.04 | 2.19 | 2.31 | 2.34 | 2.24 | 2.05 | 1.82 | - |
| P40 | 3.83 | 3.75 | 3.72 | 3.74 | 3.79 | 3.82 | 3.80 | 3.71 | 3.57 | 3.38 | - |

Table 14   Compression ratio 10-30%, predict the long-term aging trend of the thermal conductivity $\lambda_t$, W/(m.K) at 125°C  by using the equation (1.1)

| 125°C | Aging time t, year | | | | | | | | | | |
|---|---|---|---|---|---|---|---|---|---|---|---|
| | 0.0⊖ | 0.0⊕ | 0.007 | 0.009 | 0.012 | 0.017 | 0.024 | 0.033 | 0.046 | 0.063 | 0.087 |
| P20 | 1.87 | 1.87 | 1.87 | 1.87 | 1.87 | 1.87 | 1.87 | 1.87 | 1.88 | 1.88 | 1.88 |
| P40 | 3.80 | 3.80 | 3.80 | 3.80 | 3.81 | 3.81 | 3.81 | 3.82 | 3.83 | 3.84 | 3.85 |

(Continue 1) Table 14    Compression ratio 10-30%, predict the long-term aging trend of the thermal conductivity $\lambda_t$, W/(m.K) at 125°C  by using the equation (1.1)

| 125°C | Aging time t, year | | | | | | | | | | |
|---|---|---|---|---|---|---|---|---|---|---|---|
| | 0.12 | 0.17 | 0.23 | 0.32 | 0.44 | 0.61 | 0.84 | 1.2 | 1.6 | 2.2 | 3.1 |
| P20 | 1.88 | 1.88 | 1.89 | 1.89 | 1.89 | 1.90 | 1.90 | 1.90 | 1.91 | 1.91 | 1.92 |
| P40 | 3.87 | 3.88 | 3.90 | 3.92 | 3.94 | 3.96 | 3.99 | 4.02 | 4.05 | 4.07 | 4.10 |

(Continue 2) Table 14    Compression ratio 10-30%, predict the long-term aging trend of the thermal conductivity $\lambda_t$, W/(m.K) at 125°C  by using the equation (1.1)

| 125°C | Aging time t, year | | | | | | | | | | |
|---|---|---|---|---|---|---|---|---|---|---|---|
| | 4.2 | 5.8 | 8.1 | 11.2 | 15.4 | 21.3 | 29.4 | 40.7 | 56.2 | 77.6 | - |
| P20 | 1.92 | 1.93 | 1.94 | 1.94 | 1.95 | 1.96 | 1.97 | 1.98 | 1.99 | 2.01 | - |
| P40 | 4.13 | 4.15 | 4.16 | 4.17 | 4.16 | 4.15 | 4.13 | 4.10 | 4.08 | 4.09 | - |

Table 15  Compression ratio 10-30%, predict the long-term aging trend of the thermal conductivity $\lambda_t$, W/(m.K) at 95°C by using the equation (1.1)

| 95°C | Aging time t, year |
|------|--------------------|

| | 0.0⊖ | 0.0⊕ | 0.03 | 0.04 | 0.05 | 0.07 | 0.10 | 0.13 | 0.18 | 0.25 | 0.35 |
|------|------|------|------|------|------|------|------|------|------|------|------|
| P20 | 2.18 | 1.97 | 1.97 | 1.97 | 1.97 | 1.97 | 1.97 | 1.97 | 1.97 | 1.97 | 1.97 |
| P40 | 4.18 | 3.94 | 3.93 | 3.93 | 3.92 | 3.92 | 3.92 | 3.91 | 3.91 | 3.91 | 3.90 |

(Continue 1) Table 15  Compression ratio 10-30%, predict the long-term aging trend of the thermal conductivity $\lambda_t$, W/(m.K) at 95°C by using the equation (1.1)

| 95°C | Aging time t, year | | | | | | | | | | |
|------|------|------|------|-----|-----|-----|-----|-----|-----|-----|------|
| | 0.48 | 0.66 | 0.92 | 1.3 | 1.8 | 2.4 | 3.4 | 4.6 | 6.4 | 8.8 | 12.2 |
| P20 | 1.97 | 1.97 | 1.96 | 1.96 | 1.96 | 1.96 | 1.96 | 1.96 | 1.96 | 1.96 | 1.96 |
| P40 | 3.90 | 3.90 | 3.90 | 3.90 | 3.89 | 3.89 | 3.89 | 3.89 | 3.89 | 3.89 | 3.90 |

(Continue 2) Table 15  Compression ratio 10-30%, predict the long-term aging trend of the thermal conductivity $\lambda_t$, W/(m.K) at 95°C by using the equation (1.1)

| 95°C | Aging time t, year | | | | | | | | | | |
|------|------|------|------|------|------|------|-------|-------|-------|---|---|
| | 16.9 | 23.4 | 32.3 | 44.6 | 61.6 | 85.2 | 117.7 | 162.7 | 224.9 | - | - |
| P20 | 1.96 | 1.96 | 1.96 | 1.96 | 1.96 | 1.96 | 1.95 | 1.95 | 1.95 | - | - |
| P40 | 3.90 | 3.91 | 3.92 | 3.94 | 3.96 | 3.99 | 4.02 | 4.05 | 4.08 | - | - |

Table 16   Compression ratio 10-30%, predict the long-term aging trend of the thermal conductivity $\lambda_t$, W/(m.K) at 75°C by using the equation (1.1)

| 75°C | Aging time t, year | | | | | | | | | | |
|---|---|---|---|---|---|---|---|---|---|---|---|
| | 0.0⊖ | 0.0⊕ | 0.03 | 0.04 | 0.05 | 0.07 | 0.10 | 0.13 | 0.18 | 0.25 | 0.35 |
| P20 | 2.18 | 2.13 | 2.03 | 2.03 | 2.03 | 2.03 | 2.03 | 2.03 | 2.03 | 2.03 | 2.03 |
| P40 | 4.18 | 4.09 | 4.03 | 4.02 | 4.02 | 4.02 | 4.02 | 4.02 | 4.02 | 4.01 | 4.01 |

(Continue 1) Table 16   Compression ratio 10-30%, predict the long-term aging trend of the thermal conductivity $\lambda_t$, W/(m.K) at 75°C by using the equation (1.1)

| 75°C | Aging time t, year | | | | | | | | | | |
|---|---|---|---|---|---|---|---|---|---|---|---|
| | 0.48 | 0.66 | 0.92 | 1.3 | 1.8 | 2.4 | 3.4 | 4.6 | 6.4 | 8.8 | 12.2 |
| P20 | 2.03 | 2.03 | 2.03 | 2.03 | 2.03 | 2.03 | 2.03 | 2.03 | 2.03 | 2.03 | 2.03 |
| P40 | 4.01 | 4.01 | 4.01 | 4.01 | 4.00 | 4.00 | 4.00 | 4.00 | 4.00 | 4.00 | 4.00 |

(Continue 2) Table 16   Compression ratio 10-30%, predict the long-term aging trend of the thermal conductivity $\lambda_t$, W/(m.K) at 75°C by using the equation (1.1)

| 75°C | Aging time t, year | | | | | | | | | | |
|---|---|---|---|---|---|---|---|---|---|---|---|
| | 16.9 | 23.4 | 32.3 | 44.6 | 61.6 | 85.2 | 118 | 1623 | 225 | - | - |
| P20 | 2.03 | 2.03 | 2.02 | 2.02 | 2.02 | 2.02 | 2.02 | 2.02 | 2.02 | - | - |
| P40 | 4.00 | 3.99 | 3.99 | 3.99 | 4.00 | 4.00 | 4.00 | 4.01 | 4.01 | - | - |

Table 17   Compression ratio 10-30%, predict the long-term aging trend of the thermal conductivity $\lambda_t$, W/(m.K) at 50°C  by using the equation (1.1)

| 50°C | Aging time t, year | | | | | | | | | | |
|------|------|------|------|------|------|------|------|------|------|------|------|
|      | 0.0⊖ | 0.0⊕ | 0.04 | 0.05 | 0.08 | 0.10 | 0.14 | 0.20 | 0.27 | 0.38 | 0.52 |
| P20  | 2.18 | 2.21 | 2.07 | 2.07 | 2.07 | 2.07 | 2.07 | 2.07 | 2.07 | 2.07 | 2.07 |
| P40  | 4.18 | 4.25 | 4.10 | 4.10 | 4.10 | 4.10 | 4.10 | 4.10 | 4.09 | 4.09 | 4.09 |

(Continue 1) Table 17   Compression ratio 10-30%, predict the long-term aging trend of the thermal conductivity $\lambda_t$, W/(m.K) at 50°C  by using the equation (1.1)

| 50°C | Aging time t, year | | | | | | | | | | |
|------|------|------|------|------|------|------|------|------|------|------|------|
|      | 0.73 | 1.0 | 1.4 | 1.9 | 2.6 | 3.7 | 5.1 | 7.0 | 9.7 | 13.3 | 18.4 |
| P20  | 2.07 | 2.07 | 2.07 | 2.07 | 2.07 | 2.07 | 2.07 | 2.07 | 2.07 | 2.07 | 2.07 |
| P40  | 4.09 | 4.09 | 4.09 | 4.09 | 4.09 | 4.09 | 4.09 | 4.09 | 4.09 | 4.09 | 4.09 |

(Continue 2) Table 17   Compression ratio 10-30%, predict the long-term aging trend of the thermal conductivity $\lambda_t$, W/(m.K) at 50°C  by using the equation (1.1)

| 50°C | Aging time t, year | | | | | | | | | | |
|------|------|------|------|------|------|------|------|------|------|------|------|
|      | 25.5 | 35.2 | 48.7 | 67.2 | 92.9 | 128.4 | 177.5 | 245.3 | - | - | - |
| P20  | 2.07 | 2.07 | 2.07 | 2.07 | 2.07 | 2.07 | 2.07 | 2.07 | - | - | - |
| P40  | 4.09 | 4.09 | 4.09 | 4.09 | 4.09 | 4.09 | 4.09 | 4.09 | - | - | - |

Table 18　Compression ratio 10-30%, predict the long-term aging trend of the thermal conductivity $\lambda_t$, W/(m.K) at 37°C by using the equation (1.1)

| 37°C | Aging time t, year | | | | | | | | | | |
|------|------|------|------|------|------|------|------|------|------|------|------|
|      | 0.0⊖ | 0.0⊕ | 0.11 | 0.15 | 0.21 | 0.29 | 0.40 | 0.55 | 0.76 | 1.0 | 1.5 |
| P20 | 2.09 | 2.09 | 2.09 | 2.09 | 2.09 | 2.09 | 2.09 | 2.09 | 2.09 | 2.09 | 2.09 |
| P40 | 4.18 | 4.25 | 4.12 | 4.12 | 4.12 | 4.12 | 4.12 | 4.12 | 4.12 | 4.12 | 4.12 |

(Continue 1) Table 18　Compression ratio 10-30%, predict the long-term aging trend of the thermal conductivity $\lambda_t$, W/(m.K) at 37°C by using the equation (1.1)

| 37°C | Aging time t, year | | | | | | | | | | |
|------|------|------|------|------|------|------|------|------|------|------|------|
|      | 2.0 | 2.8 | 3.8 | 5.3 | 7.3 | 10.1 | 14.0 | 19.3 | 26.7 | 36.9 | 50.9 |
| P20 | 2.09 | 2.09 | 2.09 | 2.09 | 2.09 | 2.09 | 2.09 | 2.09 | 2.09 | 2.09 | 2.09 |
| P40 | 4.12 | 4.12 | 4.12 | 4.12 | 4.11 | 4.11 | 4.11 | 4.11 | 4.11 | 4.11 | 4.11 |

(Continue 2) Table 18　Compression ratio 10-30%, predict the long-term aging trend of the thermal conductivity $\lambda_t$, W/(m.K) at 37°C by using the equation (1.1)

| 37°C | Aging time t, year | | | | | | | | | | |
|------|------|------|------|------|------|------|------|------|------|------|------|
|      | 70.4 | 97.3 | 135 | 186 | 257 | - | - | - | - | - | - |
| P20 | 2.09 | 2.09 | 2.09 | 2.09 | 2.09 | - | - | - | - | - | - |
| P40 | 4.11 | 4.11 | 4.11 | 4.11 | 4.11 | - | - | - | - | - | - |

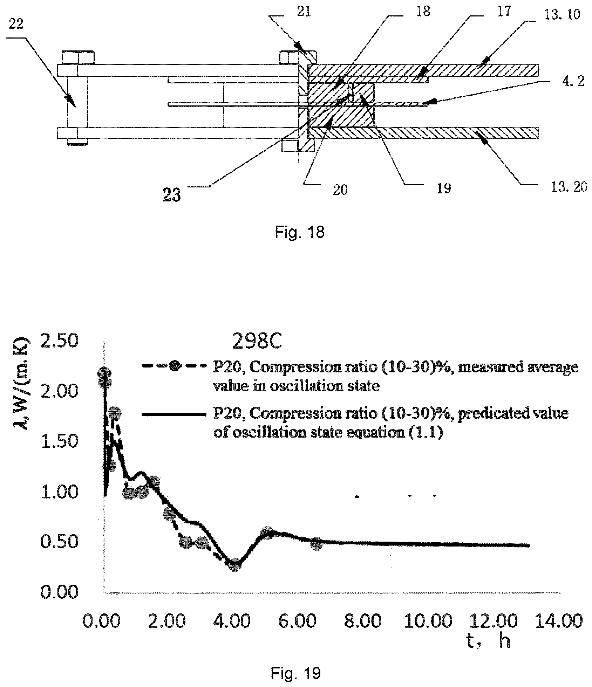

1) The predicted changes in the thermal conductivity with the aging time under three compression ratios and at the service temperatures of 298°C, 272°C, 245°C and 218°C are listed in Table 4 to Table 7; and plotting is performed by using the thermal conductivitys as vertical coordinates and the service times as abscissas, and trend curves corresponding to Table 4 to Table 7 are shown in Fig. 19 to Fig. 26.

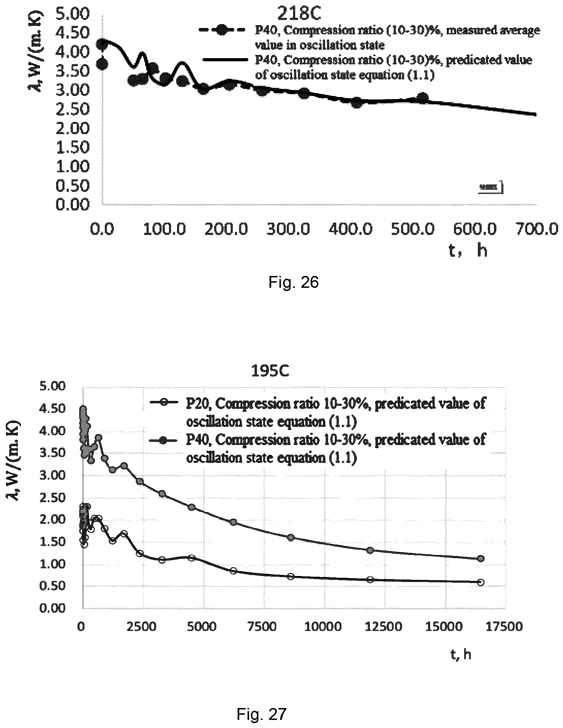

2) The predicted changes in the thermal conductivity with the aging time under three compression ratios and at the service temperatures of 195°C, 160°C, 125°C, 95°C, 75°C, 50°C and 37°C are shown in Table 12 to Table 18; and plotting is performed by using the thermal conductivitys as vertical coordinates and the service times as abscissas, and the trend curves corresponding to Table 12 to Table 18 are shown in Fig. 27 to Fig. 33.

As long as the constraint conditions under actual working conditions are consistent with the experimental conditions of simulated accelerated aging in a laboratory, and only the temperatures are different, then the equation (1) and the equation (2), when $R^2 \geq 0.999$, can be used for predicting the aging trend of the thermal conductivity under actual working conditions.

3) The variance of the deviation between the predicted result of the thermal conductivity and the measured value of the DRL thermal conductivity instrument is within ±1.96 times the standard deviation of the DRL thermal conductivity instrument itself.

It should be pointed out that, in the recognized framework standards of GB/T 20028, ASTM G 166, ASTM G 169, ISO 2578 and UL 746B for predicting the aging life of a material under a high-temperature accelerated aging condition, there is a limit on the prediction temperature width, that is, "the extended prediction temperature width is less than 0.8 times of the difference between the highest test temperature and the lowest test temperature".

This is because the Arrhenius' Equation (Arrhenius' Equation) adopted by these framework standards belongs to a single chemical component, single activation energy and a single crystal phase region; and during the prediction, it is necessary to limit the extended prediction temperature width, so as to expect that an experimental temperature region and a prediction temperature region belong to the same activation energy and the same crystal phase region as much as possible, so that the predicted result does not exceed an allowable error range.

However, the algorithm of the equation (1) in the present invention is a mathematical model that is established and experimented on the basis of the activation energy of multiple chemical reactions and the mechanism of cross-phase region, its linear correlation coefficient $R^2$ reaches the accuracy level of four "9", and when the prediction aging temperature width is extended, the range of 0.8 times of the experimental temperature width can be broken through, which is another ultimate important effect of the present invention.

## 2. Second embodiment: evaluation or prediction of the service life of compression set rate

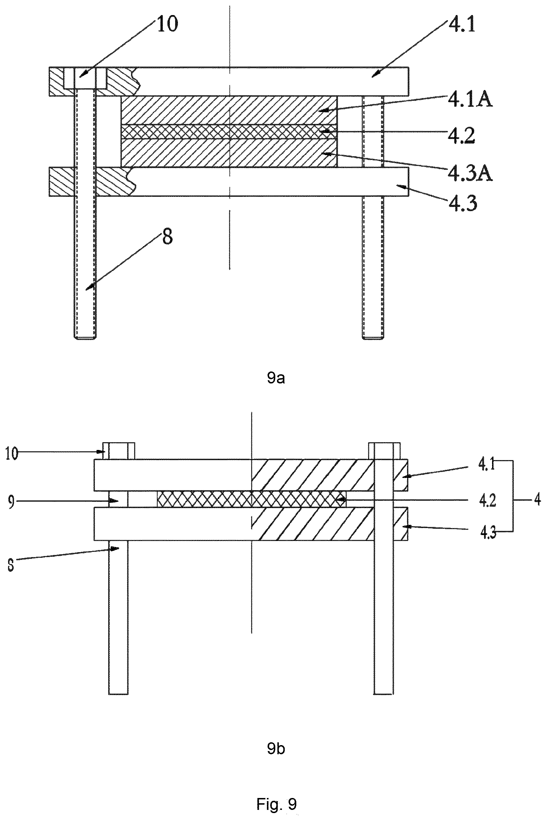

The second embodiment discloses another form of the test method and algorithm for the aging life of the new energy heat management composite, and the use thereof in the present invention. The long-term change trend of the compression set rate of an interface target specimen during long-term service under actual working conditions is evaluated or predicted by using a short-term accelerated aging test method, including: using fixture compression specimens with a compression ratio of 30%; selecting six constant temperatures for the group of fixture compression specimens within a temperature range of (85-245)°C, and making the group of fixture compression specimens respectively undergo three aging conditions of damp and hot, high and low temperature, and high and low temperature alternating circle in each specified constant temperature environment for a specified time or an accumulative number of cycles; using the fixture compression specimen shown in Fig. 9 to test the compression set rate of a target specimen 4.2 according to test procedures specified in GB/T 7759.1 or GB/T 7759.2 or ASTM D 395; using measured values of the compression set rate to fit corresponding fifteen parameters ($C_{A\infty}$, $C_{A0\ominus}$, $C_{A0\oplus}$, $\Delta C_{A1}$, $\Delta C_{A2}$, $\Delta C_{A3}$, $t_0$, $\beta_1$, $\beta_2$, $k_1$, $k_2$, $k_3$, $k$, $\theta_1$, $\theta_2$) in a micro-gasification expansion oscillation equation (1.2), and using each fitted parameter value to further fit three constants "A, B and C" contained in a dynamic correlation equation (2.2); substituting the three fitted constant values back into the dynamic correlation equation (2.2), so as to calculate new values of each parameter at the service temperatures of 75°C, 50°C and 37°C, respectively; and substituting the new values of this group of parameters back into the equation (1.2), so as to evaluate or predict the time-varying long-term change trend of the compression set rate of the target specimen after a specified service time or an accumulative number of cycles under the conditions of damp and hot, high and low temperature, and high and low temperature alternating circle at 75°C, 50°C and 37°C. The details of the implementation steps will be further disclosed in the following five chapters 2.1 to 2.5-

### 2.1 Preparation of the fixture compression specimen

As shown in Fig. 9, the preparation of the fixture compression specimen for the compression set includes: injecting a uniformly mixed toothpaste-like target specimen 4.2 into a space between an aluminum alloy upper butt joint bonding plate 4.1A and a lower butt joint bonding plate 4.3A, placing the plates on a supporting mold in advance, so that the upper butt joint bonding plate 4.1A, the target specimen 4.2 and the lower butt joint bonding plate 4.3A solidify into a parallel and coaxial "sandwich biscuit" overall structure, and then clamping the upper butt joint bonding plate 4.1A and the lower butt joint bonding plate 4.3A by using a metal screw rod 8, an upper rigid plate 4.1 and a lower rigid plate 4.3, so that the thickness of the target specimen 4.2 is adjusted to 70% of the initial thickness, that is, the compression ratio is 30%.

The target specimen 4.2 is a two-component organic silicone heat conducting adhesive product with a nominal thermal conductivity of 2 W/(m.K) and an initial casting thickness of 6.4mm, and the target specimen is referred to as S20 for short.

### 2.2 Three aging conditions

In the second embodiment, the three aging conditions include: damp and hot, high and low temperature impact,

and high and low temperature alternating cycle. Since there are more details involved, another three sections are used for further disclosure:

2.2.1 Damp and hot conditions

Four large groups of fixture compression specimens are used, each large group is divided into seven small groups, and parallel sub-specimens in each small group are executed according to corresponding test technical standards for physical, chemical and electrical properties (there are three parallel sub-specimens in the second embodiment); each large group is respectively placed in four standard aging boxes conforming to the constant temperatures specified in Table 19 in advance, and large temperature group and time small group marks and operation circulation records are made for the fixture compression specimens in advance; when the fixture compression specimens in each small group reach the constant times specified in Table 19, the fixture compression specimens in this small group are taken out from the large group of high temperature aging boxes; and the fixture compression specimens are placed at room temperature for (16-96)h, wherein the fixture compression specimens are placed under standard inspection conditions for not less than 30min, and physical, chemical and electrical property indicators of the target specimen 4.2 after aging are tested according to the test procedures specified in the corresponding technical standards for the physical, chemical and electrical properties (in the second embodiment, the compression set rate is test in accordance with the test procedure specified in GB/T 7759.1 or GB/T 7759.2 or ASTM D 395).
In the aging boxes of 195°C and 150°C, a dry hot air atmosphere is used, and the theoretical relative humidity is ≤ 15% and ≤ 30% respectively;
In the aging box of 97°C , the relative humidity refers to the gas-liquid equilibrium relative humidity of any saturated salt solution or glycerin aqueous solution in Table 20, and a saturated aqueous solution of potassium sulfate that meets the specifications of the GB/T 16496 standard, or a saturated aqueous solution of potassium chloride that meets the specifications of the GB/T 7118 standard, or an aqueous solution of glycerin (15±5)% that meets the specifications of the GB/T 13206 standard is injected into a white enamel basin with a length ≥ 420mm, a width ≥ 320mm and a depth ≥ 35mm; the enamel basin is placed on a bottom layer of an inner cavity of the aging box, during a constant temperature process, water needs to be dripped in time, so as to ensure that there is always water and undissolved solid substance in the enamel basin, or to ensure that the liquid level of the glycerin aqueous solution is between the highest and lowest liquid levels specified in advance; within a temperature range of 96-98°C, and under a closed condition in which an aging box door and a fresh air ventilation system are closed, the relative humidity controlled by this embodiment can be accurate to the (Rh±1)% level in Table 20; and according to the sampling time arrangement in Table 19, the interference on the humidity within a short time of opening and closing the box door every time, in several seconds, is negligible; and
in the aging box of 85°C, the relative humidity is realized by the automatic control of moisture evaporation by means of a built-in sensor system of the aging box.

Table 19 Damp and hot-arrangement on high temperature and time

| Aging condition | | Aging process, continuous accumulative constant temperature time t, h | | | | | | |
|---|---|---|---|---|---|---|---|---|
| Temperature ±1°C | Humidity ±3% | 0 | 1 | 2 | 3 | 4 | 5 | 6 |
| 195 | ≤15 | 5min | 12.0 | 24.0 | 48.0 | 72.0 | 120 | 192 |
| 150 | ≤30 | 8min | 24.0 | 48.0 | 72.0 | 120 | 192 | 312 |
| 97 | 97 | 13min | 48.0 | 72.0 | 120 | 192 | 312 | 504 |
| 85 | 85 | 13min | 48.0 | 72.0 | 120 | 192 | 312 | 504 |

Table 20    Damp and hot——control of relative humidity（Ph±1）%

| Temperature, ℃ | 5 | 10 | 15 | 20 | 25 | 30 | 35 | 40 | 45 | 50 | 55 |
|---|---|---|---|---|---|---|---|---|---|---|---|
| Potassium chloride | 87.7 | 86.8 | 85.9 | 85.1 | 84.3 | 83.6 | 82.9 | 82.3 | 81.7 | 81.2 | 80.7 |
| Potassium sulfate | 98.5 | 98.2 | 97.9 | 97.6 | 97.3 | 97.0 | 96.7 | 96.4 | 96.1 | 95.8 | 95.5 |
| Glycerin (15±5)% | 97.0 | 97.0 | 97.1 | 97.1 | 97.1 | 97.1 | 97.2 | 97.2 | 97.2 | 97.2 | 97.3 |

(Continue) Table 20    Damp and hot——control of relative humidity（Rh±1）%

| Temperature,℃ | 55 | 60 | 65 | 70 | 75 | 80 | 85 | 90 | 95 | 97 |
|---|---|---|---|---|---|---|---|---|---|---|
| Potassium chloride | 80.7 | 80.2 | 79.8 | 79.5 | 79.2 | 78.9 | 78.7 | 78.5 | 78.4 | 78.3 |
| Potassium sulfate | 95.5 | 95.2 | 94.9 | 94.6 | 94.3 | 94.1 | 93.8 | 93.5 | 93.2 | 93.1 |
| Glycerin (15±5)% | 97.3 | 97.3 | 97.3 | 97.4 | 97.4 | 97.4 | 97.4 | 97.5 | 97.5 | 97.5 |

Table 21 High and low temperature impact-high temperature time arrangement

| Aging condition of a large group of specimens | | Accumulative constant temperature time of a small group of specimens t, h | | | | | | |
|---|---|---|---|---|---|---|---|---|
| Temperature ±1°C | Humidity ±2% | 0 | 1 | 2 | 3 | 4 | 5 | 6 |
| 245 | ≤10 | 2.0 | 9.0 | 16.0 | 27.0 | 43.0 | 69.0 | 112 |
| 195 | ≤15 | 4.0 | 12.0 | 24.0 | 48.0 | 72.0 | 120 | 192 |
| 150 | ≤30 | 6.0 | 24.0 | 48.0 | 72.0 | 120 | 192 | 312 |
| 97 | 97or Table 20 | 10.0 | 48.0 | 72.0 | 120 | 192 | 312 | 504 |

Table 22 High and low temperature impact-low temperature time arrangement

| Aging condition of the large group of specimens | | Accumulative constant temperature time of the small of specimens t, h | | | | | | |
|---|---|---|---|---|---|---|---|---|
| Historical temperature | Temperature ±1°C | 0 | 1 | 2 | 3 | 4 | 5 | 6 |
| From 245°C | -40 | 2.0 | 9.0 | 16.0 | 27.0 | 43.0 | 69.0 | 112 |
| From 195°C | -40 | 4.0 | 12.0 | 24.0 | 48.0 | 72.0 | 120 | 192 |
| From 150°C | -40 | 6.0 | 24.0 | 48.0 | 72.0 | 120 | 192 | 312 |
| From 97°C | -40 | 10.0 | 48.0 | 72.0 | 120 | 192 | 312 | 504 |

Table 23 High and low temperature alternating cycle-arrangement on the number of cycles for high-temperature constant temperature of 1h

| Aging condition of the large group of specimens | | Accumulative number of alternations of an intermediate group of specimens ts, times | | | | | | |
|---|---|---|---|---|---|---|---|---|
| Temperature ±1°C | Humidity ±2% | 0 | 1 | 2 | 3 | 4 | 5 | 6 |
| 218 | ≤10 | 2 | 9 | 16 | 27 | 43 | 69 | 112 |
| 195 | ≤15 | 4 | 12 | 24 | 48 | 72 | 120 | 192 |
| 150 | ≤30 | 6 | 24 | 48 | 72 | 120 | 192 | 312 |
| 97 | 97 | 10 | 48 | 72 | 120 | 192 | 312 | 504 |

Table 24 High and low temperature alternating cycle-arrangement on the number of cycles for low-temperature constant temperature of 1h

| Aging condition of the large group of specimens | | Accumulative number of alternations of the intermediate group of specimens ts, times | | | | | | |
|---|---|---|---|---|---|---|---|---|
| Historical Temperature | Temperature ±1°C | 0 | 1 | 2 | 3 | 4 | 5 | 6 |
| From 218°C | -40 | 2 | 9 | 16 | 27 | 43 | 69 | 112 |
| From 195°C | -40 | 4 | 12 | 24 | 48 | 72 | 120 | 192 |
| From 150°C | -40 | 6 | 24 | 48 | 72 | 120 | 192 | 312 |
| From 97°C | -40 | 10 | 48 | 72 | 120 | 192 | 312 | 504 |

2.2.2 High and low temperature impact conditions

Four large groups of fixture compression specimens are used, each large group is divided into at least seven small groups, and parallel sub-specimens in each small group are executed according to corresponding test technical standards for the physical, chemical and electrical properties (there are three parallel sub-specimens in the second embodiment); each large group is respectively placed in four standard aging boxes conforming to the constant temperatures specified in Table 21 in advance, large temperature group and time small group marks and operation circulation records are made for the fixture compression specimens in advance; when the fixture compression specimens in each small group reach the constant times specified in Table 21, the fixture compression specimens in this small group are taken out from the large group of high temperature aging boxes, and the fixture compression specimens in this small group are placed in a freezer with a constant temperature specified according to Table 22 in advance within 10s; when the fixture compression specimens in each small group reach the constant times specified in Table 22, the fixture compression specimens in this small group are taken out from the low-temperature freezer; the fixture compression specimens are placed at room temperature for (16-96)h, wherein the fixture compression specimens are placed under standard inspection conditions for not less than 30min, and physical, chemical and electrical property indicators of the target specimen 4.2 after aging are tested according to the test procedures specified in the corresponding technical standards for the physical, chemical and electrical properties (in the second embodiment, the compression set rate is test in accordance with the test procedures specified in GB/T 7759.1 or GB/T 7759.2 or ASTM D 395).

When the equation (1.2) and the equation (2.2) are applied to the evaluation or prediction, the low-temperature constant temperature times in Table 22 are not input, and only the high-temperature constant temperature times in Table 21 are input.

2.2.3 High and low temperature alternating cycle conditions

Four large groups of fixture compression specimens are used, each large group is divided into at least seven small groups, and parallel sub-specimens in each small group are executed according to corresponding test technical standards for the physical, chemical and electrical properties (there are three parallel sub-specimens in the second embodiment); each large group is respectively placed in four standard aging boxes conforming to the constant temperatures specified in Table 23 in advance, large temperature group and time small group marks and operation circulation records are made for the fixture compression specimens in advance; and in accordance with a high and low temperature alternating cycle operation:

1) respectively four large groups of fixture compression specimens in the four standard aging boxes, respectively heating up to the temperatures specified in Table 23, when the constant temperature time reaches 1h, turning off the power supply of heating systems of the aging boxes, turning on fresh air ventilation systems of the aging boxes, controlling an air exchange rate to cool down to a temperature between 50°C and room temperature at a speed of (5-10)°C/min, and transferring all the fixture compression specimens in the four standard aging boxes into the freezer with a starting temperature between 0°C and room temperature;

2) next, continuing to cool down to (-40±1)°C at a speed of (5-10)°C/min, when the constant temperature time reaches 1h, turning off the power supply of a refrigeration system of the freezer, opening a freezer cover, changing the freezer cover into a sieve freezer cover with an aperture within the range of (60-80) meshes, controlling to heat up to a temperature between 0°C and room temperature at a speed of (5-10)°C/min, and respectively transferring all the fixture compression specimens in the four standard aging boxes with a starting temperature between room temperature and 50°C;

3) next, continuing to heat up to the temperatures specified in Table 23 at a speed of (5-10)°C/min, when the constant temperature time reaches 1h, turning off the power supply of the heating systems of the aging boxes, turning on the fresh air ventilation systems of the aging boxes, controlling the air exchange rate to cool down to a temperature between 50°C and room temperature at a speed of (5-10)°C/min, and transferring all the fixture compression specimens in the four standard aging boxes into the freezer with a starting temperature between 0°C and room temperature;

4) repeating the steps 2) and 3) back and forth; and

5) after the accumulative numbers of alternating cycles of constant temperature of the fixture compression specimens in the time group reach the numbers specified in Table 23 and Table 24 respectively, taking out the fixture compression specimens, and placing the same at room temperature for (16-96)h, wherein the fixture compression specimens are placed under standard inspection conditions for not less than 30min, and testing physical, chemical and electrical property indicators of the target specimen 4.2 after aging according to the test procedures specified in the corresponding technical standards for the physical, chemical and electrical properties (in the second embodiment, the compression set rate is test in accordance with the test procedures specified in GB/T 7759.1 or GB/T 7759.2 or ASTM D 395).

When the equation (1.2) and the equation (2.2) are applied to the evaluation or prediction, the low temperature times in Table 24 are not input, and only the high-temperature accumulative constant temperature times in Table 23 are input.

2.3 Test results

2.3.1 Damp and hot aging results

In the four temperature environments of 195°C, 150°C, 97°C and 85°C, the compression set rates after damp and hot aging are listed in Table 25-3 to Table 25-6, respectively.

2.3.2 Aging results of high and low temperature impact

In the four temperature environments of 245 °C, 195 °C, 150 °C and 97°C, the compression set rates after high and low temperature impact aging are shown in Table 25-1, Table 25-3 to Table 25-5, respectively.

2.3.3 Aging results of high and low temperature alternating cycle

In the four temperature environments of 218 °C, 195 °C, 150 °C and 97°C, the compression set rates after high and low temperature alternating cycle aging are shown in Table 25-2 to Table 25-5, respectively.

2.4 Establishment of a compression set rate equation (1.2)

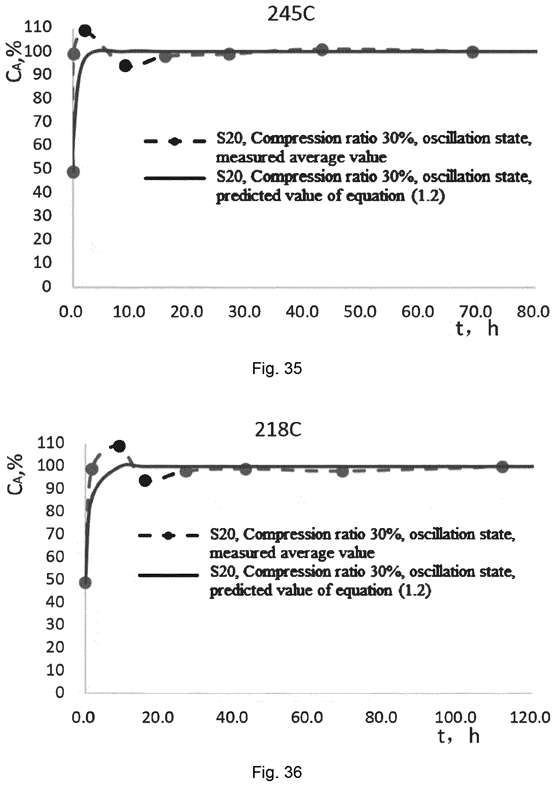

After undergoing the three aging conditions of damp and hot, high and low temperature impact, and high and low temperature alternating cycle in the six temperature environments of 245°C, 218°C, 195°C, 150°C, 97°C and 85°C, the aging trends of the compression set rate corresponding to Table 25-1 to Table 25-6 are shown in Fig. 35 to Fig. 40.

Table 25-1    245℃, Rh<10%, the aging trend of the compression set rate

| 245℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.08 | 2 | 9 | 16 | 27 | 43 |
| S20 | Damp and hot Measured | 3 | - | - | - | - | - | - | - | - |
| | High and low temperature impact Measured | 3 | - | - | - | 99 | 109 | 94 | 98 | 99 |
| | High and low temperature alternating cycle Measured | 3 | - | - | - | - | - | - | - | - |
| | Three aging conditions Measured average | 9 | - | - | - | 99 | 109 | 94 | 98 | 99 |
| | Three aging conditions Predicted according to the equation (1.2) | 9 | 49 | 63 | 97 | 100 | 100 | 100 | 100 | 100 |

(Continue) Table 25-1    245℃, Rh<10%, the aging trend of the compression set rate

| 245℃ Model number | Phase state, compression ratio | The number of sub-specimens | Aging time t, h | | | | | | | |
|---|---|---|---|---|---|---|---|---|---|---|
| | | | 69 | 112 | 192 | 312 | 505 | 817 | | 3400 |
| S20 | Damp and hot Measured | 3 | - | - | - | - | - | - | - | - |
| | High and low | 3 | 101 | 100 | - | - | - | - | - | - |

| | The number of sub-specimens | | | | | | | | |
|---|---|---|---|---|---|---|---|---|---|
| temperature impact Measured | | | | | | | | | |
| High and low temperature alternating cycle Measured | 3 | - | - | - | - | - | - | - | - |
| Three aging conditions Measured average | 9 | 101 | 100 | - | - | - | - | - | - |
| Three aging conditions Predicted according to the equation (1.2) | 9 | 100 | 100 | 100 | 100 | 100 | 100 | | 100 |

Table 25-2    218℃, Rh <13%, the aging trend of the compression set rate

| 218℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.08 | 2 | 9 | 16 | 27 | 43 |
| S20 | Damp and hot Measured | 3 | - | - | - | - | - | - | - | - |
| | High and low temperature impact Measured | 3 | - | - | - | - | - | - | - | - |
| S20 | High and low temperature alternating cycle Measured | 3 | - | - | - | 99 | 109 | 94 | 98 | 99 |

| | | | | | | | | | |
|---|---|---|---|---|---|---|---|---|---|
| Three aging conditions Measured average | 9 | - | - | - | 99 | 109 | 94 | 98 | 99 |
| Three aging conditions Predicted according to the equation (1.2) | 9 | 49 | 86 | 100 | 100 | 100 | 100 | 100 | 100 |

(Continue) Table 25-2    218℃, Rh <13%, the aging trend of the compression set rate

| 218℃ Model number | Phase state, compression ratio | The number of sub-specimens | Aging time t, h | | | | | | | |
|---|---|---|---|---|---|---|---|---|---|---|
| | | | 69 | 112 | 192 | 312 | 505 | 817 | | 3400 |
| S20 | Damp And hot Measured | 3 | - | - | - | - | - | - | - | - |
| | High and low temperature impact Measured | 3 | - | - | - | - | - | - | - | - |
| | High and low temperature alternating cycle Measured | 3 | 98 | 100 | - | - | - | - | - | - |
| | Three aging conditions Measured average | 9 | 98 | 100 | - | - | - | - | - | - |
| | Three aging conditions Predicted according to the equation (1.2) | 9 | 100 | 100 | 100 | 100 | 100 | 100 | | 100 |

Table 25-3    195℃, Rh <15%, the aging trend of the compression set rate, %

| 195℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.08 | 4 | 12 | 24 | 48 | 72 |
| S20 | Damp and hot Measured | 3 | - | - | 90 | 61 | 63 | 102 | 102 | 100 |
| | High and low temperature impact Measured | 3 | - | - | 92 | 57 | 102 | 100 | 105 | 103 |
| | High and low temperature alternating cycle Measured | 3 | - | - | 94 | 63 | 102 | 100 | 105 | 100 |
| | Three aging conditions Measured average | 9 | - | - | 92 | 60 | 89 | 101 | 104 | 101 |
| | Three aging conditions Predicted according to the equation (1.2) | 9 | 49 | 93 | 81 | 98 | 100 | 100 | 100 | 100 |

(Continue) Table 25-3  195℃, Rh <15%, the aging trend of compression set rate, %

| 195℃ Model number | Phase state, compression ratio | The number of sub-specimens | Aging time t, h | | | | | | | |
|---|---|---|---|---|---|---|---|---|---|---|
| | | | 120 | 192 | 312 | 505 | 817 | 1322 | | 3400 |
| S20 | Damp and hot Measured | 3 | 101 | 101 | - | - | - | - | - | - |
| | High and low temperature impact Measured | 3 | 101 | 102 | - | - | - | - | - | - |
| | High and low temperature alternating cycle | 3 | 101 | 100 | - | - | - | - | - | - |

| | Measured | | | | | | | | | |
|---|---|---|---|---|---|---|---|---|---|---|
| | Three aging conditions Measured average | 9 | 101 | 101 | - | - | - | - | - | - |
| | Three aging conditions Predicted according to the equation (1.2) | 9 | 100 | 100 | 100 | 100 | 100 | 100 | | 100 |

Table 25-4    150℃, Rh <30%, the aging trend of the compression set rate

| 150℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.13 | 6 | 24 | 48 | 72 | 120 |
| S20 | Damp and hot Measured | 3 | - | - | 77 | 44 | 92 | 90 | 86 | 90 |
| | High and low temperature impact Measured | 3 | - | - | - | 42 | 91 | 93 | 99 | 99 |
| | High and low temperature alternating cycle Measured | 3 | - | - | - | 42 | 91 | 93 | 99 | 99 |
| | Three aging conditions Measured average | 9 | - | - | 77 | 43 | 91 | 92 | 95 | 96 |
| | Three aging conditions Predicted according to the equation (1.2) | 9 | 49 | 42 | 63 | 88 | 97 | 99 | 100 | 100 |

(Continue) Table 25-4    150℃, Rh <30%, the aging trend of the compression set rate

| 150℃ Model number | Phase state, compression ratio | The number of sub-specimens | Aging time t, h | | | | | | | |
|---|---|---|---|---|---|---|---|---|---|---|
| | | | 192 | 312 | 504 | 810 | 1300 | 2100 | | 3400 |

| | Damp and hot Measured | 3 | 92 | 95 | 97 | - | - | - | - | - |
|---|---|---|---|---|---|---|---|---|---|---|
| | High and low temperature impact Measured | 3 | 100 | 101 | - | - | - | - | - | - |
| S20 | High and low temperature alternating cycle Measured | 3 | 99 | 100 | - | - | - | - | - | - |
| | Three aging conditions Measured average | 9 | 97 | 99 | 97 | - | - | - | - | - |
| | Three aging conditions Predicted according to the equation (1.2) | 9 | 100 | 100 | 100 | 100 | 100 | 100 | - | - |

Table 25-5    97℃, Rh 97%, the aging trend of the compression set rate

| 97℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.22 | 10 | 48 | 72 | 120 | 192 |
| S20 | Damp and hot Measured | 3 | - | - | 45 | - | 90 | 93 | 80 | 88 |
| | High and low temperature impact Measured | 3 | - | - | - | 52 | 84 | 93 | 90 | 97 |
| | High and low temperature alternating cycle Measured | 3 | - | - | - | 52 | 84 | 93 | 90 | 90 |

| | Three aging conditions Measured average | 9 | - | - | 45 | 52 | 86 | 93 | 87 | 92 |
|---|---|---|---|---|---|---|---|---|---|---|
| | Three aging conditions Predicted according to the equation (1.2) | 9 | 49 | 92 | 54 | 65 | 71 | 80 | 89 | 96 |

(Continue) Table 25-5  97℃, Rh 97%, the aging trend of the compression set rate

| 97℃ Model number | Phase state, compression ratio | The number of sub-specimens | Aging time t, h | | | | | | | |
|---|---|---|---|---|---|---|---|---|---|---|
| | | | 312 | 504 | 810 | 1300 | 2100 | 2500 | | 3400 |
| S20 | Damp and hot Measured | 3 | 92 | 96 | - | - | - | - | - | - |
| | High and low temperature impact Measured | 3 | 91 | 94 | 92 | 95 | 97 | - | - | - |
| | High and low temperature alternating cycle Measured | 3 | 92 | 95 | - | - | - | - | - | - |
| | Three aging conditions Measured average | 9 | 92 | 95 | 92 | 95 | 97 | - | - | - |
| | Three aging conditions Predicted according to the equation (1.2) | 9 | 99 | 100 | 100 | 100 | 100 | 100 | - | - |

Table 25-6  85℃, Rh 85%, the aging trend of the compression set rate

| 85℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.22 | 48 | 72 | 120 | 192 | 312 |
| S20 | Damp and hot Measured | 3 | - | - | 89 | 93 | 83 | 95 | 97 | 90 |
| | High and low temperature impact Measured | 3 | - | - | - | - | - | - | - | - |
| | High and low temperature alternating cycle Measured | 3 | - | - | - | - | - | - | - | - |
| | Three aging conditions Measured average | 9 | - | - | 80 | 84 | 75 | 86 | 87 | 90 |
| | Three aging conditions Predicted according to the equation (1.2) | 9 | 49 | 84 | 61 | 66 | 74 | 82 | 91 | 97 |

(Continue) Table 25-6　85℃, Rh 85%, the aging trend of the compression set rate

| 85℃ Model number | Phase state, compression ratio | The number of sub-specimens | Aging time t, h | | | | | | | |
|---|---|---|---|---|---|---|---|---|---|---|
| | | | 504 | 810 | 1300 | 2100 | 2500 | 3400 | | 4045 |
| S20 | Damp and hot Measured | 3 | 92 | 90 | 103 | 92 | - | - | - | - |
| | High and low temperature impact Measured | 3 | - | - | - | - | - | - | - | - |

| High and low temperature alternating cycle Measured | 3 | - | - | - | - | - | - | - | - |
|---|---|---|---|---|---|---|---|---|---|
| Three aging conditions Measured average | 9 | 92 | 90 | 103 | 92 | - | - | - | - |
| Three aging conditions Predicted according to the equation (1.2) | 9 | 99 | 100 | 100 | 100 | 100 | 100 | - | - |

### 2.4.1 T inspection

The T inspection indicates that the compression set rates under the three aging conditions of damp and hot, high and low temperature impact, and high and low temperature alternating cycle show similar aging change trends, and this indicates that the difference in the influence of the two factors, that is, high and low temperature impact, and high and low temperature alternating cycle, on the aging compression set rate of S20 is negligible. This is because the modulus of S20 is very small, and the stress impact generated by the temperature rise and fall speed of (5-10)°C/min is not sufficient to generate significant negative aging influence on S20; and the main determinant influencing the property aging is the accumulative time or the accumulative number of cycles and the mechanical compression ratio at high temperature.

Therefore, when processing the aging data of S20 that has been in service for a long time under actual working conditions, the three data specimen groups obtained under the three aging conditions of damp and hot, high and low temperature impact, and high and low temperature alternating cycle should be combined into a larger data specimen, and then data processing is carried out.

### 2.4.2 Parameter fitting in equation (1.2)

In another use of the calculation method of the micro-gasification expansion oscillation equation (1) of the present invention, a general symbol (P) of the physical, chemical and electrical properties in the equation (1) is replaced with a specific symbol ($C_A$) of the compression set rate, so as to convert the equation (1) into an equation (1.2):

$$C_{At} = C_{A\infty} + \left\{ C_{A0\ominus} + \left[ \Delta C_{A1} e^{-k_1 t} \times \mathrm{Sin}\beta_1 \left( \frac{t}{t_0} - 1 \right)^{\theta_1} \pi + \Delta C_{A2} e^{-k_2 t} \times \mathrm{Sin}\beta_2 \left( \frac{t}{t_0} - 1 \right)^{\theta_2} \pi + \Delta C_{A3} e^{-k_3 t} \right] - C_{A\infty} \right\} e^{-kt} \quad \ldots (1.2)$$

in the equation (1.2),

$C_A$-the compression set rate of the target specimen, and the measured values are listed in Table 25-1 to Table 25-6 for fitting and verification;
$C_{At}$-the compression set rate of the target specimen at any specified constant temperature for any service time, a predicted value;

$C_{Aa}$-the compression set rate after weathering for more than 100 years, determined by numerical simulation of a material formula, or a fitted value;

$C_{A0\ominus}$-initial compression set rate before aging, a measured value;

$C_{A0\oplus}$-the compression set rate during micro-gasification expansion; a fitted value;

Sin-micro-gasification oscillation trigonometric function;

$\Delta C_{A1}$-micro-gasification internal influence parameter, $\Delta C_{A1} = (C_{A0\oplus} - C_{A0\ominus})$;

$\Delta C_{A2}$-micro-gasification interface influence parameter, a fitted value;

$\Delta C_{A3}$-echanical stress influence parameter, a fitted value;

t-aging time or service time, determined by a specified time or an accumulative number of cycles;

$t_0$-migration lag time of low molecular substances, a fitted value;

$\beta_1$-migration oscillation frequency coefficient, a fitted value;

$\beta_2$-volatilization oscillation frequency coefficient, a fitted value;

$k_1$-migration rate parameter, a fitted value;

$k_2$-volatilization rate parameter, a fitted value;

$k_3$-relaxation rate parameter, a fitted value;

k-chemical reaction rate parameter, a fitted value;

$\theta_1$-migration oscillation frequency index, a fitted value; and

$\theta_2$-volatilization oscillation frequency index, a fitted value.

In the equation (1.2) in this embodiment, some of the fifteen parameters contained in the compression set rate are negligible, and thus are assigned as "0"; although all the parameters are difficult to be obtained directly through linear fitting, the average values of the measured values in Table 25-1 to Table 25-6 are taken as specimens, starting with the "0" assignment, the assignment is tentatively increased step by step with a step pitch as small as possible, the parameter is repeatedly and iteratively input into the equation (1.2) by using the parallax method, after each parameter is iterated for more than 50 times, the standard deviation of a difference value between a calculated value ($C_{At}$) and a measured value ($C_A$) converges to the minimum, and optimal values of the fifteen parameters "Q" of the thermal conductivity at each temperature are obtained, and the results of iterative optimization are listed in Table 26. Because of the mathematical frequency doubling effect, when there is more than one optimal value among the fitted values of the fifteen parameters, only the smaller group of fifteen "Q" values closest to "1 time" is selected as optimal parameters.

2.4.3 Constant fitting in equation (2.2)

In the second embodiment, for the fifteen parameters in the compression set rate equation (1.2) contained in Table 26, on mechanism, each parameter does not change with time, but only changes with temperature. Each parameter that changes with temperature further includes the constants expressed by the corresponding three symbols "A, B and C" in the equation (2), and during the fitting process, the constants, which are corresponding to the parameters in the compression set rate equation (1.2), in the equation (2) need to be replaced with corresponding symbols, so as to convert the equation (2) into an equation (2.2):

Table 26 Parameter values of the compression set rate of S20 in the equation (1.2)

| Serial | S20, parameters in the | Parameter values of various temperatures |
| --- | --- | --- |

| number | micro-gasification expansion oscillation equation (1) | 245°C | 218°C | 195°C | 150°C | 97°C | 85°C |
|---|---|---|---|---|---|---|---|
| 1 | $C_A\infty$ compression set rate after weathering for more than 100 years, % | 100 | 100 | 100 | 100 | 100 | 100 |
| 2 | $t_0$ migration lag time of low molecular substances, h | 4.00 | 4.90 | 5.90 | 8.90 | 17.0 | 20.0 |
| 3 | $\Delta C_{A1}$ micro-gasification internal influence constant, % | 46.0 | 45.0 | 44.2 | 42.4 | 40.0 | 39.4 |
| 4 | $\Delta C_{A2}$ micro-gasification interface influence constant, % | 0 | 0 | 0 | 0 | 0 | 0 |
| 5 | $\Delta C_{A3}$ mechanical stress influence constant, % | 20.0 | 20.0 | 20.0 | 20.0 | 20.0 | 20.0 |
| 6 | $C_{A0\oplus}$ compression set rate in micro-gasification phase state, % | 95.0 | 94.0 | 93.2 | 91.4 | 89.0 | 88.4 |
| 7 | $C_{A0\ominus}$ initial compression set rate before aging, % | 49.0 | 49.0 | 49.0 | 49.0 | 49.0 | 49.0 |
| 8 | $\beta_1$ migration oscillation frequency coefficient, dimensionless | 29.60 | 14.60 | 6.80 | 1.85 | 0.30 | 0.19 |
| 9 | $\beta_2$ volatilization oscillation frequency coefficient, dimensionless | 0 | 0 | 0 | 0 | 0 | 0 |
| 10 | $k_1$ migration rate constant, 1/h | 0.70 | 0.62 | 0.56 | 0.43 | 0.30 | 0.27 |
| 11 | $k_2$ volatilization rate constant, 1/h | 0 | 0 | 0 | 0 | 0 | 0 |
| 12 | $k_3$ relaxation rate constant, 1/h | 7.80 | 6.30 | 5.25 | 3.55 | 2.00 | 1.73 |
| 13 | k chemical reaction rate constant, 1/h | 1.300 | 0.600 | 0.260 | 0.060 | 0.0080 | 0.0055 |
| 14 | $\theta_1$ migration oscillation frequency index, dimensionless | 1.00 | 0.80 | 0.65 | 0.42 | 0.22 | 0.19 |

| 15 | $\theta_2$ volatilization oscillation frequency index, dimensionless | 1.0 | 1.0 | 1.0 | 1.0 | 1.0 | 1.0 |

(Continue) Table 26  Parameter and constant values of the compression set rate of S20 in the equation (1.2)

| Serial number | S20, parameters in the micro-gasification expansion oscillation equation (1) | Constant values of various parameters | | | | |
|---|---|---|---|---|---|---|
| | | General expression formula | A | B | C | $R^2$ |
| 1 | $C_{A\infty}$ compression set rate after weathering for more than 100 years, % | $\ln C_{A\infty} = \dfrac{A}{T + C} + B$ | 0.00 | 4.605 | 0 | - |
| 2 | $t_0$ migration lag time of low molecular substances, h | $\ln t_0 = \dfrac{A}{T + C} + B$ | 1867 | -2.215 | 0 | 0.9999 |
| 3 | $\Delta C_{A1}$ micro-gasification internal influence constant, % | $\Delta C_{A1} = \Delta C_{A0\oplus} - \Delta C_{A0\ominus}$ | -304.9 | 4.298 | 130 | 0.9999 |
| 4 | $\Delta C_{A2}$ micro-gasification interface influence constant, % | $\ln (\Delta C_A) = \dfrac{A}{T + C} + B$ | 0.00 | -13.82 | 0 | - |
| 5 | $\Delta C_{A3}$ mechanical stress influence constant, % | $\ln (\Delta C_{A3}) = \dfrac{A}{T + C} + B$ | 0.00 | 2.9957 | 0 | - |
| 6 | $C_{A0\oplus}$ compression set rate in micro-gasification phase state, % | $\Delta C_{A0\oplus} = A(1 + e^{\frac{-B}{T+C}})$ | 49.0 | 71.7 | 95 | 0.9999 |

| | | | A | B | C | |
|---|---|---|---|---|---|---|
| 7 | $C_{A0\ominus}$ initial compression set rate before aging, % | $\ln C_{A0\ominus} = \dfrac{A}{T+C} + B$ | 0.00 | 3.892 | 0 | - |
| 8 | $\beta_1$ migration oscillation frequency coefficient, dimensionless | $\ln\beta_1 = \dfrac{A}{T+C} + B$ | -30536 | 31.97 | 550 | 0.9997 |
| 9 | $\beta_2$ volatilization oscillation frequency coefficient, dimensionless | $\ln\beta_2 = \dfrac{A}{T+C} + B$ | 0.00 | -13.82 | 0 | - |
| 10 | $k_1$ migration rate constant, 1/h | $\ln k_1 = \dfrac{A}{T+C} + B$ | -1094 | 1.749 | 0 | 0.9999 |
| 11 | $k_2$ volatilization rate constant, 1/h | $\ln k_2 = \dfrac{A}{T+C} + B$ | 0.00 | -13.82 | 0 | - |
| 12 | $k_3$ relaxation rate constant, 1/h | $\ln k_3 = \dfrac{A}{T+C} + B$ | -1733 | 5.374 | 0 | 0.9994 |
| 13 | k chemical reaction rate constant, 1/h | $\ln k = \dfrac{A}{T+C} + B$ | -36566 | 33.11 | 595 | 0.9996 |
| 14 | $\theta_1$ migration oscillation frequency index, dimensionless | $\ln\theta_1 = \dfrac{A}{T+C} + B$ | -2535 | 4.376 | 60 | 0.9999 |
| 15 | $\theta_2$ volatilization oscillation frequency index, dimensionless | $\ln\theta_2 = \dfrac{A}{T+C} + B$ | 0.00 | 0.0 | 0 | - |

$$\ln Q = \frac{A}{T+C} + B \ldots\ldots\ldots\ldots\ldots\ldots\ldots\ldots\ldots\ldots\ldots\ldots\ldots\ldots\ldots\ldots\ldots\ldots\ldots\ldots\ldots\ldots\ldots\ldots\ldots\ldots \quad (2.2)$$

in the equation (2.2),

Q-corresponding to one of the fifteen parameters in the equation (1.2) at any temperature;

A-empirical constant of each parameter associated with the reaction activation energy and the diffusion activation energy of multiple components, a fitted value, K;

B-empirical constant of each parameter associated with the chemical reaction rate and the diffusion rate of multiple components, a fitted value, dimensionless;

C-conformal constant of Fourier series transformation of each parameter associated with the activation energy of multiple components, a fitted value, K;

T-absolute temperature, specified constant temperature +273.15, K;

"Q" in the equation (2.2) is replaced with the fifteen parameters in Table 26, plotting is performed by respectively using the logarithms of the fifteen parameters as vertical coordinates and using $1/(T+C)$ as abscissas, repeated iteration is performed by using a least square method electronic calculation program or a parallax method, and different "C" values are input, until $R^2$ automatically output by the system is $\geq 0.990$, it is considered that the line has been a straight line, and "A, B, C" and $R^2$ in one-to-one correspondence with the fifteen parameters are optimal values thereof, which are listed in Table 26, respectively.

2.5 Prediction of changes in the compression set rate of S20

As long as the constraint conditions under the actual working conditions are consistent with the accelerated aging test conditions, and only the temperatures are different, the equation (1.2) and the equation (2.2) are applied. When $R^2 \geq 0.999$, it is accurate to predict the aging trend of the compression set rate under actual working conditions.

In this embodiment, it is only necessary to substitute the one-to-one corresponding three constants "A, B and C" in Table 26 and any service temperature below 245°C back into the "general expression formula" in Table 26, that is, the equation (2.2) or its shifted variant form, so as to respectively figure out new "Q" values of the one-to-one corresponding fifteen parameters, and then any service time and the new "Q" values of the fifteen parameters are substituted back into the equation (1.2), so as to predict the long-term change trend of the compression set rate of S20 at any temperature and any time, when the compression ratio is 30%.

1) The equation (1.2) is used for predicting the time-varying changes of the compression set rate under the compression ratio of 30% and at the service temperatures of 245°C, 218°C, 195°C, 150°C, 97°C and 85°C, which are listed in Table 25-1 to Table 25-6; and plotting is performed by using the compression set rates as vertical coordinates and the service times as abscissas, and the trend curves corresponding to Table 25-1 to Table 25-6 are shown in Fig. 35 to Fig. 40.

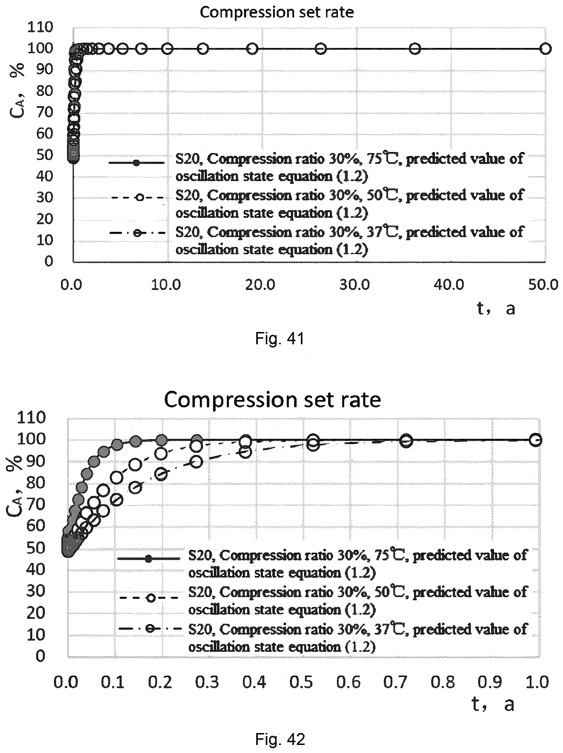

2) The equation (1.2) is used for predicting the change trends of the compression set rate with the service time under the compression ratio of 30% and at the service temperatures of 75°C, 50°C and 37°C, which are listed in Table 27; and plotting is performed by using the compression set rates as vertical coordinates and the service times as abscissas, and the trend curves corresponding to Table 27 are shown in Fig. 41 and Fig.42.

3) The standard deviation between the predicted result and the measured value of the compression set rate is within the range of $\pm 1.96$) Th sigma.

Table 27 When the compression ratio is 30%, the long-term change trend of the compression set rate $C_{At}$, % of S20 predicted by using the equation (1.2)

| S20 | Aging time t, year | | | | | | | | | | |
|---|---|---|---|---|---|---|---|---|---|---|---|
| | 0.0⊖ | 0.0⊕ | 0.011 | 0.015 | 0.02 | 0.03 | 0.04 | 0.05 | 0.07 | 0.10 | 0.14 |
| 75°C | 49 | 90.7 | 63 | 67 | 73 | 78 | 84 | 90 | 95 | 98 | 99 |
| 50°C | 49 | 90.3 | 54 | 56 | 59 | 62 | 66 | 71 | 77 | 83 | 89 |
| 37°C | 49 | 90.1 | 52 | 53 | 55 | 57 | 60 | 63 | 67 | 72 | 78 |

(Continue) Table 27 When the compression ratio is 30%, the long-term change trend of the compression set rate $C_{At}$, % of S20 predicted by using the equation (1.2)

| S20 | Aging time t, year | | | | | | | | | | |
|---|---|---|---|---|---|---|---|---|---|---|---|
| | 0.20 | 0.30 | 0.40 | 0.50 | 0.70 | 1.0 | 1.4 | 1.9 | 2.6 | 4.0 | 5.0 |
| 75°C | 100 | 100 | 100 | 100 | 100 | 100 | 100 | 100 | 100 | 100 | 100 |
| 50°C | 94 | 97 | 99 | 100 | 100 | 100 | 100 | 100 | 100 | 100 | 100 |
| 37°C | 84 | 90 | 95 | 98 | 99 | 100 | 100 | 100 | 100 | 100 | 100 |

3. Third embodiment: evaluation or prediction of the service life of hardness

The third embodiment discloses another form of the test method and algorithm for the aging life of the new energy heat management composite, and the use thereof in the present invention. The long-term change trend of the hardness of an interface target specimen during long-term service under actual working conditions is evaluated or predicted by using a short-term accelerated aging test method, including: using fixture compression specimens with a compression ratio of 30%; selecting six constant temperatures for the group of fixture compression specimens within a temperature range of (85-245)°C, and making the group of fixture compression specimens respectively undergo three aging conditions of damp and hot, high and low temperature, and high and low temperature alternating circle in each specified constant temperature environment for a specified time or an accumulative number of cycles; using the fixture compression specimen shown in Fig. 9 to test the hardness of a target specimen 4.2 according to test procedures specified in GB/T 2411 or GB/T 6031 or ASTM D 2240; using measured values of the hardness to fit corresponding fifteen parameters ($H_\infty$, $H_{0\ominus}$, $H_{0\oplus}$, $\Delta H_1$, $\Delta H_2$, $\Delta H_3$, $t_0$, $\beta_1$, $\beta_2$, $k_1$, $k_2$, $k_3$, $k$, $\theta_1$, $\theta_2$) in a micro-gasification expansion oscillation equation (1.3), and using each fitted parameter value to further fit three constants "A, B and C" contained in a dynamic correlation equation (2.3); substituting the three fitted constant values back into the dynamic correlation equation (2.3), so as to calculate new values of each parameter at the service temperatures of 75°C, 50°C and 37°C, respectively; and substituting the new values of this group of parameters back into the equation (1.3), so as to evaluate or predict the time-varying long-term change trend of the hardness of the target specimen after a specified service time or an accumulative number of cycles under the conditions of damp and hot, high and low temperature, and high and low temperature alternating circle at 75°C, 50°C and 37°C. The details of the implementation steps will be further disclosed in the following five chapters 3.1 to 3.50-

3.1 Preparation of the fixture compression specimen

As shown in Fig. 9, the preparation of the fixture compression specimen for the hardness includes: injecting a uniformly mixed toothpaste-like target specimen 4.2 into a space between an aluminum alloy upper butt joint bonding plate 4.1A and a lower butt joint bonding plate 4.3A, placing the plates on a supporting mold in advance, so that the upper butt joint bonding plate 4.1A, the target specimen 4.2 and the lower butt joint bonding plate 4.3A solidify into a parallel and coaxial "sandwich biscuit" overall structure, and then clamping the upper butt joint bonding plate 4.1A and the lower butt joint bonding plate 4.3A by using a metal screw rod 8, an upper rigid plate 4.1 and a lower rigid plate 4.3, so that the thickness of the target specimen 4.2 is adjusted to 70% of the initial thickness, that is, the compression ratio is 30%.

After the aging process is completed, a bonding interface between the target specimen 4.2 and the upper butt joint bonding plate 4.1A is smoothly cut and peeled by using a sharp thin blade, and then a hardness test procedure is carried out; or, the target specimen 4.2 and the upper butt joint bonding plate 4.1A are not cut or peeled, only the probe of a hardmeter is in contact with the edge of the target specimen 4.2 between the upper butt joint bonding plate 4.1A and the lower butt joint bonding plate 4.3A to test the hardness, and the measured results of the two test methods are equivalent.

The target specimen 4.2 is a two-component organic silicone heat conducting adhesive product, with a nominal thermal conductivity of 2 W/(m.K) and an initial casting thickness of 12.7mm, and the target specimen is referred to as S20 for short.

3.2 Three aging conditions

In the third embodiment, the three aging conditions include: damp and hot, high and low temperature impact, and high and low temperature alternating cycle, which are completely the same as the three aging conditions disclosed in the second embodiment.

3.3 Test results

3.3.1 Damp and hot aging results

In the four temperature environments of 195°C, 150°C, 97°C and 85°C, the hardness after damp and hot is listed in Table 28-3 to Table 28-6, respectively.

3.3.2 Aging results of high and low temperature impact

In the four temperature environments of 245°C, 195°C, 150°C and 97°C, the hardness after high and low temperature impact is shown in Table 28-1, and Table 28-3 to Table 28-5, respectively.

3.3.3 Aging results of high and low temperature alternating cycle

In the four temperature environments of 218°C, 195°C, 150°C and 97°C, the hardness after high and low temperature alternating cycle is shown in Table 28-2 to Table 28-5, respectively.

Table 28-1    245℃, Rh<10%, hardness aging trend

| 245℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.08 | 2 | 9 | 16 | 27 | 43 |
| S20 | Damp and hot Measured | 5 | - | - | - | - | - | - | - | - |
| | High and low temperature impact Measured | 5 | - | - | - | 65 | 70 | 72 | 73 | 77 |
| | High and low temperature alternating cycle Measured | 5 | - | - | - | - | - | - | - | - |
| | Three aging conditions Measured average | 15 | - | - | - | 65 | 70 | 72 | 73 | 77 |
| | Three aging conditions Predicted according to the equation (1.3) | 15 | 64 | 59 | 71 | 66 | 73 | 76 | 77 | 78 |

(Continue) Table 28-1    245℃, Rh<10%, hardness aging trend

| 245℃ Model number | Phase state, compression ratio | The number of sub-specimens | Aging time t, h | | | | | | | |
|---|---|---|---|---|---|---|---|---|---|---|
| | | | 69 | 112 | 192 | 311 | 503 | 813 | | 3400 |
| S20 | Damp and hot Measured | 5 | - | - | - | - | - | - | | - |
| | High and low | 5 | 76 | 77 | - | - | - | - | | - |

| | The number of sub-specimens | 0.0⊖ | 0.0⊕ | 0.08 | 1.8 | 9 | 16 | 27 | 43 |
|---|---|---|---|---|---|---|---|---|---|
| temperature impact Measured | | | | | | | | | |
| High and low temperature alternating cycle Measured | 5 | - | - | - | - | - | - | - | - |
| Three aging conditions Measured average | 15 | 76 | 77 | - | - | - | - | - | - |
| Three aging conditions Predicted according to the equation (1.3) | 15 | 78 | 78 | 78 | 78 | 78 | 78 | 78 | 78 |

Table 28-2   218℃, Rh <13%, hardness aging trend

| 218℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.08 | 1.8 | 9 | 16 | 27 | 43 |
| S20 | Damp and hot Measured | 5 | - | - | - | - | - | - | - | - |
| | High and low temperature impact Measured | 5 | - | - | - | - | - | - | - | - |
| | High and low temperature alternating cycle Measured | 5 | - | - | - | 67 | 69 | 70 | 70 | 75 |

74

| | Three aging conditions Measured average | 15 | - | - | - | 67 | 69 | 70 | 70 | 75 |
|---|---|---|---|---|---|---|---|---|---|---|
| | Three aging conditions Predicted according to the equation (1.3) | 15 | 64 | 59 | 67 | 65 | 70 | 73 | 75 | 77 |

(Continue) Table 28-2  218℃, Rh <13%, hardness aging trend

| 218℃ Model number | Phase state, compression ratio | The number of sub-specimens | Aging time t, h | | | | | | |
|---|---|---|---|---|---|---|---|---|---|
| | | | 69 | 112 | 192 | 311 | 503 | 813 | 3400 |
| S20 | Damp and hot Measured | 5 | - | - | - | - | - | - | - |
| | High and low temperature impact Measured | 5 | - | - | - | - | - | - | - |
| | High and low temperature alternating cycle Measured | 5 | 77 | 79 | - | - | - | - | - |
| | Three aging conditions Measured average | 15 | 77 | 79 | - | - | - | - | - |
| | Three aging conditions Predicted according to the equation (1.3) | 15 | 77 | 77 | 77 | 77 | 77 | 77 | 77 |

Table 28-3   195℃, Rh <15%, hardness aging trend

| 195℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.08 | 4 | 12 | 24 | 48 | 72 |
| S20 | Damp and hot Measured | 5 | - | - | 66 | - | 67 | 71 | 75 | 78 |
| | High and low temperature impact Measured | 5 | - | - | - | 70 | 68 | 74 | 75 | 76 |
| | High and low temperature alternating cycle Measured | 5 | - | - | - | 65 | 66 | 65 | 66 | 77 |
| | Three aging conditions Measured average | 15 | - | - | 66 | 68 | 67 | 70 | 72 | 77 |
| | Three aging conditions Predicted according to the equation (1.3) | 15 | 64 | 59 | 69 | 66 | 69 | 72 | 75 | 76 |

(Continue) Table 28-3   195℃, Rh <15%, hardness aging trend

| 195℃ Model number | Phase state, compression ratio | The number of sub-specimens | Aging time t, h | | | | | | |
|---|---|---|---|---|---|---|---|---|---|
| | | | 120 | 192 | 312 | 505 | 817 | 1322 | 3400 |
| S20 | Damp and hot Measured | 5 | 82 | 81 | - | - | - | - | - |
| | High and low temperature impact Measured | 5 | 79 | 79 | - | - | - | - | - |

| High and low temperature alternating cycle Measured | 5 | 77 | 80 | - | - | - | - | | - |
| Three aging conditions Measured average | 15 | 79 | 80 | - | - | - | - | | - |
| Three aging conditions Predicted according to the equation (1.3) | 15 | 77 | 77 | 77 | 77 | 77 | 77 | | 77 |

Table 28-4    150℃, Rh <30%, hardness aging trend

| 150℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.1 | 6.0 | 24.0 | 48 | 72 | 120 |
| | Damp and hot Measured | 5 | - | - | 68 | | 65 | 70 | 71 | 72 |
| S20 | High and low temperature impact Measured | 5 | - | - | - | 65 | 66 | 68 | 71 | 71 |
| | High and low temperature alternating cycle Measured | 5 | - | - | - | 67 | 68 | 67 | 69 | 71 |
| | Three aging conditions Measured average | 15 | - | - | 68 | 66 | 66 | 68 | 70 | 71 |
| | Three aging | 15 | 64 | 60 | 74 | 65 | 68 | 71 | 72 | 74 |

| | conditions Predicted according to the equation (1.3) | | | | | | | | | | |
|---|---|---|---|---|---|---|---|---|---|---|---|

(Continue) Table 28-4   150℃, Rh <30%, hardness aging trend

| 150℃ Model number | Phase state, compression ratio | The number of sub-specimens | Aging time t, h | | | | | | | |
|---|---|---|---|---|---|---|---|---|---|---|
| | | | 192 | 312 | 504 | 810 | 1300 | 2100 | | 3400 |
| S20 | Damp and hot Measured | 5 | 73 | 74 | | - | - | - | - | - |
| | High and low temperature impact Measured | 5 | 73 | 73 | | - | - | - | - | - |
| | High and low temperature alternating cycle Measured | 5 | 72 | 74 | 74 | - | - | - | - | - |
| | Three aging conditions Measured average | 15 | 73 | 74 | 74 | - | - | - | - | - |
| | Three aging conditions Predicted according to the equation (1.3) | 15 | 75 | 75 | 75 | 75 | 75 | 75 | | 75 |

Table 28-5   97℃, Rh 97%, hardness aging trend

| 97℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.22 | 10 | 48 | 72 | 120 | 192 |
| S20 | Damp and hot Measured | 5 | - | - | 66 | | 68 | 67 | 67 | 69 |
| | High and low | 5 | - | - | - | 65 | 66 | 67 | 67 | 68 |

| Phase state, compression ratio | The number of sub-specimens | 312 | 504 | 810 | 1300 | 2100 | 2500 | | 3400 |
|---|---|---|---|---|---|---|---|---|---|
| temperature impact Measured | | | | | | | | | |
| High and low temperature alternating cycle Measured | 5 | - | - | - | 66 | 66 | 67 | 66 | 68 |
| Three aging conditions Measured average | 15 | - | - | 66 | 66 | 67 | 67 | 67 | 68 |
| Three aging conditions Predicted according to the equation (1.3) | 15 | 64 | 60 | 69 | 64 | 66 | 67 | 68 | 70 |

(Continue) Table 28-5　97℃, Rh 97%, hardness aging trend

| 97℃ Model number | Phase state, compression ratio | The number of sub-specimens | Aging time t, h | | | | | | | |
|---|---|---|---|---|---|---|---|---|---|---|
| | | | 312 | 504 | 810 | 1300 | 2100 | 2500 | | 3400 |
| S20 | Damp and hot Measured | 5 | 69 | 71 | - | - | - | - | - | - |
| | High and low temperature impact Measured | 5 | 70 | 71 | 72 | 73 | - | - | - | - |
| | High and low temperature alternating cycle Measured | 5 | 70 | 71 | - | - | - | - | - | - |
| | Three aging conditions Measured average | 15 | 70 | 71 | 72 | 73 | - | - | - | - |

| | Three aging conditions Predicted according to the equation (1.3) | 15 | 72 | 73 | 73 | 73 | 73 | 73 | | 73 |
|---|---|---|---|---|---|---|---|---|---|---|

Table 28-6 85℃, Rh 85%, hardness aging trend

| 85℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.22 | 10 | 48 | 72 | 120 | 192 |
| S20 | Damp and hot Measured | 5 | - | - | 69 | | 67 | 68 | 69 | 70 |
| | High and low temperature impact Measured | 5 | - | - | - | - | - | - | - | - |
| | High and low temperature alternating cycle Measured | 5 | - | - | - | - | - | - | - | - |
| | Three aging conditions Measured average | 15 | - | - | 69 | - | 67 | 68 | 69 | 70 |
| | Three aging conditions Predicted according to the equation (1.3) | 15 | 64 | 60 | 70 | 64 | 66 | 66 | 67 | 69 |

(Continue) Table 28-6 85℃, Rh 85%, hardness aging trend

| 85℃ Model number | Phase state, compression ratio | The number of sub-specimens | Aging time t, h | | | | | | | |
|---|---|---|---|---|---|---|---|---|---|---|
| | | | 312 | 504 | 810 | 1300 | 2100 | 2500 | | 4050 |

| | | | | | | | | | | | |
|---|---|---|---|---|---|---|---|---|---|---|---|
| S20 | Damp and hot Measured | 5 | 70 | 71 | 72 | 73 | - | - | - | - |
| | High and low temperature impact Measured | 5 | - | - | - | - | - | - | - | - |
| | High and low temperature alternating cycle Measured | 5 | - | - | - | - | - | - | - | - |
| | Three aging conditions Measured average | 15 | 70 | 71 | 72 | 73 | - | - | - | - |
| | Three aging conditions Predicted according to the equation (1.3) | 15 | 70 | 72 | 73 | 73 | 73 | 73 | | 73 |

3.4 Establishment of a hardness equation (1.3)

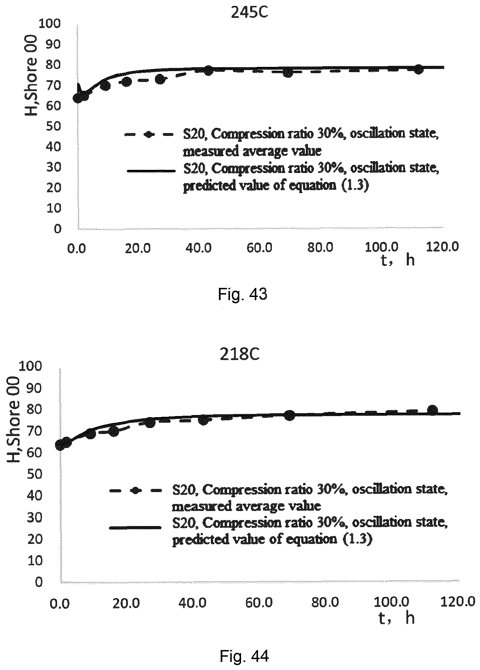

After undergoing the three aging conditions of damp and hot, high and low temperature impact, and high and low temperature alternating cycle in the six temperature environments of 245°C, 218°C, 195°C, 150°C, 97°C and 85°C, the hardness aging trends corresponding to Table 28-1 to Table 28-6 are shown in Fig. 43 to Fig. 48.

3.4.1 T inspection

The T inspection indicates that the hardness under the three aging conditions of damp and hot, high and low temperature impact, and high and low temperature alternating cycle shows similar aging change trends, and this indicates that the difference in the influence of the two factors, that is, high and low temperature impact, and high and low temperature alternating cycle, on the aging hardness of S20 is negligible. This is because the modulus of S20 is very small, and the stress impact generated by the temperature rise and fall speed of (5-10)°C/min is not sufficient to generate significant negative aging influence on S20; and the main determinant influencing the property aging is the accumulative time or the accumulative number of cycles at high temperature.

Therefore, when processing the aging data of S20 that has been in service for a long time under actual working conditions, the three data specimen groups obtained under the three aging conditions of damp and hot, high and low temperature impact, and high and low temperature alternating cycle should be combined into a larger data specimen, and then data processing is carried out.

3.4.2 Parameter fitting in equation (1.3)

In another use of the calculation method of the micro-gasification expansion oscillation equation (1) of the present invention, a general symbol (P) of the physical, chemical and electrical properties in the equation (1) is replaced with a specific hardness symbol (H), so as to convert the equation (1) into an equation (1.3):

$$H_t = H_\infty + \left\{ H_{0\ominus} + \left[ \Delta H_1 e^{-k_1 t} \times Sin\beta_1 \left(\frac{t}{t_0} - 1\right)^{\theta_1} \pi + \Delta H_2 e^{-k_2 t} \times Sin\beta_2 \left(\frac{t}{t_0} - 1\right)^{\theta_2} \pi + \right. \right.$$

$$\left. \left. \Delta H_3 e^{-k_3 t} \right] - H_\infty \right\} e^{-kt} \quad \ldots (1.3)$$

in the equation (1.3),

H-the hardness of the target specimen, and the measured values are listed in Table 28-1 to Table 28-6 for fitting and verification;

$H_t$-the hardness of the target specimen at any specified constant temperature for any service time, a predicted value;

$H_\infty$-the hardness after weathering for more than 100 years, determined by numerical simulation of a material formula, or a fitted value;

$H_{0\ominus}$-initial hardness before aging, a measured value;

$H_{0\oplus}$-the hardness during micro-gasification expansion; a fitted value;

Sin-micro-gasification oscillation trigonometric function;

$\Delta H_1$-micro-gasification internal influence parameter, $\Delta H_1 = (H_{0\oplus} - H_{0\ominus})$;

$\Delta H_2$-micro-gasification interface influence parameter, a fitted value;

$\Delta H_3$-mechanical stress influence parameter, a fitted value;

t-aging time or service time, determined by a specified time or an accumulative number of cycles;

$t_0$-migration lag time of low molecular substances, a fitted value;

$\beta_1$-migration oscillation frequency coefficient, a fitted value;

$\beta_2$-volatilization oscillation frequency coefficient, a fitted value;

$k_1$-migration rate parameter, a fitted value;

$k_2$-volatilization rate parameter, a fitted value;

$k_3$-relaxation rate parameter, a fitted value;

k-chemical reaction rate parameter, a fitted value;

$\theta_1$- migration oscillation frequency index, a fitted value; and

$\theta_2$- volatilization oscillation frequency index, a fitted value.

In the formula (1.3) in this embodiment, some of the fifteen parameters contained in the hardness are negligible, and thus are assigned as "0"; although all the parameters are difficult to be obtained directly through linear fitting, the average values of the measured values in Table 28-1 to Table 28-6 are taken as specimens, starting with the "0" assignment, the assignment is tentatively increased step by step with a step pitch as small as possible, the parameter is repeatedly and iteratively input into the equation (1.3) by using an electronic calculation program or a parallax method, after each parameter is iterated for more than 50 times, the standard deviation of a difference value between a calculated value ($H_t$) and a measured value (H) converges to the minimum, and optimal values of the fifteen parameters "Q" of the thermal conductivity at each temperature are obtained, and the results of iterative optimization are listed in Table 29.

Because of the mathematical frequency doubling effect, when there is more than one optimal value among the fitted values of the fifteen parameters, only the smaller group of fifteen "Q" values closest to "1 time" is selected as optimal parameters.

3.4.3 Constant fitting in equation (2.3)

In the third embodiment, for the fifteen parameters in the hardness equation (1.3) contained in Table 29, on mechanism, each parameter does not change with time, but only changes with temperature. Each parameter that changes with temperature further includes the constants expressed by the corresponding three symbols "A, B and C" in the equation (2), and during the fitting process, the constants, which are corresponding to the parameters in the hardness equation (1.3), in the equation (2) need to be replaced with corresponding symbols, so as to convert the equation (2) into an equation (2.3):

$$\ln Q = \frac{A}{T+C} + B \dots\dots\dots\dots\dots\dots\dots\dots\dots\dots\dots\dots\dots\dots (2.3)$$

in the equation (2.3),

Q-corresponding to one of the fifteen parameters in the equation (1.3) at any temperature;

A-empirical constant of each parameter associated with the reaction activation energy and the diffusion activation energy of multiple components, a fitted value, K;

B-empirical constant of each parameter associated with the chemical reaction rate and the diffusion rate of multiple components, a fitted value, dimensionless;

C-conformal constant of Fourier series transformation of each parameter associated with the activation energy of multiple components, a fitted value, K;

T-absolute temperature, specified constant temperature +273.15, K;

"Q" in the equation (2.3) is replaced with the fifteen parameters in Table 29, plotting is performed by respectively using the logarithms of the fifteen parameters as vertical coordinates and using 1/(T+C) as abscissas, repeated iteration is performed by using a least square method electronic calculation program or a parallax method, and different "C" values are input, until $R^2$ automatically output by the system is $\geq 0.990$, it is considered that the line has been a straight line, and "A, B, C" and $R^2$ in one-to-one correspondence with the obtained fifteen parameters are optimal values thereof, which are listed in Table 29, respectively.

3.5 Prediction of changes in the hardness of S20

As long as the constraint conditions under the actual working conditions are consistent with the accelerated aging test conditions, and only the temperatures are different, the equation (1.3) and the equation (2.3) are applied. When $R^2 \geq 0.999$, it is accurate to predict the hardness aging trend under actual working conditions.

In this embodiment, it is only necessary to substitute the one-to-one corresponding three constants "A, B and C" in Table 29 and any service temperature below 245°C back into the "general expression formula" in Table 29, that is, the equation (2.3) or its shifted variant form, so as to respectively figure out new "Q" values of the one-to-one corresponding fifteen parameters, and then any service time and the new "Q" values of the fifteen parameters are substituted back into the equation (1.3), so as to predict the long-term change trend of the hardness of S20 at any temperature and any time, when the compression ratio is 30%.

Table 29  Parameter values of the hardness of S20 in the equation (1.3)

| Serial number | S20, parameters in the micro-gasification expansion oscillation equation (1) | Parameter values of various temperatures | | | | | |
|---|---|---|---|---|---|---|---|
| | | 245°C | 218°C | 195°C | 150°C | 97°C | 85°C |
| 1 | $H_\infty$ hardness after weathering for more than 100 years, % | 78 | 77 | 77 | 75 | 73 | 73 |
| 2 | $t_0$ migration lag time of low molecular substances, h | 4.00 | 4.90 | 5.90 | 8.90 | 17.0 | 20.0 |
| 3 | $\Delta H_1$ micro-gasification internal influence constant, % | -5.0 | -4.8 | -4.7 | -4.4 | -4.0 | -3.9 |
| 4 | $\Delta H_2$ micro-gasification interface influence constant, % | 0.0 | 0.0 | 0.0 | 0.0 | 0.0 | 0.0 |
| 5 | $\Delta H_3$ mechanical stress influence constant, % | 12.0 | 12.0 | 12.0 | 12.0 | 12.0 | 12.0 |
| 6 | $H_{0\oplus}$ hardness in micro-gasification phase state, % | 59.0 | 59.2 | 59.4 | 59.7 | 60.0 | 60.1 |
| 7 | $H_{0\ominus}$ initial hardness before aging, % | 64.0 | 64.0 | 64.0 | 64.0 | 64.0 | 64.0 |

| 8 | $\beta_1$ migration oscillation frequency coefficient, dimensionless | 29.60 | 14.60 | 6.80 | 1.80 | 0.30 | 0.19 |
| 9 | $\beta_2$ volatilization oscillation frequency coefficient, dimensionless | 0.0 | 0.0 | 0.0 | 0.0 | 0.0 | 0.0 |
| 10 | $k_1$ migration rate constant, 1/h | 0.70 | 0.62 | 0.56 | 0.43 | 0.30 | 0.27 |
| 11 | $k_2$ volatilization rate constant, 1/h | 0.0 | 0.0 | 0.0 | 0.0 | 0.0 | 0.0 |
| 12 | $k_3$ relaxation rate constant, 1/h | 7.80 | 6.30 | 5.25 | 3.55 | 2.00 | 1.73 |
| 13 | k chemical reaction rate constant, 1/h | 0.115 | 0.073 | 0.046 | 0.019 | 0.0053 | 0.0039 |
| 14 | $\theta_1$ migration oscillation frequency index, dimensionless | 1.00 | 0.80 | 0.65 | 0.42 | 0.22 | 0.19 |
| 15 | $\theta_2$ volatilization oscillation frequency index, dimensionless | 1.0 | 1.0 | 1.0 | 1.0 | 1.0 | 1.0 |

(Continue) Table 29 Parameter and constant values of the hardness of S20 in the equation (1.3)

| Serial number | S20, parameters in the micro-gasification expansion oscillation equation (1) | Constant values of various parameters | | | | |
| --- | --- | --- | --- | --- | --- | --- |
| | | General expression formula | A | B | C | $R^2$ |
| 1 | $H_\infty$ hardness after weathering for more than 100 years, % | $\ln H_\infty = \dfrac{A}{T+C} + B$ | -71.40 | 4.497 | -15 | 0.9999 |
| 2 | $t_0$ migration lag time of low molecular substances, h | $\ln t_0 = \dfrac{A}{T+C} + B$ | 1867 | -2.215 | 0 | 0.9999 |
| 3 | $\Delta H_1$ micro-gasification internal influence | $\Delta H_1 = \Delta H_{0\oplus} - \Delta H_{0\ominus}$ | -4397.1 | 4.096 | 1250 | 0.9998 |

| | | | | | | |
|---|---|---|---|---|---|---|
| | constant, % | | | | | |
| 4 | $\Delta H_2$ micro-gasification interface influence constant, % | $\ln(\Delta H_2) = \dfrac{A}{T+C} + B$ | 0.00 | -13.82 | 0 | - |
| 5 | $\Delta H_3$ mechanical stress influence constant, % | $\ln(\Delta H_3) = \dfrac{A}{T+C} + B$ | 0.00 | 2.4849 | 0 | - |
| 6 | $H_{0\oplus}$ hardness in micro-gasification phase state, % | $H_{0\oplus} = A(1 + e^{\frac{-B}{T+C}})$ | 64.0 | 4503.4 | 1250 | 0.9998 |
| 7 | $H_{0\ominus}$ initial hardness before aging, % | $\ln H_{0\ominus} = \dfrac{A}{T+C} + B$ | 0.00 | 4.159 | 0 | - |
| 8 | $\beta_1$ migration oscillation frequency coefficient, dimensionless | $\ln\beta_1 = \dfrac{A}{T+C} + B$ | -52131 | 41.51 | 850 | 0.9998 |
| 9 | $\beta_2$ volatilization oscillation frequency coefficient, dimensionless | $\ln\beta_2 = \dfrac{A}{T+C} + B$ | 0.00 | -13.82 | 0 | - |
| 10 | $k_1$ migration rate constant, 1/h | $\ln k_1 = \dfrac{A}{T+C} + B$ | -1094 | 1.749 | 0 | 0.9999 |
| 11 | $k_2$ volatilization rate constant, 1/h | $\ln k_2 = \dfrac{A}{T+C} + B$ | 0.00 | -13.82 | 0 | - |
| 12 | $k_3$ relaxation rate constant, 1/h | $\ln k_3 = \dfrac{A}{T+C} + B$ | -3180 | 6.808 | 150 | 0.9999 |
| 13 | k chemical reaction rate constant, 1/h | $\ln k = \dfrac{A}{T+C} + B$ | -9890 | 10.71 | 250 | 0.9999 |
| 14 | $\theta_1$ migration oscillation frequency index, dimensionless | $\ln\theta_1 = \dfrac{A}{T+C} + B$ | -2535 | 4.376 | 60 | 0.9999 |

| 15 | $\theta_2$ volatilization oscillation frequency index, dimensionless | $\ln\theta_2 = \dfrac{A}{T+C} + B$ | 0.00 | 0.0 | 0 | - |
|---|---|---|---|---|---|---|

1) The equation (1.3) is used for predicting the time-varying changes of the hardness under the compression ratio of 30% and at the service temperatures of 245°C, 218°C, 195°C, 150°C, 97°C and 85°C, which are listed in Table 28-1 to Table 28-6; and plotting is performed by using the hardness as vertical coordinates and the service times as abscissas, and trend curves corresponding to Table 28-1 to Table 28-6 are shown in Fig. 43 to Fig. 48.

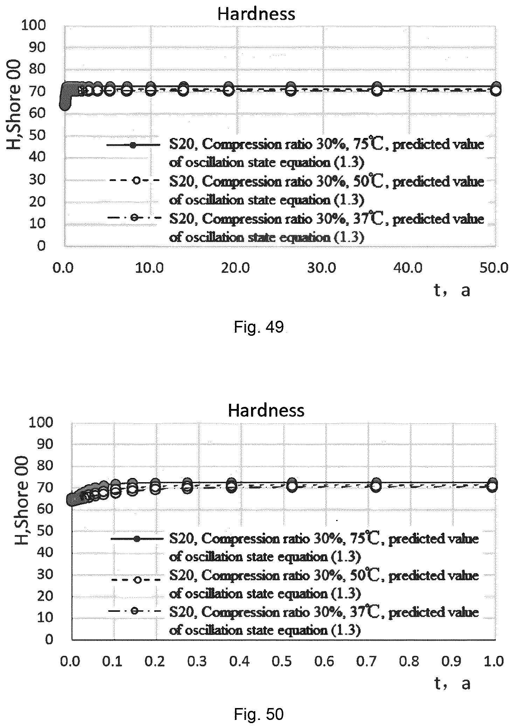

2) The equation (1.3) is used for predicting the change trends of the compression set rate with the service time under the compression ratio of 30% and at the service temperatures of 75°C, 50°C and 37°C, which are listed in Table 30; and plotting is performed by using the hardness as vertical coordinates and the service times as abscissas, and the trend curves corresponding to Table 30 are shown in Fig. 49 and Fig.50.

3) The standard deviation between the predicted result and the measured value of the hardness is within the range of $\pm 1.96$) Th sigma.

Table 30   When the compression ratio is 30%, the long-term change trend of the hardness $H_t$, Shore 00 of S20 predicted by using the equation (1.3)

| S20 | Aging time t, year | | | | | | | | | | |
|---|---|---|---|---|---|---|---|---|---|---|---|
| | 0.0⊖ | 0.0⊕ | 0.107 | 0.148 | 0.20 | 0.28 | 0.39 | 0.54 | 0.75 | 1.03 | 1.42 |
| 75°C | 64 | 65 | 72 | 72 | 72 | 72 | 72 | 72 | 72 | 72 | 72 |
| 50°C | 64 | 64 | 69 | 70 | 71 | 71 | 71 | 71 | 71 | 71 | 71 |
| 37°C | 64 | 64 | 68 | 69 | 69 | 70 | 70 | 70 | 70 | 70 | 70 |

(Continue) Table 30   When the compression ratio is 30%, the long-term change trend of the hardness $H_t$, Shore 00 of S20 predicted by using the equation (1.3)

| S20 | Aging time t, year | | | | | | | | | | |
|---|---|---|---|---|---|---|---|---|---|---|---|
| | 2.0 | 2.7 | 3.8 | 5.2 | 7.2 | 9.9 | 13.7 | 18.9 | 26.2 | 36 | 50 |
| 75°C | 72 | 72 | 72 | 72 | 72 | 72 | 72 | 72 | 72 | 72 | 72 |
| 50°C | 71 | 71 | 71 | 71 | 71 | 71 | 71 | 71 | 71 | 71 | 71 |
| 37°C | 70 | 70 | 70 | 70 | 70 | 70 | 70 | 70 | 70 | 70 | 70 |

4. Fourth embodiment: evaluation or prediction of the service life of tensile strength

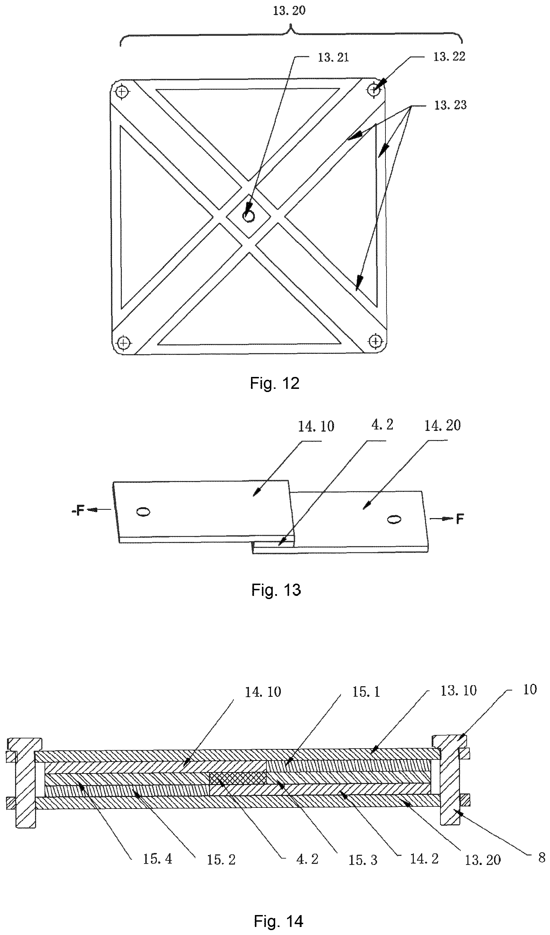

The fourth embodiment discloses another form of the test method and algorithm for the aging life of the new energy heat management composite, and the use thereof in the present invention. The long-term change trend of the tensile strength of an interface target specimen during long-term service under actual working conditions is evaluated or predicted by using a short-term accelerated aging test method, including: using fixture compression specimens with a compression ratio of 30%; selecting six constant temperatures for the group of fixture compression specimens within a temperature range of (85-245)°C, and making the group of fixture compression specimens respectively undergo three aging conditions of damp and hot, high and low temperature, and high and low temperature alternating circle in each specified constant temperature environment for a specified time or an accumulative number of cycles; using the fixture compression specimen combined by a square clamping plate shown in Fig. 11 and Fig. 12 to test the tensile strength of a target specimen 4.2 according to test procedures specified in GB/T 1040.3 or GB/T 528 or ASTM D 412; using measured values of the tensile strength to fit corresponding fifteen parameters ($\sigma_\infty$, $\sigma_{0\ominus}$, $\sigma_{0\oplus}$, $\Delta\sigma_1$, $\Delta\sigma_2$, $\Delta\sigma_3$, $t_0$, $\beta_1$, $\beta_2$, $k_1$, $k_2$, $k_3$, $k$, $\theta_1$, $\theta_2$) in a micro-gasification expansion oscillation equation (1.4), and using each fitted parameter value to further fit three constants "A, B and C" contained in a dynamic correlation equation (2.4); substituting the three fitted constant values back into the dynamic correlation equation (2.4), so as to calculate new values of each parameter at the service temperatures of 75°C, 50°C and 37°C, respectively; and substituting the new values of this group of parameters back into the equation (1.4), so as to evaluate or predict the time-varying long-term change trend of the tensile strength of the target specimen after a specified service time or an accumulative number of cycles under the conditions of damp and hot, high and low temperature, and high and low temperature alternating circle at 75°C, 50°C and 37°C. The details of the implementation steps will be further disclosed in the following five chapters 4.1 to 4.5-

4.1 Preparation of the fixture compression specimen

The preparation of the tensile strength specimen includes: firstly paving a layer of isolating membrane (such as a PI membrane) that has no substance exchange and no chemical reaction with the target specimen 4.2 on the bottom of a rigid double-square-shaped mold frame with a depth of 1mm, injecting the uniformly mixed toothpaste-like target specimen 4.2 into the rigid mold frame, paving a layer of isolating membrane on the target specimen 4.2 after leveling the same, planishing the target specimen 4.2 under a press, performing curing molding on the target specimen 4.2, cutting the target specimen 4.2 into a 155×155×1 mm blank specimen, cutting the 155×155×1 mm blank specimen into five dumbbell specimens by using a Type 2 or Type 1B dumbbell cutting die of the GB/T 1040.3 standard, but at this time, the dumbbell specimens should not be separated from the 155×155×1 mm blank specimen or taken out, instead, clamping the 155×155×1 mm blank specimen that has been cut into the dumbbell specimens by using a pair of square compression fixtures and a pair of isolating membranes as shown in Fig. 11 and Fig. 12, wherein the isolating membranes are located among an upper square clamping plate 13.10 and the blank specimen, and between the blank specimen and a lower square clamping plate 13.20, so as to facilitate demolding, fastening the upper square clamping plate 13.10 and the lower square clamping plate 13.20 by using metal screw rods 8, so that the thickness of the blank specimen is compressed and locked to 70% of the initial thickness, that is, the compression ratio is 30%, and then the fixture compression specimen for the tensile strength is molded.

The target specimen 4.2 is a two-component organic silicone heat conducting adhesive product, with a nominal thermal conductivity of 2 W/(m.K) and an initial casting thickness of 1.0mm, and the target specimen is referred to as S20 for short.

4.2 Three aging conditions

In this embodiment, the three aging conditions include: damp and hot, high and low temperature impact, and high and low temperature alternating cycle, and the specific details are completely the same as those disclosed in the second embodiment.

4.3 Test results

4.3.1 Damp and hot aging results

In the four temperature environments of 195°C, 150°C, 97°C and 85°C, the tensile strength after damp and hot is listed in Table 31-3 to Table 31-6, respectively.

4.3.2 Aging results of high and low temperature impact

In the four temperature environments of 245°C, 195°C, 150°C and 97°C, the tensile strength after high and low temperature impact is shown in Table 31-1, and Table 31-3 to Table 31-5, respectively.

4.3.3 Aging results of high and low temperature alternating cycle

In the four temperature environments of 218°C, 195°C, 150°C and 97°C, the tensile strength after high and low temperature alternating cycle is shown in Table 31-2 to Table 31-5, respectively.

4.4 Establishment of a tensile strength equation (1.4)

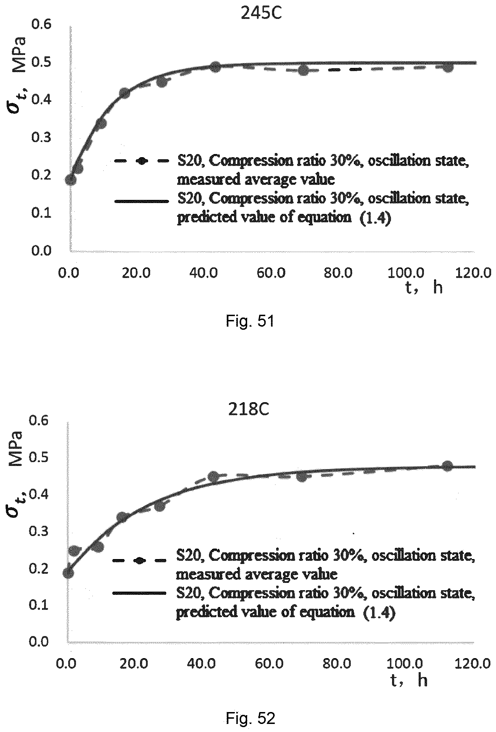

After undergoing the three aging conditions of damp and hot, high and low temperature impact, and high and low temperature alternating cycle in the six temperature environments of 245°C, 218°C, 195°C, 150°C, 97°C and 85°C, the aging trends of the tensile strength corresponding to Table 31-1 to Table 31-6 are shown in Fig. 51 to Fig. 56.

Table 31-1　245℃, Rh<10%, the tensile strength, MPa, the aging trend

| 245℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.08 | 2 | 9 | 16 | 27 | 43 |
| S20 | Damp and hot Measured | 5 | 0.19 | 0.20 | - | - | - | - | - | - |
| | High and low temperature impact Measured | 5 | 0.19 | 0.20 | - | 0.22 | 0.34 | 0.42 | 0.45 | 0.49 |

| Phase state | The number of sub-specimens | | | | | | | | |
|---|---|---|---|---|---|---|---|---|---|
| High and low temperature alternating cycle Measured | 5 | 0.19 | 0.20 | - | - | - | - | - | - |
| Three aging conditions Measured average | 15 | 0.19 | 0.20 | - | 0.22 | 0.34 | 0.42 | 0.45 | 0.49 |
| Three aging conditions Predicted according to the equation (1.4) | 15 | 0.19 | 0.20 | 0.24 | 0.35 | 0.42 | 0.47 | 0.49 | 0.50 |

(Continue) Table 31-1   245℃, Rh<10%, the tensile strength, MPa, the aging trend

| 245℃ Model number | Phase state, compression ratio | The number of sub-specimens | Aging time t, h | | | | | | | |
|---|---|---|---|---|---|---|---|---|---|---|
| | | | 69 | 112 | 192 | 312 | 502 | 817 | | 3400 |
| S20 | Damp and hot Measured | 5 | - | - | - | - | - | - | - | - |
| | High and low temperature impact Measured | 5 | 0.48 | 0.49 | - | - | - | - | - | - |
| | High and low temperature alternating cycle Measured | 5 | - | - | - | - | - | - | - | - |
| | Three aging conditions Measured average | 15 | 0.48 | 0.49 | - | - | - | - | - | - |

| Three aging conditions Predicted according to the equation (1.4) | 15 | 0.50 | 0.50 | 0.50 | 0.50 | 0.50 | 0.50 | | 0.50 |

Table 31-2   218℃, Rh <13%, the tensile strength, MPa, the aging trend

| 218℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.08 | 2 | 9 | 16 | 27 | 43 |
| S20 | Damp and hot Measured | 5 | 0.19 | 0.20 | - | - | - | - | - | - |
| | High and low temperature impact Measured | 5 | 0.19 | 0.20 | - | - | - | - | - | - |
| | High and low temperature alternating cycle Measured | 5 | 0.19 | 0.20 | - | 0.25 | 0.26 | 0.34 | 0.37 | 0.45 |
| | Three aging conditions Measured average | 15 | 0.19 | 0.20 | - | 0.25 | 0.26 | 0.34 | 0.37 | 0.45 |
| | Three aging conditions Predicted according to the equation (1.4) | 15 | 0.19 | 0.20 | 0.28 | 0.33 | 0.39 | 0.43 | 0.46 | 0.48 |

(Continue) Table 31-2   218℃, Rh <13%, the tensile strength, MPa, the aging trend

| 218℃ Model number | Phase state, compression ratio | The number of sub-specimens | Aging time t, h | | | | | | | |
|---|---|---|---|---|---|---|---|---|---|---|
| | | | 69 | 112 | 192 | 312 | 502 | 817 | | 3400 |
| S20 | Damp and hot Measured | 5 | - | - | - | - | - | - | - | - |
| | High and low temperature impact Measured | 5 | - | - | - | - | - | - | - | - |
| | High and low temperature alternating cycle Measured | 5 | 0.45 | 0.48 | - | - | - | - | - | - |
| | Three aging conditions Measured average | 15 | 0.45 | 0.48 | - | - | - | - | - | - |
| | Three aging conditions Predicted according to the equation (1.4) | 15 | 0.48 | 0.48 | 0.48 | 0.48 | 0.48 | 0.48 | | 0.48 |

Table 31-3　195℃, Rh <15%, the tensile strength, MPa, the aging trend

| 195℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.08 | 4 | 12 | 24 | 48 | 72 |
| S20 | Damp and hot Measured | 5 | 0.19 | 0.20 | 0.21 | - | 0.36 | 0.35 | 0.40 | 0.40 |
| | High and low temperature impact Measured | 5 | 0.19 | 0.20 | - | 0.19 | 0.17 | 0.38 | 0.42 | 0.47 |

| | | | | | | | | | | |
|---|---|---|---|---|---|---|---|---|---|---|
| High and low temperature alternating cycle Measured | 5 | 0.19 | 0.20 | - | 0.20 | 0.21 | 0.23 | 0.31 | 0.36 | |
| Three aging conditions Measured average | 15 | 0.19 | 0.20 | 0.21 | 0.20 | 0.25 | 0.32 | 0.38 | 0.41 | |
| Three aging conditions Predicted according to the equation (1.4) | 15 | 0.19 | 0.20 | 0.20 | 0.21 | 0.25 | 0.30 | 0.36 | 0.40 | |

(Continue) Table 31-3    195℃, Rh <15%, the tensile strength, MPa, the aging trend

| 195℃ Model number | Phase state, compression ratio | The number of sub-specimens | Aging time t, h | | | | | | | |
|---|---|---|---|---|---|---|---|---|---|---|
| | | | 120 | 192 | 312 | 505 | 817 | 1322 | | 3400 |
| S20 | Damp and hot Measured | 5 | 0.48 | 0.49 | - | - | - | - | - | - |
| | High and low temperature impact Measured | 5 | 0.45 | 0.49 | - | - | - | - | - | - |
| | High and low temperature alternating cycle Measured | 5 | 0.37 | 0.42 | - | - | - | - | - | - |
| | Three aging conditions Measured average | 15 | 0.43 | 0.47 | - | - | - | - | - | - |

| | Three aging conditions Predicted according to the equation (1.4) | 15 | 0.44 | 0.45 | 0.45 | 0.45 | 0.45 | 0.45 | | 0.45 |
|---|---|---|---|---|---|---|---|---|---|---|

Table 31-4    150℃, Rh <30%, the tensile strength, MPa, the aging trend

| 150 Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.13 | 6 | 24 | 48 | 72 | 120 |
| S20 | Damp and hot Measured | 5 | 0.19 | 0.20 | 0.23 | - | 0.21 | 0.19 | 0.28 | 0.34 |
| | High and low temperature impact Measured | 5 | 0.19 | 0.20 | - | 0.25 | 0.24 | 0.26 | 0.28 | 0.28 |
| | High and low temperature alternating cycle Measured | 5 | 0.19 | 0.20 | - | 0.22 | 0.25 | 0.26 | 0.21 | 0.25 |
| | Three aging conditions Measured average | 15 | 0.19 | 0.20 | 0.23 | 0.24 | 0.23 | 0.24 | 0.26 | 0.29 |
| | Three aging conditions Predicted according to the equation (1.4) | 15 | 0.19 | 0.20 | 0.19 | 0.20 | 0.22 | 0.24 | 0.26 | 0.29 |

(Continue) Table 31-4    150℃, Rh <30%, the tensile strength, MPa, the aging trend

| 150℃ | Phase state, | The number of | Aging time t, h |
|---|---|---|---|

| Model number | compression ratio | sub-specimens | 192 | 312 | 504 | 810 | 1300 | 2100 | | 3400 |
|---|---|---|---|---|---|---|---|---|---|---|
| S20 | Damp and hot Measured | 5 | 0.34 | 0.39 | - | - | - | - | | - |
| | High and low temperature impact Measured | 5 | 0.32 | 0.38 | - | - | - | - | | - |
| | High and low temperature alternating cycle Measured | 5 | 0.28 | 0.28 | - | - | - | - | | - |
| | Three aging conditions Measured average | 15 | 0.31 | 0.35 | - | - | - | - | | - |
| | Three aging conditions Predicted according to the equation (1.4) | 15 | 0.33 | 0.37 | 0.39 | 0.41 | 0.41 | 0.41 | | 0.41 |

Table 31-5   97℃, Rh 97%, the tensile strength, MPa, the aging trend

| 97℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.22 | 10 | 48 | 72 | 120 | 192 |
| S20 | Damp and hot Measured | 5 | 0.19 | 0.19 | 0.22 | - | 0.20 | 0.21 | 0.19 | 0.20 |
| | High and low temperature impact | 5 | 0.19 | 0.19 | - | 0.20 | 0.19 | 0.17 | 0.18 | 0.20 |

| | | | 312 | 504 | 810 | 1300 | 2100 | 2500 | 3400 |
|---|---|---|---|---|---|---|---|---|---|
| | Measured | | | | | | | | |
| | High and low temperature alternating cycle Measured | 5 | 0.19 | 0.19 | - | 0.17 | 0.17 | 0.20 | 0.17 | 0.21 |
| | Three aging conditions Measured average | 15 | 0.19 | 0.19 | 0.22 | 0.19 | 0.19 | 0.19 | 0.18 | 0.20 |
| | Three aging conditions Predicted according to the equation (1.4) | 15 | 0.19 | 0.19 | 0.19 | 0.19 | 0.19 | 0.20 | 0.20 | 0.21 |

(Continue) Table 31-5   97℃, Rh 97%, the tensile strength, MPa, the aging trend

| 97℃ Model number | Phase state, compression ratio | The number of sub-specimens | Aging time t, h | | | | | | | |
|---|---|---|---|---|---|---|---|---|---|---|
| | | | 312 | 504 | 810 | 1300 | 2100 | 2500 | | 3400 |
| S20 | Damp and hot Measured | 5 | 0.21 | 0.23 | - | - | - | - | - | - |
| | High and low temperature impact Measured | 5 | 0.20 | 0.21 | - | - | - | - | - | - |
| | High and low temperature alternating cycle Measured | 5 | 0.21 | 0.22 | - | - | - | - | - | - |
| | Three aging conditions Measured | 15 | 0.21 | 0.22 | - | - | - | - | - | - |

| | | | | | | | | | | |
|---|---|---|---|---|---|---|---|---|---|---|
| | average | | | | | | | | | |
| | Three aging conditions Predicted according to the equation (1.4) | 15 | | 0.22 | 0.23 | 0.25 | 0.28 | 0.31 | 0.32 | 0.34 |

Table 31-6   85℃, Rh 85%, the tensile strength, MPa, the aging trend

| 85℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.22 | 48 | 72 | 120 | 192 | 312 |
| S20 | Damp and hot Measured | 5 | 0.19 | 0.19 | 0.24 | 0.20 | 0.21 | 0.19 | 0.22 | 0.21 |
| | High and low temperature impact Measured | 5 | 0.19 | 0.19 | - | - | - | - | - | - |
| | High and low temperature alternating cycle Measured | 5 | 0.19 | 0.19 | - | - | - | - | - | - |
| | Three aging conditions Measured average | 15 | 0.19 | 0.19 | 0.24 | 0.20 | 0.21 | 0.19 | 0.22 | 0.21 |
| | Three aging conditions Predicted according to the equation (1.4) | 15 | 0.19 | 0.19 | 0.19 | 0.19 | 0.19 | 0.20 | 0.20 | 0.21 |

(Continue) Table 31-6   85℃, Rh 85%, the tensile strength, MPa, the aging trend

| 85℃ Model number | Phase state, compression ratio | The number of sub-specimens | Aging time t, h | | | | | | | |
|---|---|---|---|---|---|---|---|---|---|---|
| | | | 504 | 810 | 1300 | 2100 | 2500 | 4045 | | 8000 |
| S20 | Damp and hot Measured | 5 | 0.20 | 0.21 | 0.24 | 0.29 | - | - | - | - |
| | High and low temperature impact Measured | 5 | - | - | - | - | - | - | - | - |
| | High and low temperature alternating cycle Measured | 5 | - | - | - | - | - | - | - | - |
| | Three aging conditions Measured average | 15 | 0.20 | 0.21 | 0.24 | 0.29 | - | - | - | - |
| | Three aging conditions Predicted according to the equation (1.4) | 15 | 0.22 | 0.23 | 0.25 | 0.27 | 0.28 | 0.30 | | 0.33 |

4.4.1 T inspection

The T inspection indicates that the tensile strength under the three aging conditions of damp and hot, high and low temperature impact, and high and low temperature alternating cycle shows similar aging change trends, and thus a larger data specimen may be assembled for evaluation or prediction.

4.4.2 Parameter fitting in equation (1.4)

In another use of the calculation method of the micro-gasification expansion oscillation equation (1) of the present invention, a general symbol (P) of the physical, chemical and electrical properties in the equation (1) is replaced with a specific symbol ($\sigma$) of the tensile strength, so as to convert the equation (1) into an equation (1.4):

$$\sigma_t = \sigma_\infty + \left\{ \sigma_{0\ominus} + \left[ \Delta\sigma_1 e^{-k_1 t} \times \mathrm{Sin}\beta_1 \left( \frac{t}{t_0} - 1 \right)^{\theta_1} \pi + \Delta\sigma_2 e^{-k_2 t} \times \mathrm{Sin}\beta_2 \left( \frac{t}{t_0} - 1 \right)^{\theta_2} \pi + \right.\right.$$

$$\left.\left. \Delta\sigma_3 e^{-k_3 t} \right] - \sigma_\infty \right\} e^{-kt} \quad \dots (1.4)$$

in the equation (1.4)

$\sigma$-the tensile strength of the target specimen, and the measured values are listed in Table 31-1 to Table 31-6 for fitting and verification;

$\sigma_t$-the tensile strength of the target specimen at any specified constant temperature for any service time, a predicted value;

$\sigma_\infty$-the tensile strength after weathering for more than 100 years, determined by numerical simulation of a material formula, or a fitted value;

$\sigma_{0\ominus}$-initial tensile strength before aging, a measured value;

$\sigma_{0\oplus}$-the tensile strength during micro-gasification expansion; a fitted value;

Sin-micro-gasification oscillation trigonometric function;

$\Delta\sigma_1$-micro-gasification internal influence parameter, $\Delta\sigma_1 = (\sigma_{0\oplus} - \sigma_{0\ominus})$;

$\Delta\sigma_2$-Micro-gasification interface influence parameter, a fitted value;

$\Delta\sigma_3$-mechanical stress influence parameter, a fitted value;

t-aging time or service time, determined by a specified time or an accumulative number of cycles;

$t_0$-migration lag time of low molecular substances, a fitted value;

$\beta_1$-migration oscillation frequency coefficient, a fitted value;

$\beta_2$-volatilization oscillation frequency coefficient, a fitted value;

$k_1$-migration rate parameter, a fitted value;

$k_2$-volatilization rate parameter, a fitted value;

$k_3$-relaxation rate parameter, a fitted value;

k-chemical reaction rate parameter, a fitted value;

$\theta_1$-migration oscillation frequency index, a fitted value; and

$\theta_2$-volatilization oscillation frequency index, a fitted value.

In the formula (1.4) in this embodiment, some of the fifteen parameters contained in the tensile strength are negligible, and thus are assigned as "0"; although all the parameters are difficult to be obtained directly through linear fitting, the average values of the measured values in Table 31-1 to Table 31-6 are taken as specimens, starting with the "0" assignment, the assignment is tentatively increased step by step with a step pitch as small as possible, the parameter is repeatedly and iteratively input into the equation (1.4) by using an electronic calculation program or a parallax method, after each parameter is iterated for more than 50 times, the standard deviation of a difference value between a calculated value ($\sigma_t$) and a measured value ($\sigma$) converges to the minimum, and optimal values of the fifteen parameters "Q" of the thermal conductivity at each temperature are obtained, and the results of iterative optimization are listed in Table 32. Because of the mathematical frequency doubling effect, when there is more than one optimal value among the fitted values of the fifteen parameters, only the smaller group of fifteen "Q" values closest to "1 time" is selected as optimal parameters.

4.4.3 Constant fitting in equation (2.4)

In the fourth embodiment, for the fifteen parameters in the tensile strength equation (1.4) contained in Table 32, on mechanism, each parameter does not change with time, but only changes with temperature. Each parameter that changes with temperature further includes the constants expressed by the corresponding three symbols "A, B and C" in the equation (2), and during the fitting process, the constants, which are corresponding to the parameters in the tensile strength equation (1.4), in the equation (2) need to be replaced with corresponding symbols, so as to convert the equation (2) into an equation (2.4):

$$\ln Q = \frac{A}{T+C} + B \dots\dots\dots\dots\dots\dots\dots\dots\dots\dots\dots\dots\dots\dots\dots\dots (2.4)$$

in the equation (2.4),

Q-corresponding to one of the fifteen parameters in the equation (1.4) at any temperature;

A-empirical constant of each parameter associated with the reaction activation energy and the diffusion activation energy of multiple components, a fitted value, K;

B-empirical constant of each parameter associated with the chemical reaction rate and the diffusion rate of multiple components, a fitted value, dimensionless;

C-conformal constant of Fourier series transformation of each parameter associated with the activation energy of multiple components, a fitted value, K;

T-absolute temperature, specified constant temperature +273.15, K;

"Q" in the equation (2.4) is replaced with the fifteen parameters in Table 32, plotting is performed by respectively using the logarithms of the fifteen parameters as vertical coordinates and using $1/(T+C)$ as abscissas, repeated iteration is performed by using a least square method electronic calculation program or a parallax method, and different "C" values are input, until $R^2$ automatically output by the system is $\geq 0.990$, it is considered that the line has been a straight line, and "A, B, C" and $R^2$ in one-to-one correspondence with the obtained fifteen parameters of the tensile strength are optimal values thereof, which are listed in Table 32, respectively.

4.5 Prediction of changes in the tensile strength of S20

As long as the constraint conditions under the actual working conditions are consistent with the accelerated aging test conditions, and only the temperatures are different, the equation (1.4) and the equation (2.4) are applied. When $R^2 \geq 0.999$, it is accurate to predict the aging trend of the tensile strength under actual working conditions.

In this embodiment, it is only necessary to substitute the one-to-one corresponding three constants "A, B and C" in Table 32 and any service temperature below 245°C into the "general expression formula" in Table 32, that is, the equation (2.4) or its shifted variant form, so as to respectively figure out new "Q" values of the one-to-one corresponding fifteen parameters, and then any service time and the new "Q" values of the fifteen parameters are substituted back into the equation (1.4), so as to predict the long-term change trend of the tensile strength of S20 at any temperature and any time, when the compression ratio is 30%.

1) The equation (1.4) is used for predicting the time-varying changes of the tensile strength under the compression ratio of 30% and at the service temperatures of 245°C, 218°C, 195°C, 150°C, 97°C and 85°C, which are listed in Table 31-1 to Table 31-6; and plotting is performed by using the tensile strength as vertical coordinates and the service times as abscissas, and trend curves corresponding to Table 31-1 to Table 31-6 are shown in Fig. 51 to Fig. 56.

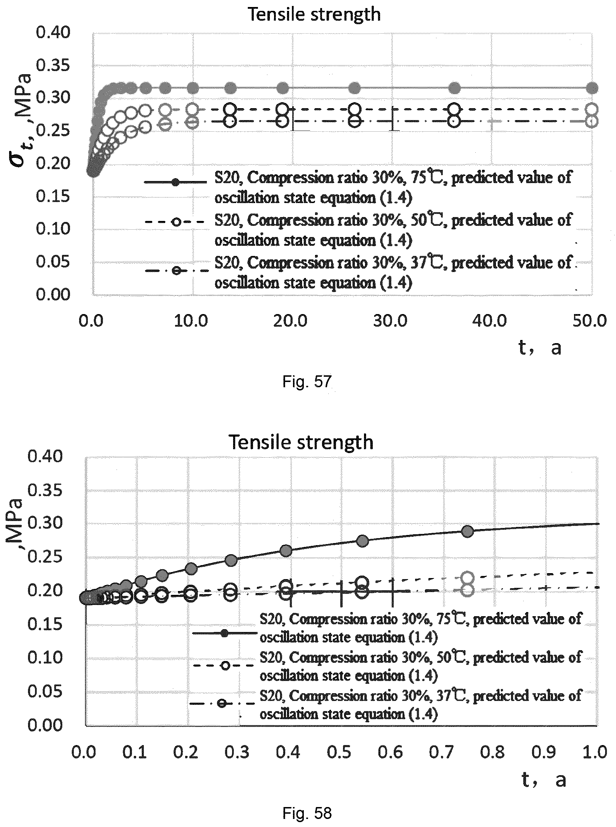

2) The equation (1.4) is used for predicting the change trends of the tensile strength with the service time under the compression ratio of 30% and at the service temperatures of 75°C, 50°C and 37°C, which are listed in Table 33; and plotting is performed by using the tensile strength as vertical coordinates and the service times as abscissas, and the trend curves corresponding to Table 33 are shown in Fig. 57 and Fig. 58.

3) The standard deviation between the predicted result and the measured value of the tensile strength is within the range of $\pm 1.96$) Th sigma.

Table 32    Parameter values of the tensile strength of S20 in the equation (1.4)

| Serial number | S20, parameters in the micro-gasification expansion oscillation equation (1) | Parameter values of various temperatures | | | | | |
|---|---|---|---|---|---|---|---|
| | | 245°C | 218°C | 195°C | 150°C | 97°C | 85°C |
| 1 | $\sigma_\infty$ tensile strength after weathering for more than 100 years, % | 0.50 | 0.48 | 0.46 | 0.41 | 0.34 | 0.33 |
| 2 | $t_0$ migration lag time of low molecular substances, h | 4.00 | 4.90 | 5.90 | 8.90 | 17.0 | 20.0 |
| 3 | $\Delta\sigma_1$ micro-gasification internal influence constant, % | 0.0 | 0.0 | 0.0 | 0.0 | 0.0 | 0.0 |
| 4 | $\Delta\sigma_2$ micro-gasification interface influence constant, % | 0.0 | 0.0 | 0.0 | 0.0 | 0.0 | 0.0 |
| 5 | $\Delta\sigma_3$ mechanical stress influence constant, % | 0.0 | 0.0 | 0.0 | 0.0 | 0.0 | 0.0 |

| 6 | $\sigma_{0\oplus}$ tensile strength in micro-gasification phase state, % | 0.202 | 0.200 | 0.198 | 0.195 | 0.193 | 0.192 |
|---|---|---|---|---|---|---|---|
| 7 | $\sigma_{0\ominus}$ initial tensile strength before aging, % | 0.190 | 0.190 | 0.190 | 0.190 | 0.190 | 0.190 |
| 8 | $\beta_1$ migration oscillation frequency coefficient, dimensionless | 29.60 | 14.60 | 6.80 | 1.80 | 0.30 | 0.19 |
| 9 | $\beta_2$ volatilization oscillation frequency coefficient, dimensionless | 0.0 | 0.0 | 0.0 | 0.0 | 0.0 | 0.0 |
| 10 | $k_1$ migration rate constant, 1/h | 0.70 | 0.62 | 0.56 | 0.43 | 0.30 | 0.27 |
| 11 | $k_2$ volatilization rate constant, 1/h | 0.0 | 0.0 | 0.0 | 0.0 | 0.0 | 0.0 |
| 12 | $k_3$ relaxation rate constant, 1/h | 7.80 | 6.30 | 5.25 | 3.55 | 2.00 | 1.73 |
| 13 | k chemical reaction rate constant, 1/h | 0.082 | 0.042 | 0.022 | 0.0053 | 0.00068 | 0.00040 |
| 14 | $\theta_1$ migration oscillation frequency index, dimensionless | 1.00 | 0.80 | 0.65 | 0.42 | 0.22 | 0.19 |
| 15 | $\theta_2$ volatilization oscillation frequency index, dimensionless | 1.0 | 1.0 | 1.0 | 1.0 | 1.0 | 1.0 |

(Continue) Table32　Parameter and constant values of the tensile strength of S20 in the equation (1.4)

| Serial number | S20, parameters in the micro-gasification expansion oscillation equation (1) | Constant values of various parameters | | | | |
|---|---|---|---|---|---|---|
| | | General expression formula | A | B | C | $R^2$ |

| | | | A | B | C | |
|---|---|---|---|---|---|---|
| 1 | $\sigma_\infty$ tensile strength after weathering for more than 100 years, % | $\ln \sigma_\infty = \dfrac{A}{T + C} + B$ | -379.5 | 0.120 | -50 | 0.9997 |
| 2 | $t_0$ migration lag time of low molecular substances, h | $\ln t_0 = \dfrac{A}{T + C} + B$ | 1867 | -2.215 | 0 | 0.9999 |
| 3 | $\Delta\sigma_1$ micro-gasification internal influence constant, % | $\Delta\lambda_1 = \Delta\sigma_{0\oplus} - \Delta\sigma_{0\ominus}$ | -1935 | -0.559 | -15 | 0.9996 |
| 4 | $\Delta\sigma_2$ micro-gasification interface influence constant, % | $\ln (\Delta\sigma) = \dfrac{A}{T + C} + B$ | 0.00 | -13.82 | 0 | - |
| 5 | $\Delta\sigma_3$ mechanical stress influence constant, % | $\ln (\Delta\sigma_3) = \dfrac{A}{T + C} + B$ | 0.00 | -13.82 | 0 | - |
| 6 | $\sigma_{0\oplus}$ tensile strength in micro-gasification phase state, % | $\Delta\sigma_{0\oplus} = A(1 + e^{\frac{-B}{T+C}})$ | 0.190 | 1296 | -65 | 0.9997 |
| 7 | $\sigma_{0\ominus}$ initial tensile strength before aging, % | $\ln \sigma_{0\ominus} = \dfrac{A}{T + C} + B$ | 0.00 | -1.661 | 0 | - |
| 8 | $\beta_1$ migration oscillation frequency coefficient, dimensionless | $\ln\beta_1 = \dfrac{A}{T + C} + B$ | -52131 | 41.51 | 850 | 0.9998 |
| 9 | $\beta_2$ volatilization oscillation frequency coefficient, dimensionless | $\ln\beta_2 = \dfrac{A}{T + C} + B$ | 0.00 | -13.82 | 0 | - |
| 10 | $k_1$ migration rate constant, 1/h | $\ln k_1 = \dfrac{A}{T + C} + B$ | -1094 | 1.749 | 0 | 0.9999 |
| 11 | $k_2$ volatilization rate constant, 1/h | $\ln k_2 = \dfrac{A}{T + C} + B$ | 0.00 | -13.82 | 0 | - |
| 12 | $k_3$ relaxation rate constant, 1/h | $\ln k_3 = \dfrac{A}{T + C} + B$ | -3180 | 6.808 | 150 | 0.9999 |

| 13 | k chemical reaction rate constant, 1/h | $\ln k = \dfrac{A}{T+C} + B$ | -6472 | 9.733 | 10 | 0.9999 |
|----|----|----|----|----|----|----|
| 14 | $\theta_1$ migration oscillation frequency index, dimensionless | $\ln \theta_1 = \dfrac{A}{T+C} + B$ | -2431 | 4.269 | 50 | 0.9999 |
| 15 | $\theta_2$ volatilization oscillation frequency index, dimensionless | $\ln \theta_2 = \dfrac{A}{T+C} + B$ | 0.00 | 0.0 | 0 | - |

Table 33 When the compression ratio is 30%, the long-term change trend of the tensile strength $\sigma_t$, MPa of S20 predicted by using the equation (1.4)

| S20 | Aging time t, year | | | | | | | | | | |
|-----|------|------|-------|-------|------|------|------|------|------|------|------|
|     | 0.0⊖ | 0.0⊕ | 0.107 | 0.148 | 0.20 | 0.28 | 0.39 | 0.54 | 0.75 | 1.03 | 1.42 |
| 75°C | 0.19 | 0.19 | 0.22 | 0.22 | 0.23 | 0.25 | 0.26 | 0.28 | 0.29 | 0.30 | 0.31 |
| 50°C | 0.19 | 0.19 | 0.20 | 0.20 | 0.20 | 0.20 | 0.21 | 0.21 | 0.22 | 0.23 | 0.24 |
| 37°C | 0.19 | 0.19 | 0.19 | 0.19 | 0.19 | 0.19 | 0.20 | 0.20 | 0.20 | 0.21 | 0.21 |

(Continue) Table 33 When the compression ratio is 30%, the long-term change trend of the tensile strength $\sigma_t$, MPa of S20 predicted by using the equation (1.4)

| S20 | Aging time t, year | | | | | | | | | | |
|-----|-----|-----|-----|-----|-----|-----|------|------|------|------|------|
|     | 2.0 | 2.7 | 3.8 | 5.2 | 7.2 | 9.9 | 13.7 | 18.9 | 26.2 | 36 | 50 |
| 75°C | 0.31 | 0.32 | 0.32 | 0.32 | 0.32 | 0.32 | 0.32 | 0.32 | 0.32 | 0.32 | 0.32 |
| 50°C | 0.25 | 0.26 | 0.27 | 0.28 | 0.28 | 0.28 | 0.28 | 0.28 | 0.28 | 0.28 | 0.28 |
| 37°C | 0.22 | 0.23 | 0.23 | 0.24 | 0.25 | 0.26 | 0.26 | 0.26 | 0.26 | 0.26 | 0.26 |

5. Fifth embodiment: evaluation or prediction of the service life of shear bonding strength

The fifth embodiment discloses another form of the test method and algorithm for the aging life of the new energy heat management composite, and the use thereof in the present invention. The long-term change trend of the shear bonding strength of an interface target specimen during long-term service under actual working conditions is evaluated or predicted by using a short-term accelerated aging test method, including: using fixture compression specimens with a compression ratio of 30%; selecting six constant temperatures for the group of fixture compression specimens within a temperature range of (85-245)°C, and making the group of fixture compression specimens respectively undergo three aging conditions of damp and hot, high and low temperature, and high and low temperature alternating circle in each specified constant temperature environment for a specified time or an accumulative number of cycles; using the fixture compression specimen combined by a square clamping plate shown in Fig. 11 and Fig. 12 to test the shear bonding strength of a target specimen 4.2 according to test procedures specified in GB/T 7124 or ISO 4587 or ASTM D 1002; using measured values of the shear bonding strength to fit corresponding fifteen parameters ($S_\infty$, $S_{0\ominus}$, $S_{0\oplus}$, $\Delta S_1$, $\Delta S_2$, $\Delta S_3$, $t_0$, $\beta_1$, $\beta_2$, $k_1$, $k_2$, $k_3$, $k$, $\theta_1$, $\theta_2$) in a micro-gasification expansion oscillation equation (1.5), and using each fitted parameter value to further fit three constants "A, B and C" contained in a dynamic correlation equation (2.5); substituting the three fitted constant values back into the dynamic correlation equation (2.5), so as to calculate new values of each parameter at the service temperatures of 75°C, 50°C and 37°C, respectively; and substituting the new values of this group of parameters back into the equation (1.5), so as to evaluate or predict the time-varying long-term change trend of the shear bonding strength of the target specimen after a specified service time or an accumulative number of cycles under the conditions of damp and hot, high and low temperature, and high and low temperature alternating circle at 75°C, 50°C and 37°C. The details of the implementation steps will be further disclosed in the following five chapters 5.1 to 5.5-

5.1 Preparation of the fixture compression specimen

As shown in Fig. 13, according to test procedures and requirements specified in GB/T 7124 or ISO 4587 or ASTM D 1002, lap joint bonding sheets are prepared, including: an upper lap joint bonding sheet 14.10, the target specimen 4.2 and a lower lap joint bonding sheet 14.20; and the upper lap joint bonding sheet 14.10 and the lower lap joint bonding sheet 14.20 are bonded and cured into an integral lap joint specimen by the target specimen 4.2.

As shown in Fig. 14, the fixture compression specimen for the shear bonding strength includes: screw rods 8, nuts 10, an upper square clamping plate 13.10, a lower square clamping plate 13.20, an upper lap joint bonding sheet 14.10, a lower lap joint bonding sheet 14.20, an upper positioning sheet A 15.1, a lower positioning sheet A 15.2, an upper positioning sheet B 15.3, and a lower positioning sheet B 15.4, wherein the upper lap joint bonding sheet 14.10 and the lower lap joint bonding sheet 14.20 are bonded and cured into an integral lap joint specmen by the target specimen 4.2; the upper square clamping plate 13.10, the lower square clamping plate 13.20, the upper lap joint bonding sheet 14.10, the integral lap joint specmen, the upper positioning sheet A 15.1, the lower positioning sheet A 15.2, the upper positioning sheet B 15.3 and the lower positioning sheet B 15.4 are fastened into an entirety by the screw rods 8 and nuts 10, so that the thickness of the target specimen 4.2 is compressed to 70% of the initial value, that is, the compression ratio is 30%; and the upper positioning sheet A 15.1, the lower positioning sheet A 15.2, the upper positioning sheet B 15.3 and the lower positioning sheet B 15.4 are used for balancing the compression torque and limiting the compression thickness of the target specimen 4.2.

The upper square clamping plate 13.10 is consistent with Fig. 11, and the lower square clamping plate 13.20 is consistent with Fig. 12.

The target specimen 4.2 is a two-component organic silicone heat conducting adhesive product, with a nominal thermal conductivity of 2 W/(m.K) and an initial casting thickness of 0.40mm, and the target specimen is referred to as S20 for short.

5.2 Three aging conditions

In this embodiment, the three aging conditions include: damp and hot, high and low temperature impact, and high and low temperature alternating cycle, and the specific details are completely the same as those disclosed in the second embodiment.

5.3 Test results

5.3.1 Damp and hot aging results

In the four temperature environments of 195°C, 150°C, 97°C and 85°C, the shear bonding strength after damp and hot is listed in Table 34-3 to Table 34-6, respectively.

5.3.2 Aging results of high and low temperature impact

In the four temperature environments of 245°C, 195°C, 150°C and 97°C, the shear bonding strength after high and low temperature impact is shown in Table 34-1, and Table 34-3 to Table 34-5, respectively.

5.3.3 Aging results of high and low temperature alternating cycle

In the four temperature environments of 218°C, 195°C, 150°C and 97°C, the shear bonding strength after high and low temperature alternating cycle is shown in Table 31-2 to Table 31-5, respectively.

5.4 Establishment of a shear bonding strength equation (1.5)

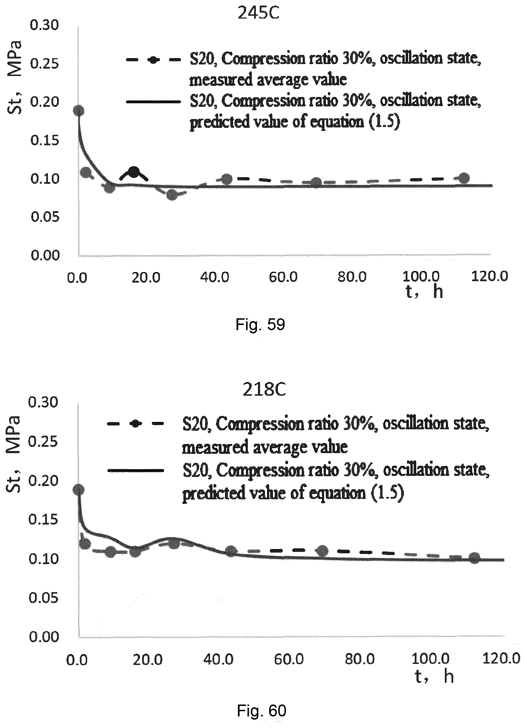

After undergoing the three aging conditions of damp and hot, high and low temperature impact, and high and low temperature alternating cycle in the six temperature environments of 245°C, 218°C, 195°C, 150°C, 97°C and 85°C, the aging trends of the shear bonding strength corresponding to Table 34-1 to Table 34-6 are shown in Fig. 59 to Fig. 64.

Table 34-1　245℃, Rh<10%, the shear bonding strength, MPa, the aging trend

| 245℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.08 | 2 | 9 | 16 | 27 | 43 |
| S20 | Damp and hot Measured | 5 | - | - | - | - | - | - | - | - |
| | High and low temperature impact Measured | 5 | - | - | - | 0.11 | 0.09 | 0.11 | 0.08 | 0.10 |
| | High and low temperature alternating cycle Measured | 5 | - | - | - | - | - | - | - | - |
| | Three aging conditions Measured average | 15 | - | - | - | 0.11 | 0.09 | 0.11 | 0.08 | 0.10 |

| | | | | | | | | | |
|---|---|---|---|---|---|---|---|---|---|
| Three aging conditions Predicted according to the equation (1.5) | 15 | 0.19 | 0.20 | 0.17 | 0.13 | 0.10 | 0.09 | 0.09 | 0.09 |

(Continue) Table 34-1 245℃, Rh<10%, the shear bonding strength, MPa, the aging trend

| 245℃ Model number | Phase state, compression ratio | The number of sub-specimens | Aging time t, h | | | | | | | |
|---|---|---|---|---|---|---|---|---|---|---|
| | | | 69 | 112 | 192 | 311 | 503 | 813 | | 3400 |
| S20 | Damp and hot Measured | 5 | - | - | - | - | - | - | - | - |
| | High and low temperature impact Measured | 5 | 0.10 | 0.10 | - | - | - | - | - | - |
| | High and low temperature alternating cycle Measured | 5 | - | - | - | - | - | - | - | - |
| | Three aging conditions Measured average | 15 | 0.10 | 0.10 | - | - | - | - | - | - |
| | Three aging conditions Predicted according to the equation (1.5) | 15 | 0.09 | 0.09 | 0.09 | 0.09 | 0.09 | 0.09 | | 0.09 |

Table 34-2   218℃, Rh <13%, the shear bonding strength, MPa, the aging trend

| 218℃ | Phase state, | The number of | Aging time t, h |
|---|---|---|---|
| | | | |

| Model number | compression ratio | sub-specimens | 0.0⊖ | 0.0⊕ | 0.08 | 2 | 9 | 16 | 27 | 43 |
|---|---|---|---|---|---|---|---|---|---|---|
| S20 | Damp and hot Measured | 5 | - | - | - | - | - | - | - | - |
| | High and low temperature impact Measured | 5 | - | - | - | - | - | - | - | - |
| | High and low temperature alternating cycle Measured | 5 | - | - | - | 0.12 | 0.11 | 0.11 | 0.12 | 0.11 |
| | Three aging conditions Measured average | 15 | - | - | - | 0.12 | 0.11 | 0.11 | 0.12 | 0.11 |
| | Three aging conditions Predicted according to the equation (1.5) | 15 | 0.19 | 0.20 | 0.18 | 0.14 | 0.13 | 0.11 | 0.13 | 0.11 |

(Continue) Table 34-2  218℃, Rh <13%, the shear bonding strength, MPa, the aging trend

| 218℃ Model number | Phase state, compression ratio | The number of sub-specimens | Aging time t, h | | | | | | | |
|---|---|---|---|---|---|---|---|---|---|---|
| | | | 112 | 192 | 311 | 503 | 813 | | | 3400 |
| S20 | Damp and hot Measured | 5 | - | - | - | - | - | - | - | - |

| | | | | | | | | | | |
|---|---|---|---|---|---|---|---|---|---|---|
| High and low temperature impact Measured | 5 | - | - | - | - | - | - | - | - | |
| High and low temperature alternating cycle Measured | 5 | 0.10 | - | - | - | - | - | - | - | |
| Three aging conditions Measured average | 15 | 0.10 | - | - | - | - | - | - | - | |
| Three aging conditions Predicted according to the equation (1.5) | 15 | 0.10 | 0.10 | 0.10 | 0.10 | 0.10 | | | 0.10 | |

Table 34-3   195℃, Rh <15%, the shear bonding strength, MPa, the aging trend

| 195℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.08 | 4 | 12 | 24 | 48 | 72 |
| S20 | Damp and hot Measured | 5 | - | - | 0.17 | | 0.12 | 0.12 | 0.15 | 0.13 |
| | High and low temperature impact Measured | 5 | - | - | - | 0.11 | 0.16 | 0.12 | 0.15 | 0.12 |
| | High and low temperature alternating cycle Measured | 5 | - | - | - | 0.19 | 0.13 | 0.15 | 0.14 | 0.12 |
| | Three aging conditions | 15 | - | - | 0.17 | 0.15 | 0.14 | 0.13 | 0.15 | 0.12 |

| | | | | | | | | | |
|---|---|---|---|---|---|---|---|---|---|
| Measured average | | | | | | | | | |
| Three aging conditions Predicted according to the equation (1.5) | 15 | 0.19 | 0.20 | 0.19 | 0.17 | 0.14 | 0.14 | 0.15 | 0.12 |

(Continue) Table 34-3   195℃, Rh <15%, the shear bonding strength, MPa, the aging trend

| 195℃ Model number | Phase state, compression ratio | The number of sub-specimens | Aging time t, h | | | | | | | |
|---|---|---|---|---|---|---|---|---|---|---|
| | | | 120 | 192 | 312 | 505 | 817 | 1322 | | 3400 |
| S20 | Damp and hot Measured | 5 | 0.12 | 0.1 | - | - | - | - | - | - |
| | High and low temperature impact Measured | 5 | 0.11 | 0.12 | - | - | - | - | - | - |
| | High and low temperature alternating cycle Measured | 5 | 0.1 | 0.11 | - | - | - | - | - | - |
| | Three aging conditions Measured average | 15 | 0.11 | 0.11 | - | - | - | - | - | - |
| | Three aging conditions Predicted according to the equation (1.5) | 15 | 0.11 | 0.11 | 0.11 | 0.11 | 0.11 | 0.11 | | 0.11 |

Table 34-4    150℃, Rh <30%, the shear bonding strength, MPa, the aging trend

| 150℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.13 | 6.0 | 24.0 | 48 | 72 | 120 |
| S20 | Damp and hot Measured | 5 | - | - | 0.16 | | 0.21 | 0.20 | 0.20 | 0.17 |
| | High and low temperature impact Measured | 5 | - | - | - | 0.18 | 0.25 | 0.22 | 0.21 | 0.20 |
| | High and low temperature alternating cycle Measured | 5 | - | - | - | 0.24 | 0.22 | 0.23 | 0.21 | 0.17 |
| | Three aging conditions Measured average | 15 | - | - | 0.16 | 0.21 | 0.22 | 0.22 | 0.21 | 0.18 |
| | Three aging conditions Predicted according to the equation (1.5) | 15 | 0.19 | 0.20 | 0.18 | 0.17 | 0.14 | 0.18 | 0.20 | 0.17 |

(Continue) Table 34-4    150℃, Rh <30%, the shear bonding strength, MPa, the aging trend

| 150℃ Model number | Phase state, compression ratio | The number of sub-specimens | Aging time t, h | | | | | | |
|---|---|---|---|---|---|---|---|---|---|
| | | | 192 | 312 | 504 | 810 | 1300 | 2100 | 3400 |

| | Phase state | Number | | | | | | | |
|---|---|---|---|---|---|---|---|---|---|
| S20 | Damp and hot Measured | 5 | 0.16 | 0.14 | - | - | - | - | - |
| | High and low temperature impact Measured | 5 | 0.19 | 0.17 | 0.15 | 0.13 | 0.12 | - | - |
| | High and low temperature alternating cycle Measured | 5 | 0.17 | 0.16 | 0.15 | - | - | - | - |
| | Three aging conditions Measured average | 15 | 0.17 | 0.16 | 0.15 | 0.13 | 0.12 | - | - |
| | Three aging conditions Predicted according to the equation (1.5) | 15 | 0.17 | 0.16 | 0.15 | 0.14 | 0.12 | 0.11 | 0.11 |

Table 34-5    97℃, Rh 97%, the shear bonding strength, MPa, the aging trend

| 97℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.22 | 10 | 48 | 72 | 120 | 192 |
| S20 | Damp and hot Measured | 5 | - | - | 0.19 | | 0.16 | 0.16 | 0.23 | 0.19 |
| | High and low | 5 | - | - | - | 0.20 | 0.20 | 0.21 | 0.21 | 0.19 |

| | | | | | | | | |
|---|---|---|---|---|---|---|---|---|
| temperature impact Measured | | | | | | | | |
| High and low temperature alternating cycle Measured | 5 | - | - | - | 0.17 | 0.18 | 0.20 | 0.17 | 0.21 |
| Three aging conditions Measured average | 15 | - | - | 0.19 | 0.18 | 0.18 | 0.19 | 0.20 | 0.20 |
| Three aging conditions Predicted according to the equation (1.5) | 15 | 0.19 | 0.20 | 0.20 | 0.17 | 0.15 | 0.16 | 0.20 | 0.19 |

(Continue) Table 34-5   97℃, Rh 97%, the shear bonding strength, MPa, the aging trend

| 97℃ Model number | Phase state, compression ratio | The number of sub-specimens | Aging time t, h | | | | | | | |
|---|---|---|---|---|---|---|---|---|---|---|
| | | | 312 | 504 | 810 | 1300 | 2100 | 2500 | | 3400 |
| S20 | Damp and hot Measured | 5 | 0.17 | 0.18 | - | - | - | - | - | - |
| | High and low temperature impact Measured | 5 | 0.21 | 0.19 | - | - | - | - | - | - |
| | High and low temperature alternating cycle Measured | 5 | 0.20 | 0.19 | - | - | - | - | - | - |
| | Three aging | 15 | 0.19 | 0.19 | - | - | - | - | - | - |

| | conditions Measured average | | | | | | | | | |
|---|---|---|---|---|---|---|---|---|---|---|
| | Three aging conditions Predicted according to the equation (1.5) | 15 | 0.19 | 0.19 | 0.19 | 0.19 | 0.19 | 0.19 | | 0.19 |

Table 34-6　85℃, Rh 85%, the shear bonding strength, MPa, the aging trend

| 85℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.22 | 48 | 72 | 120 | 192 | 312 |
| S20 | Damp and hot Measured | 5 | - | - | 0.15 | 0.16 | 0.21 | 0.21 | 0.20 | 0.19 |
| | High and low temperature impact Measured | 5 | - | - | - | - | - | - | - | - |
| | High and low temperature alternating cycle Measured | 5 | - | - | - | - | - | - | - | - |
| | Three aging conditions Measured average | 15 | - | - | 0.15 | 0.16 | 0.21 | 0.21 | 0.20 | 0.19 |
| | Three aging conditions Predicted according to the equation (1.5) | 15 | 0.19 | 0.20 | 0.20 | 0.16 | 0.16 | 0.19 | 0.20 | 0.19 |

(Continue) Table 34-6　85℃, Rh 85%, the shear bonding strength, MPa, the aging trend

| 85℃ Model number | Phase state, compression ratio | The number of sub-specimens | Aging time t, h | | | | | | | |
|---|---|---|---|---|---|---|---|---|---|---|
| | | | 504 | 810 | 1300 | 2100 | | | | 3400 |
| S20 | Damp and hot Measured | 5 | 0.18 | 0.18 | 0.19 | 0.18 | - | - | - | - |
| | High and low temperature impact Measured | 5 | - | - | - | - | - | - | - | - |
| | High and low temperature alternating cycle Measured | 5 | - | - | - | - | - | - | - | - |
| | Three aging conditions Measured average | 15 | 0.18 | 0.18 | 0.19 | 0.18 | - | - | | - |
| | Three aging conditions Predicted according to the equation (1.5) | 15 | 0.19 | 0.19 | 0.19 | 0.19 | 0.19 | 0.19 | | 0.19 |

5.4.1 T inspection

The T inspection indicates that the shear bonding strength under the three aging conditions of damp and hot, high and low temperature impact, and high and low temperature alternating cycle shows similar aging change trends, and this indicates that the difference in the influence of the two factors, that is, high and low temperature impact, and high and low temperature alternating cycle, on the aging shear bonding strength of S20 is negligible.

Therefore, when processing the aging data of S20 that has been in service for a long time under actual working conditions, the three data specimen groups obtained under the three aging conditions of damp and hot, high and low temperature impact, and high and low temperature alternating cycle should be combined into a larger data specimen, and then data processing is carried out.

5.4.2 Parameter fitting in equation (1.5)

In another use of the calculation method of the micro-gasification expansion oscillation equation (1) of the present invention, a general symbol (P) of the physical, chemical and electrical properties in the equation (1) is replaced with a specific symbol (S) of the shear bonding strength, so as to convert the equation (1) into an equation (1.5):

$$S_t = S_\infty + \left\{ S_{0\ominus} + \left[ \Delta S_1 e^{-k_1 t} \times \mathrm{Sin}\beta_1 \left( \frac{t}{t_0} - 1 \right)^{\theta_1} \pi + \Delta S_2 e^{-k_2 t} \times \mathrm{Sin}\beta_2 \left( \frac{t}{t_{0\lambda}} - 1 \right)^{\theta_2} \pi + \right.\right.$$

$$\left.\left. \Delta S_3 e^{-k_3 t} \right] - S_\infty \right\} e^{-kt} \quad \ldots (1.5)$$

in the equation (1.5)

S-the shear bonding strength of the target specimen, and the measured values are listed in Table 34-1 to Table 34-6 for fitting and verification;

$S_t$-the shear bonding strength of the target specimen at any specified constant temperature for any service time, a predicted value;

$S_\infty$-the shear bonding strength after weathering for more than 100 years, determined by numerical simulation of a material formula, or a fitted value;

$S_{0\ominus}$-initial shear bonding strength before aging, a measured value;

$S_{0\oplus}$-the shear bonding strength during micro-gasification expansion; a fitted value;

Sin-micro-gasification oscillation trigonometric function;

$\Delta S_1$-micro-gasification internal influence parameter of the shear bonding strength, $\Delta\sigma_1 = (\sigma_{0\oplus} - \sigma_{0\ominus})$;

$\Delta S_2$-micro-gasification interface influence parameter of the shear bonding strength, a fitted value;

$\Delta S_3$-mechanical stress influence parameter of the shear bonding strength, a fitted value;

t-aging time or service time, determined by a specified time or an accumulative number of cycles;

$t_0$-migration lag time of low molecular substances, a fitted value;

$\beta_1$-migration oscillation frequency coefficient, a fitted value;

$\beta_2$-volatilization oscillation frequency coefficient, a fitted value;

$k_1$-migration rate parameter, a fitted value;

$k_2$-volatilization rate parameter, a fitted value;

$k_3$-relaxation rate parameter, a fitted value;

k-chemical reaction rate parameter, a fitted value;

$\theta_1$-migration oscillation frequency index, a fitted value; and

$\theta_2$-volatilization oscillation frequency index, a fitted value.

In the formula (1.5) in this embodiment, some of the fifteen parameters contained in the shear bonding strength are negligible, and thus are assigned as "0"; although all the parameters are difficult to be obtained directly through linear fitting, the average values of the measured values in Table 34-1 to Table 34-6 are taken as specimens, starting with the "0" assignment, the assignment is tentatively increased step by step with a step pitch as small as possible, the parameter is repeatedly and iteratively input into the equation (1.5) by using an electronic calculation program or a parallax method, after each parameter is iterated for more than 50 times, the standard deviation of a difference value between a calculated value ($S_t$) and a measured value (S) converges to the minimum, optimal values of the fifteen parameters "Q" of the thermal conductivity at each temperature are obtained, and the results of iterative optimization are listed in Table 35. Because of the mathematical frequency doubling effect, when there is more than one optimal value among the fitted values of the fifteen parameters, only the smaller group of fifteen "Q" values closest to "1 time" is selected as optimal parameters.

5.4.3 Constant fitting in equation (2.5)

In the fifth embodiment, for the fifteen parameters in the shear bonding strength equation (1.5) contained in Table 35, on mechanism, each parameter does not change with time, but only changes with temperature. Each parameter that changes with temperature further includes the constants expressed by the corresponding three symbols "A, B and C" in the equation (2), and during the fitting process, the constants, which are corresponding to the parameters in the shear bonding strength equation (1.5), in the equation (2) need to be replaced with corresponding symbols, so as to convert the equation (2) into an equation (2.5):

$$\ln Q = \frac{A}{T+C} + B \dots\dots\dots\dots\dots\dots\dots\dots\dots\dots\dots\dots\dots\dots\dots\dots\dots\dots（2.5）$$

in the equation (2.5),

Q-corresponding to one of the fifteen parameters in the equation (1.5) at any temperature;

A-empirical constant of each parameter associated with the reaction activation energy and the diffusion activation energy of multiple components, a fitted value, K;

B-empirical constant of each parameter associated with the chemical reaction rate and the diffusion rate of multiple components, a fitted value, dimensionless;

C-conformal constant of Fourier series transformation of each parameter associated with the activation energy of multiple components, a fitted value, K;

T-absolute temperature, specified constant temperature +273.15, K;

"Q" in the equation (2.5) is replaced with the fifteen parameters in Table 35, plotting is performed by respectively using the logarithms of the fifteen parameters as vertical coordinates and using 1/(T+C) as abscissas, repeated iteration is performed by using a least square method electronic calculation program or a parallax method, and different "C" values are input, until $R^2$ automatically output by the system is $\geq$ 0.990, it is considered that the line has been a straight line, and "A, B, C" and $R^2$ in one-to-one correspondence with the obtained fifteen parameters of the shear bonding strength are optimal values thereof, which are listed in Table 35, respectively.

5.5 Prediction of changes in the shear bonding strength of S20

As long as the constraint conditions under the actual working conditions are consistent with the accelerated aging test conditions, and only the temperatures are different, the equation (1.5) and the equation (2.5) are applied. When $R^2 \geq 0.999$, it is accurate to predict the aging trend of the shear bonding strength under actual working conditions.

Table 35 Parameter values of the shear bonding strength of S20 in the equation (1.5)

| Serial number | S20, parameters in the micro-gasification expansion oscillation equation (1) | Parameter values of various temperatures | | | | | |
|---|---|---|---|---|---|---|---|
| | | 245°C | 218°C | 195°C | 150°C | 97°C | 85°C |
| 1 | $S_{\infty 5}$shear bonding strength after weathering for more than 100 years, % | 0.090 | 0.097 | 0.105 | 0.122 | 0.150 | 0.160 |
| 2 | $t_0$ migration lag time of low molecular substances, h | 4.00 | 4.90 | 5.90 | 8.90 | 17.0 | 20.0 |
| 3 | $\Delta S_1$ micro-gasification internal influence constant, % | 0.010 | 0.010 | 0.010 | 0.010 | 0.010 | 0.010 |
| 4 | $\Delta S_2$ micro-gasification interface influence constant, % | -0.09 | -0.09 | -0.09 | -0.09 | -0.09 | -0.09 |
| 5 | $\Delta S_3$ mechanical stress influence constant, % | 0.08 | 0.08 | 0.08 | 0.08 | 0.08 | 0.08 |

| 6 | $S_{0\oplus}$ shear bonding strength in micro-gasification phase state, % | 0.20 | 0.20 | 0.20 | 0.20 | 0.20 | 0.20 |
|---|---|---|---|---|---|---|---|
| 7 | $S_{0\ominus}$ initial shear bonding strength before aging, % | 0.19 | 0.19 | 0.19 | 0.19 | 0.19 | 0.19 |
| 8 | $\beta_1$ migration oscillation frequency coefficient, dimensionless | 29.60 | 14.60 | 6.80 | 1.80 | 0.30 | 0.19 |
| 9 | $\beta_2$ volatilization oscillation frequency coefficient, dimensionless | 0.29 | 0.27 | 0.25 | 0.22 | 0.19 | 0.18 |
| 10 | $k_1$ migration rate constant, 1/h | 0.70 | 0.62 | 0.56 | 0.43 | 0.30 | 0.27 |
| 11 | $k_2$ volatilization rate constant, 1/h | 2.7E-02 | 2.5E-02 | 2.3E-02 | 2.0E-02 | 1.5E-02 | 1.4E-02 |
| 12 | $k_3$ relaxation rate constant, 1/h | 7.80 | 6.30 | 5.25 | 3.55 | 2.00 | 1.73 |
| 13 | $k$ chemical reaction rate constant, 1/h | 2.1E-01 | 5.2E-02 | 1.9E-02 | 1.4E-03 | 4.5E-05 | 1.9E-05 |
| 14 | $\theta_1$ migration oscillation frequency index, dimensionless | 1.0 | 1.0 | 1.0 | 1.0 | 1.0 | 1.0 |

| 15 | $\theta_2$ volatilization oscillation frequency index, dimensionless | 1.0 | 1.0 | 1.0 | 1.0 | 1.0 | 1.0 |
|----|----|----|----|----|----|----|----|

(Continue) Table 35   Parameter and constant values of the shear bonding strength of S20 in the equation (1.5)

| Serial number | P40, parameters in the micro-gasification expansion oscillation equation (1) | Constant values of various parameters | | | | |
|---------------|-------------------------------------------------------------------------------|----------------------------------------|---|---|---|---|
| | | General expression formula | A | B | C | $R^2$ |
| 1 | $S_\infty$ shear bonding strength after weathering for more than 100 years, % | $\ln S_\infty = \dfrac{A}{T+C} + B$ | 1210 | -4.216 | 150 | 0.9996 |
| 2 | $t_0$ migration lag time of low molecular substances, h | $\ln t_0 = \dfrac{A}{T+C} + B$ | 1867 | -2.215 | 0 | 0.9999 |
| 3 | $\Delta S_1$ micro-gasification internal influence constant, % | $\Delta S_1 = \Delta \sigma_{0\oplus} - \Delta \sigma_{0\ominus}$ | 0 | -4.605 | 0 | - |
| 4 | $\Delta S_2$ micro-gasification interface influence constant, % | $\ln (\Delta S_2) = \dfrac{A}{T+C} + B$ | 0.0 | -2.465 | 0 | - |
| 5 | $\Delta S_3$ mechanical stress influence constant, % | $\ln (\Delta S_3) = \dfrac{A}{T+C} + B$ | 0.0 | -2.526 | 0 | - |
| 6 | $S_{0\oplus}$ shear bonding strength in micro-gasification phase state, % | $\Delta S_{0\oplus} = A(1 - e^{\frac{-B}{T+C}})$ | 0.190 | 1266 | 0 | - |
| 7 | $S_{0\ominus}$ initial shear bonding strength before | $\ln S_{0\ominus} = \dfrac{A}{T+C} + B$ | 0.0 | -1.661 | 0 | - |

| | aging, % | | | | | |
|---|---|---|---|---|---|---|
| 8 | $\beta_1$ migration oscillation frequency coefficient, dimensionless | $\ln\beta_1 = \dfrac{A}{T+C} + B$ | -52131 | 41.51 | 850 | 0.9998 |
| 9 | $\beta_2$ volatilization oscillation frequency coefficient, dimensionless | $\ln\beta_2 = \dfrac{A}{T+C} + B$ | -8859 | 3.487 | 1350 | 0.9997 |
| 10 | $k_1$ migration rate constant, 1/h | $\ln k_1 = \dfrac{A}{T+C} + B$ | -1094 | 1.749 | 0 | 0.9999 |
| 11 | $k_2$ volatilization rate constant, 1/h | $\ln k_2 = \dfrac{A}{T+C} + B$ | -748.2 | -2.171 | 0 | 0.9997 |
| 12 | $k_3$ relaxation rate constant, 1/h | $\ln k_3 = \dfrac{A}{T+C} + B$ | -3180 | 6.808 | 150 | 0.9999 |
| 13 | k chemical reaction rate constant, 1/h | $\ln k = \dfrac{A}{T+C} + B$ | -23249 | 30.77 | 200 | 0.9998 |
| 14 | $\theta_1$ migration oscillation frequency index, dimensionless | $\ln\theta_1 = \dfrac{A}{T+C} + B$ | 0 | 0 | 0 | - |
| 15 | $\theta_2$ volatilization oscillation frequency index, dimensionless | $\ln\theta_2 = \dfrac{A}{T+C} + B$ | 0 | 0 | 0 | - |

Table 36　When the compression ratio is 30%, the long-term change trend of the shear bonding strength $S_t$, MPa of S20 predicted by using the equation (1.5)

| S20 | Aging time t, year | | | | | | | | | | |
|---|---|---|---|---|---|---|---|---|---|---|---|
| | 0.0⊖ | 0.0⊕ | 0.01 | 0.003 | 0.004 | 0.006 | 0.008 | 0.011 | 0.015 | 0.021 | 0.029 |
| 75°C | 0.19 | 0.195 | 0.19 | 0.19 | 0.17 | 0.16 | 0.16 | 0.17 | 0.18 | 0.19 | 0.19 |
| 50°C | 0.19 | 0.194 | 0.19 | 0.18 | 0.19 | 0.18 | 0.17 | 0.17 | 0.17 | 0.18 | 0.19 |
| 37°C | 0.19 | 0.193 | 0.19 | 0.18 | 0.18 | 0.19 | 0.18 | 0.17 | 0.17 | 0.18 | 0.19 |

(Continue) Table 36　When the compression ratio is 30%, the long-term change trend of the shear bonding strength $S_t$, MPa of S20 predicted by using the equation (1.5)

| S20 | Aging time t, year | | | | | | | | | | |
|---|---|---|---|---|---|---|---|---|---|---|---|
| | 0.04 | 0.06 | 0.08 | 0.107 | 0.148 | 0.20 | 0.28 | 0.39 | 10 | 20 | 50 |
| 75°C | 0.19 | 0.19 | 0.19 | 0.19 | 0.19 | 0.19 | 0.19 | 0.19 | 0.19 | 0.19 | 0.19 |
| 50°C | 0.19 | 0.19 | 0.19 | 0.19 | 0.19 | 0.19 | 0.19 | 0.19 | 0.19 | 0.19 | 0.19 |
| 37°C | 0.19 | 0.19 | 0.19 | 0.19 | 0.19 | 0.19 | 0.19 | 0.19 | 0.19 | 0.19 | 0.19 |

In the fifth embodiment, it is only necessary to substitute the one-to-one corresponding three constants "A, B and C" in Table 35 and any service temperature below 245°C into the "general expression formula" in Table 35, that is, the equation (2.5) or its shifted variant form, so as to respectively figure out new "Q" values of the one-to-one corresponding fifteen parameters, and then any service time and the new "Q" values of the fifteen parameters are substituted back into the equation (1.5), so as to predict the long-term change trend of the shear bonding strength of S20 at any temperature and any time, when the compression ratio is 30%.

1) The equation (1.5) is used for predicting the time-varying changes of the shear bonding strength under the compression ratio of 30% and at the service temperatures of 245°C, 218°C, 195°C, 150°C, 97°C and 85°C, which are listed in Table 34-1 to Table 34-6; and plotting is performed by using the shear bonding strength as vertical coordinates and the service times as abscissas, and trend curves corresponding to Table 34-1 to Table 34-6 are shown in Fig. 59 to Fig. 64.

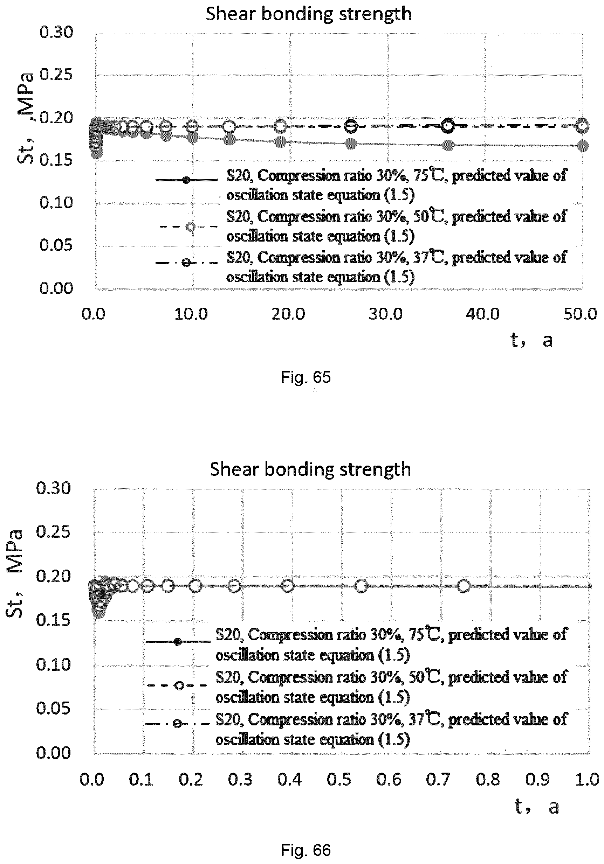

2) The equation (1.5) is used for predicting the change trends of the compression set rate with the service time under the compression ratio of 30% and at the service temperatures of 75°C, 50°C and 37°C, which are listed in Table 36; and plotting is performed by using the shear bonding strength as vertical coordinates and the service times as abscissas, and the trend curves corresponding to Table 36 are shown in Fig. 65 and Fig.66.

3) The standard deviation between the predicted result and the measured value of the shear bonding strength is within the range of ±1.96) Th sigma.

6. Sixth embodiment: evaluation or prediction of the service life of breakdown strength

The sixth embodiment discloses another form of the test method and algorithm for the aging life of the new energy heat management composite, and the use thereof in the present invention. The long-term change trend of the breakdown strength of an interface target specimen during long-term service under actual working conditions is evaluated or predicted by using a short-term accelerated aging test method, including: using fixture compression specimens with a compression ratio of 30%; selecting six constant temperatures for the group of fixture compression specimens within a temperature range of (85-245)°C, and making the group of fixture compression specimens respectively undergo three aging conditions of damp and hot, high and low temperature, and high and low temperature alternating circle in each specified constant temperature environment for a specified time or an accumulative number of cycles; using the fixture compression specimen combined by a square clamping plate shown in Fig. 11 and Fig. 12 to test the breakdown strength of a target specimen 4.2 according to test procedures specified in GB/T 1408.1 or GB/T 1695 or IEC 60243-1 or ASTM D 149; using measured values of the breakdown strength to fit corresponding fifteen parameters ($E_\infty$, $E_{0\ominus}$, $E_{0\oplus}$, $\Delta E_1$, $\Delta E_2$, $\Delta E_3$, $t_0$, $\beta_1$, $\beta_2$, $k_1$, $k_2$, $k_3$, $k$, $\theta_1$, $\theta_2$) in a micro-gasification expansion oscillation equation (1.6), and using each fitted parameter value to further fit three constants "A, B and C" contained in a dynamic correlation equation (2.6); substituting the three fitted constant values back into the dynamic correlation equation (2.6), so as to calculate new values of each parameter at the service temperatures of 75°C, 50°C and 37°C, respectively; and substituting the new values of this group of parameters back into the equation (1.6), so as to evaluate or predict the time-varying long-term change trend of the breakdown strength of the target specimen after a specified service time or an accumulative number of cycles under the conditions of damp and hot, high and low temperature, and high and low temperature alternating circle at 75°C, 50°C and 37°C. The details of the implementation steps will be further disclosed in the following five chapters 6.1 to 6.5-

6.1 Preparation of the fixture compression specimen

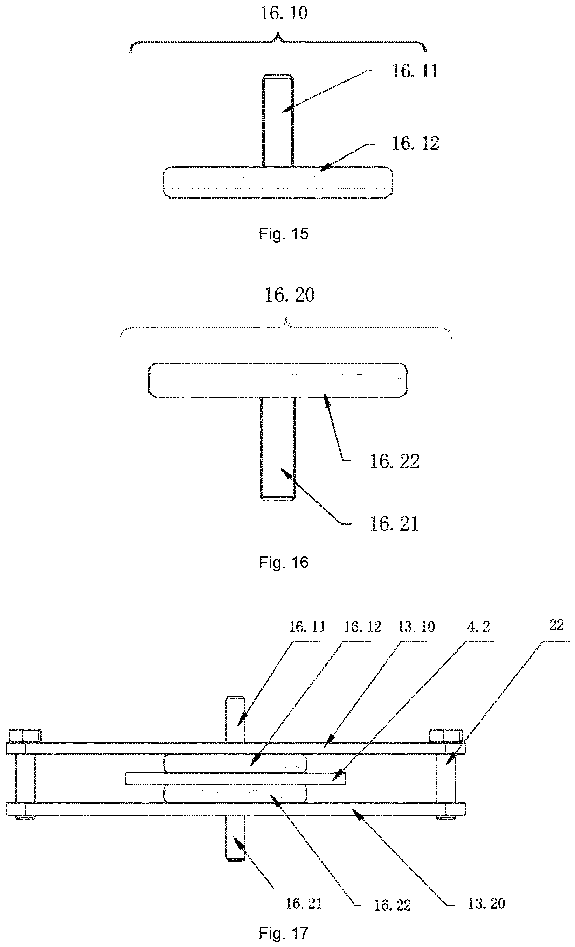

As shown in Fig. 15 and Fig. 16, according to the electrode requirements defined in GB/T 1408.1 or GB/T 1695 or IEC 60243-1 or ASTM D 149, an upper electrode 16.10 and a lower electrode 16.20 are fabricated, wherein the upper electrode 16.10 includes an upper electrode tip 16.11 and an upper electrode plate 16.12, the lower electrode 16.20 includes a lower electrode tip 16.21 and a lower electrode plate 16.22, and the electrode tips and the electrode plates are connected into an electrode entirely by threaded connection or soldering.

As shown in Fig. 17, the fixture compression specimen for the breakdown strength includes: a target specimen 4.2, screw rods 8, nuts 10, an upper square clamping plate 13.10, a lower square clamping plate 13.20, an upper electrode tip 16.11, an upper electrode plate 16.12, a lower electrode tip 16.21 and a lower electrode plate 16.22, wherein the electrode tips and the electrode plates are connected into an electrode entirely by threaded connection or soldering; the target specimen 4.2 is clamped by a pair of electrodes; and the pair of electrodes is fastened and locked by a pair of square clamping plates and insulating screw rods 8, so that the thickness of the target specimen 4.2 is compressed to 70% of the initial value, that is, the compression ratio is 30%, and a breakdown strength aging specimen is constituted.

The upper square clamping plate 13.10 is consistent with Fig. 11, and the lower square clamping plate 13.20 is consistent with Fig. 12.

The target specimen 4.2 is selected to be flaky shape formed by solidifying solidified two-component organic silicone heat conducting adhesive, with a thickness of (0.7-1.2)mm and a nominal thermal conductivity of 2 W/(m.K), referred to as S20 for short.

6.2 Three aging conditions

In this embodiment, the three aging conditions include: damp and hot, high and low temperature impact, and high and low temperature alternating cycle, and the specific details are completely the same as those disclosed in the second embodiment.

6.3 Test results

6.3.1 Damp and hot aging results

In the four temperature environments of 195°C, 150°C, 97°C and 85°C, the breakdown strength after damp and hot is listed in Table 37-3 to Table 37-6, respectively.

6.3.2 Aging results of high and low temperature impact

In the four temperature environments of 245°C, 195°C, 150°C and 97°C, the breakdown strength after high and low temperature impact is shown in Table 37-1, and Table 37-3 to Table 37-5, respectively.

6.3.3 Aging results of high and low temperature alternating cycle

In the four temperature environments of 218°C, 195°C, 150°C and 97°C, the breakdown strength after high and low temperature alternating cycle is shown in Table 37-2 to Table 37-5, respectively.

Table 37-1 245℃, Rh<10%, the breakdown strength, E, kV/mm, the aging trend

| 245℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.08 | 2 | 9 | 16 | 27 | 43 |
| S20 | Damp and hot Measured | 3 | 11.6 | 12.4 | - | - | - | - | - | - |
| | High and low temperature impact Measured | 3 | 11.6 | 12.4 | - | 11.8 | 10.2 | 10.5 | 10.0 | 9.5 |
| | High and low temperature alternating cycle Measured | 3 | 11.6 | 12.4 | - | - | - | - | - | - |
| | Three aging conditions Measured average | 9 | 11.6 | 12.4 | - | 11.8 | 10.2 | 10.5 | 10.0 | 9.5 |
| | Three aging conditions Predicted according to the equation (1.6) | 9 | 11.6 | 12.4 | 11.5 | 10.9 | 9.8 | 9.5 | 9.5 | 9.5 |

(Continue) Table 37-1 245℃, Rh<10%, the breakdown strength, E, kV/mm, the aging trend

| 245℃ Model number | Phase state, compression ratio | The number of sub-specimens | Aging time t, h | | | | | | | |
|---|---|---|---|---|---|---|---|---|---|---|
| | | | 69 | 112 | 192 | 311 | 503 | 813 | | 3400 |
| S20 | Damp and hot Measured | 3 | - | - | - | - | - | - | | - |
| | High and low temperature impact Measured | 3 | 9.3 | 9.0 | - | - | - | - | | - |
| | High and low temperature alternating cycle Measured | 3 | - | - | - | - | - | - | | - |
| | Three aging conditions Measured average | 9 | 9.3 | 9.0 | - | - | - | - | | - |
| | Three aging conditions Predicted according to the equation (1.6) | 9 | 9.5 | 9.5 | 9.5 | 9.5 | 9.5 | 9.5 | | 9.5 |

Table 37-2　218℃, Rh <13%, the breakdown strength, E, kV/mm, the aging trend

| 218℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.08 | 1.8 | 9 | 16 | 27 | 43 |
| S20 | Damp and hot Measured | 3 | 11.6 | 12.4 | - | - | - | - | - | - |
| | High and low temperature impact | 3 | 11.6 | 12.4 | - | - | - | - | - | - |

| Phase state, compression ratio | The number of sub-specimens | 69 | 112 | 192 | 311 | 503 | 813 | | 3400 |
|---|---|---|---|---|---|---|---|---|---|
| Measured | | | | | | | | | |
| High and low temperature alternating cycle Measured | 3 | 11.6 | 12.4 | - | 10.8 | 10.8 | 10.8 | 10.3 | 10.6 |
| Three aging conditions Measured average | 9 | 11.6 | 12.4 | - | 10.8 | 10.8 | 10.8 | 10.3 | 10.6 |
| Three aging conditions Predicted according to the equation (1.6) | 9 | 11.6 | 12.4 | 11.6 | 11.2 | 10.9 | 10.5 | 10.2 | 9.9 |

(Continue) Table 37-2　218℃, Rh <13%, the breakdown strength, E, kV/mm, the aging trend

| 218℃ Model number | Phase state, compression ratio | The number of sub-specimens | Aging time t, h | | | | | | | |
|---|---|---|---|---|---|---|---|---|---|---|
| | | | 69 | 112 | 192 | 311 | 503 | 813 | | 3400 |
| S20 | Damp and hot Measured | 3 | - | - | - | - | - | - | - | - |
| | High and low temperature impact Measured | 3 | - | - | - | - | - | - | - | - |
| | High and low temperature alternating cycle Measured | 3 | 9.5 | 9.3 | - | - | - | - | - | - |
| | Three aging conditions Measured average | 9 | 9.5 | 9.3 | - | - | - | - | - | - |

| | Three aging conditions Predicted according to the equation (1.6) | 9 | 9.8 | 9.7 | 9.7 | 9.7 | 9.7 | 9.7 | | 9.7 |
|---|---|---|---|---|---|---|---|---|---|---|

Table 37-3　195℃, Rh <15%, the breakdown strength, E, kV/mm, the aging trend

| 195℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.08 | 4 | 12 | 24 | 48 | 72 |
| S20 | Damp and hot Measured | 3 | 11.6 | 12.4 | 11.8 | - | 11.6 | 11.0 | 9.7 | 10.5 |
| | High and low temperature impact Measured | 3 | 11.6 | 12.4 | - | 11.8 | 11.8 | 10.8 | 11.6 | 10.5 |
| | High and low temperature alternating cycle Measured | 3 | 11.6 | 12.4 | - | 11.1 | 11.8 | 11.2 | 11.1 | 11.0 |
| | Three aging conditions Measured average | 9 | 11.6 | 12.4 | 11.8 | 11.5 | 11.7 | 11.0 | 10.8 | 10.7 |
| | Three aging conditions Predicted according to the equation (1.6) | 9 | 11.6 | 12.4 | 11.3 | 11.5 | 11.2 | 11.0 | 10.6 | 10.4 |

(Continue) Table 37-3　195℃, Rh <15%, the breakdown strength, E, kV/mm, the aging trend

| 195℃ | Phase state, | The number of | Aging time t, h |
|---|---|---|---|
| | | | |

| Model number | compression ratio | sub-specimens | 120 | 192 | 312 | 505 | 817 | 1322 | | 3400 |
|---|---|---|---|---|---|---|---|---|---|---|
| S20 | Damp and hot Measured | 3 | 9.8 | 9.1 | - | - | - | - | - | - |
| | High and low temperature impact Measured | 3 | 10.3 | 10.6 | - | - | - | - | - | - |
| | High and low temperature alternating cycle Measured | 3 | 10.5 | 9.5 | - | - | - | - | - | - |
| | Three aging conditions Measured average | 9 | 10.2 | 9.7 | - | - | - | - | - | - |
| | Three aging conditions Predicted according to the equation (1.6) | 9 | 10.1 | 10.0 | 9.9 | 9.9 | 9.9 | 9.9 | | 9.9 |

Table 37-4 150℃, Rh <30%, the breakdown strength, E, kV/mm, the aging trend

| 150℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.13 | 6 | 24 | 48 | 72 | 120 |
| S20 | Damp and hot Measured | 3 | 11.6 | 12.4 | 11.9 | - | 11.4 | 11.1 | 12.2 | 12.0 |
| | High and low temperature impact Measured | 3 | 11.6 | 12.4 | - | 11.8 | 11.1 | 11.9 | 12.0 | 11.5 |
| | High and low temperature | 3 | 11.6 | 12.4 | - | 11.7 | 11.4 | 11.4 | 10.1 | 10.7 |

| | | | | | | | | | |
|---|---|---|---|---|---|---|---|---|---|
| alternating cycle Measured | | | | | | | | | |
| Three aging conditions Measured average | 9 | 11.6 | 12.4 | 11.9 | 11.8 | 11.3 | 11.5 | 11.4 | 11.4 |
| Three aging conditions Predicted according to the equation (1.6) | 9 | 11.6 | 12.4 | 11.5 | 11.6 | 11.5 | 11.5 | 11.5 | 11.4 |

(Continue) Table 37-4　150℃, Rh <30%, the breakdown strength, E, kV/mm, the aging trend

| 150℃ Model number | Phase state, compression ratio | The number of sub-specimens | Aging time t, h | | | | | | |
|---|---|---|---|---|---|---|---|---|---|
| | | | 192 | 312 | 504 | 810 | | | 3400 |
| S20 | Damp and hot Measured | 3 | 11.6 | 11.0 | - | - | - | - | - | - |
| | High and low temperature impact Measured | 3 | 11.8 | 11.2 | - | - | - | - | - | - |
| | High and low temperature alternating cycle Measured | 3 | 11.3 | 11.6 | 11.1 | - | - | - | - | - |
| | Three aging conditions Measured average | 9 | 11.6 | 11.3 | 11.1 | - | - | - | - | - |

| | Three aging conditions Predicted according to the equation (1.6) | 9 | 11.3 | 11.2 | 11.0 | 10.8 | 10.6 | 10.5 | | 10.4 |

Table 37-5    97℃, Rh 97%, the breakdown strength, E, kV/mm, the aging trend

| 97℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.22 | 10 | 48 | 72 | 120 | 192 |
| S20 | Damp and hot Measured | 3 | 11.6 | 12.4 | 11.1 | - | 11.9 | 11.3 | 12.0 | 11.5 |
| | High and low temperature impact Measured | 3 | 11.6 | 12.4 | - | 12.1 | 10.4 | 11.0 | 11.4 | 11.8 |
| | High and low temperature alternating cycle Measured | 3 | 11.6 | 12.4 | - | 10.7 | 11.5 | 11.2 | 11.0 | 11.5 |
| | Three aging conditions Measured average | 9 | 11.6 | 12.4 | 11.1 | 11.4 | 11.3 | 11.2 | 11.5 | 11.6 |

EP 4 050 329 A1

| | Three aging conditions Predicted according to the equation (1.6) | 9 | 11.6 | 12.4 | 12.2 | 11.6 | 11.6 | 11.6 | 11.6 | 11.6 |

(Continue) Table 37-5  97℃, Rh 97%, the breakdown strength, E, kV/mm, the aging trend

| 97℃ Model number | Phase state, compression ratio | The number of sub-specimens | Aging time t, h | | | | | | |
|---|---|---|---|---|---|---|---|---|---|
| | | | 312 | 504 | 810 | 1300 | 2100 | 2500 | 3400 |
| S20 | Damp and hot Measured | 3 | 11.8 | 11.1 | - | - | - | - | - | - |
| | High and low temperature impact Measured | 3 | 11.5 | 11.7 | - | - | - | - | - | - |
| | High and low temperature alternating cycle Measured | 3 | 11.8 | 11.3 | - | - | - | - | - | - |
| | Three aging conditions Measured average | 9 | 11.7 | 11.4 | - | - | - | - | - | - |
| | Three aging conditions Predicted according to the equation (1.6) | 9 | 11.6 | 11.6 | 11.6 | 11.6 | 11.6 | 11.6 | | 11.6 |

142

Table 37-6   85℃, Rh 85%, the breakdown strength, E, kV/mm, the aging trend

| 85℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.22 | 48 | 72 | 120 | 192 | 312 |
| S20 | Damp and hot Measured | 3 | 11.6 | 12.4 | 11.0 | 11.4 | 11.6 | 11.4 | 11.7 | 11.3 |
| | High and low temperature impact Measured | 3 | 11.6 | 12.4 | - | - | - | - | - | - |
| | High and low temperature alternating cycle Measured | 3 | 11.6 | 12.4 | - | - | - | - | - | - |
| | Three aging conditions Measured average | 9 | 11.6 | 12.4 | 11.0 | 11.4 | 11.6 | 11.4 | 11.7 | 11.3 |
| | Three aging conditions Predicted according to the equation (1.6) | 9 | 11.6 | 12.4 | 12.0 | 11.6 | 11.6 | 11.6 | 11.6 | 11.6 |

(Continue) Table 37-6  85℃, Rh 85%, the breakdown strength, E, kV/mm, the aging trend

| 85℃ Model number | Phase state, compression ratio | The number of sub-specimens | Aging time t, h | | | | | | | |
|---|---|---|---|---|---|---|---|---|---|---|
| | | | 504 | 810 | 1300 | 2100 | 2500 | 3400 | | 5500 |

| | | | | | | | | | | | |
|---|---|---|---|---|---|---|---|---|---|---|---|
| S20 | Damp and hot Measured | 3 | 11.7 | 11.4 | 11.7 | 11.3 | - | - | - | - | |
| | High and low temperature impact Measured | 3 | - | - | - | - | - | - | - | - | |
| | High and low temperature alternating cycle Measured | 3 | - | - | - | - | - | - | - | - | |
| | Three aging conditions Measured average | 9 | 11.7 | 11.4 | 11.7 | 11.3 | - | - | - | - | |
| | Three aging conditions Predicted according to the equation (1.6) | 9 | 11.6 | 11.6 | 11.6 | 11.6 | 11.6 | 11.6 | | 11.6 | |

6.4 Establishment of a breakdown strength equation (1.6)

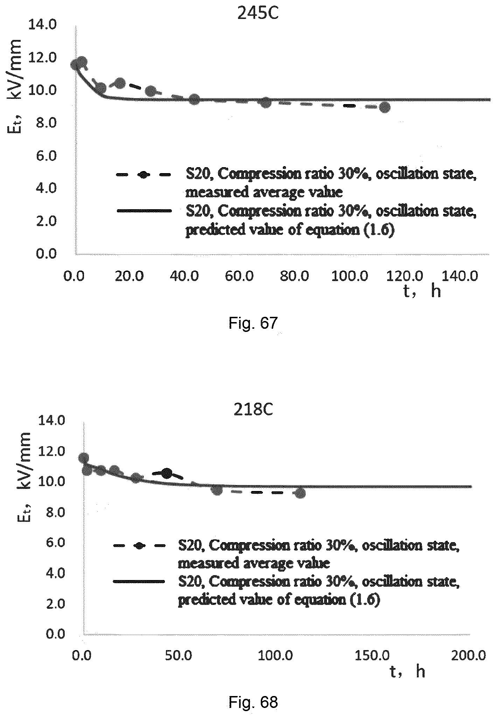

After undergoing the three aging conditions of damp and hot, high and low temperature impact, and high and low temperature alternating cycle in the six temperature environments of 245°C, 218°C, 195°C, 150°C, 97°C and 85°C, the aging trends of the breakdown strength corresponding to Table 37-1 to Table 37-6 are shown in Fig. 67 to Fig. 72.

6.4.1 T inspection

The T inspection indicates that the breakdown strength under the three aging conditions of damp and hot, high and low temperature impact, and high and low temperature alternating cycle shows similar aging change trends, and this indicates that the difference in the influence of the two factors, that is, high and low temperature impact, and high and low temperature alternating cycle, on the aging breakdown strength of S20 is negligible.

Therefore, when processing the aging data of S20 that has been in service for a long time under actual working conditions, the three data specimen groups obtained under the three aging conditions of damp and hot, high and low temperature impact, and high and low temperature alternating cycle should be combined into a larger data specimen, and then data processing is carried out.

6.4.2 Parameter fitting in equation (1.6)

In another use of the calculation method of the micro-gasification expansion oscillation equation (1) of the present invention, a general symbol (P) of the physical, chemical and electrical properties in the equation (1) is replaced with a specific symbol (E) of the breakdown strength, so as to convert the equation (1) into an equation (1.6):

$$E_t = E_\infty + \left\{ E_{0\ominus} + \left[ \Delta E_1 e^{-k_1 t} \times Sin\beta_1 \left( \frac{t}{t_0} - 1 \right)^{\theta_1} \pi + \Delta E_2 e^{-k_2 t} \times Sin\beta_2 \left( \frac{t}{t_{0\lambda}} - 1 \right)^{\theta_2} \pi + \Delta E_3 e^{-k_3 t} \right] - E_\infty \right\} e^{-kt} \quad \dots (1.6)$$

in the equation (1.6)

E-the breakdown strength of the target specimen, and the measured values are listed in Table 37-1 to Table 37-6 for fitting and verification;

$E_t$-the breakdown strength of the target specimen at any specified constant temperature for any service time, a predicted value;

$E_\infty$-the breakdown strength after weathering for more than 100 years, determined by numerical simulation of a material formula, or a fitted value;

$E_{0\ominus}$-initial breakdown strength before aging, a measured value;

$E_{0\oplus}$-the breakdown strength during micro-gasification expansion; a fitted value;

Sin-micro-gasification oscillation trigonometric function;

$\Delta E_1$-micro-gasification internal influence parameter of the breakdown strength, $\Delta E_1 = (E_{0\oplus} - E_{0\ominus})$;

$\Delta E_2$-micro-gasification interface influence parameter of the breakdown strength, a fitted value;

$\Delta E_3$-mechanical stress influence parameter of the breakdown strength, a fitted value;

t-aging time or service time, determined by a specified time or an accumulative number of cycles;

$t_0$-migration lag time of low molecular substances, a fitted value;

$\beta_1$-migration oscillation frequency coefficient, a fitted value;

$\beta_2$-volatilization oscillation frequency coefficient, a fitted value;

$k_1$-migration rate parameter, a fitted value;

$k_2$-volatilization rate parameter, a fitted value;

$k_3$-relaxation rate parameter, a fitted value;

k-chemical reaction rate parameter, a fitted value;

$\theta_1$-migration oscillation frequency index, a fitted value; and

$\theta_2$-volatilization oscillation frequency index, a fitted value.

In the formula (1.6) in this embodiment, some of the fifteen parameters contained in the breakdown strength are negligible, and thus are assigned as "0"; although all the parameters are difficult to be obtained directly through linear fitting, the average values of the measured values in Table 37-1 to Table 37-6 are taken as specimens, starting with the "0" assignment, the assignment is tentatively increased step by step with a step pitch as small as possible, the parameter is repeatedly and iteratively input into the equation (1.6) by using an electronic calculation program or a parallax method, after each parameter is iterated for more than 50 times, the standard deviation of a difference value between a calculated value ($E_t$) and a measured value (E) converges to the minimum, optimal values of the fifteen parameters "Q" of the thermal conductivity at each temperature are obtained, and the results of iterative optimization are listed in Table 38. Because of the mathematical frequency doubling effect, when there is more than one optimal value among the fitted values of the fifteen parameters, only the smaller group of fifteen "Q" values closest to "1 time" is selected as optimal parameters.

6.4.3 Constant fitting in equation (2.6)

In the fifth embodiment, for the fifteen parameters in the breakdown strength equation (1.6) contained in Table 38, on mechanism, each parameter does not change with time, but only changes with temperature. Each parameter that changes with temperature further includes the constants expressed by the corresponding three symbols "A, B and C" in the equation (2), and during the fitting process, the constants, which are corresponding to the parameters in the breakdown strength equation (1.6), in the equation (2) need to be replaced with corresponding symbols, so as to convert the equation (2) into an equation (2.6):

$$\ln Q = \frac{A}{T+C} + B \dots\dots\dots\dots\dots\dots\dots\dots (2.6)$$

in the equation (2.6),

Q-corresponding to one of the fifteen parameters in the equation (1.6) at any temperature;
A-empirical constant of each parameter associated with the reaction activation energy and the diffusion activation energy of multiple components, a fitted value, K;
B-empirical constant of each parameter associated with the chemical reaction rate and the diffusion rate of multiple components, a fitted value, dimensionless;
C-conformal constant of Fourier series transformation of each parameter associated with the activation energy of multiple components, a fitted value, K;
T-absolute temperature, specified constant temperature +273.15, K.

Table 38   Parameter values of the breakdown strength of S20 in the equation (1.6)

| Serial number | S20, parameters in the micro-gasification expansion oscillation equation (1) | Parameter values of various temperatures | | | | | |
| --- | --- | --- | --- | --- | --- | --- | --- |
| | | 245℃ | 218℃ | 195℃ | 150℃ | 97℃ | 85℃ |
| 1 | E∞ breakdown strength after weathering for more than 100 years, % | 9.470 | 9.720 | 9.940 | 10.500 | 11.400 | 11.650 |

| 2 | $t_0$ migration lag time of low molecular substances, h | 4.00 | 4.90 | 5.90 | 8.90 | 17.0 | 20.0 |
|---|---|---|---|---|---|---|---|
| 3 | $\Delta E_1$ micro-gasification internal influence constant, % | 0.800 | 0.800 | 0.800 | 0.800 | 0.800 | 0.800 |
| 4 | $\Delta E_2$ micro-gasification interface influence constant, % | -0.09 | -0.09 | -0.09 | -0.09 | -0.09 | -0.09 |
| 5 | $\Delta E_3$ mechanical stress influence constant, % | 0.08 | 0.08 | 0.08 | 0.08 | 0.08 | 0.08 |
| 6 | $E_{0\oplus}$ breakdown strength in micro-gasification phase state, % | 12.40 | 12.40 | 12.40 | 12.40 | 12.40 | 12.40 |
| 7 | $E_{0\ominus}$ initial breakdown strength before aging, % | 11.60 | 11.60 | 11.60 | 11.60 | 11.60 | 11.60 |
| 8 | $\beta_1$ migration oscillation frequency coefficient, dimensionless | 29.60 | 14.60 | 7.20 | 2.00 | 0.30 | 0.19 |
| 9 | $\beta_2$ volatilization oscillation frequency coefficient, dimensionless | 0.29 | 0.27 | 0.25 | 0.22 | 0.19 | 0.18 |
| 10 | $k_1$ migration rate constant, 1/h | 0.70 | 0.62 | 0.56 | 0.43 | 0.30 | 0.27 |
| 11 | $k_2$ volatilization rate constant, 1/h | 2.7E-02 | 2.5E-02 | 2.3E-02 | 2.0E-02 | 1.5E-02 | 1.4E-02 |
| 12 | $k_3$ relaxation rate constant, 1/h | 7.80 | 6.30 | 5.25 | 3.55 | 2.00 | 1.73 |
| 13 | k chemical reaction rate | 2.1E-01 | 5.2E-02 | 1.9E-02 | 1.6E-03 | 4.5E-05 | 1.9E-05 |

| | | | | | | | |
|---|---|---|---|---|---|---|---|
| | constant, 1/h | | | | | | |
| 14 | $\theta_1$ migration oscillation frequency index, dimensionless | 1.0 | 1.0 | 1.0 | 1.0 | 1.0 | 1.0 |
| 15 | $\theta_2$ volatilization oscillation frequency index, dimensionless | 1.0 | 1.0 | 1.0 | 1.0 | 1.0 | 1.0 |

(Continue) Table 38 Parameter and constant values of the breakdown strength of S20 in the equation (1.6)

| Serial number | P40, parameters in the micro-gasification expansion oscillation equation (1) | Constant values of various parameters | | | | |
|---|---|---|---|---|---|---|
| | | General expression formula | A | B | C | $R^2$ |
| 1 | $E_\infty$ breakdown strength after weathering for more than 100 years, % | $\ln E_\infty = \dfrac{A}{T+C} + B$ | 240.3 | 1.784 | 0 | 0.9999 |
| 2 | $t_0$ migration lag time of low molecular substances, h | $\ln t_0 = \dfrac{A}{T+C} + B$ | 1867 | -2.215 | 0 | 0.9999 |
| 3 | $\Delta E_1$ micro-gasification internal influence constant, % | $\Delta E_1 = \Delta\sigma_{0\oplus} - \Delta\sigma_{0\ominus}$ | 0.0 | -0.223 | 0 | - |
| 4 | $\Delta E_2$ micro-gasification interface influence constant, % | $\ln(\Delta E_2) = \dfrac{A}{T+C} + B$ | 0.0 | -2.47 | 0 | - |
| 5 | $\Delta E_3$ mechanical stress influence constant, % | $\ln(\Delta E_3) = \dfrac{A}{T+C} + B$ | 0.0 | -2.53 | 0 | - |
| 6 | $E_{0\oplus}$ breakdown strength in micro-gasification | $\Delta E_{0\oplus} = A(1 - e^{\frac{-B}{T+C}})$ | 11.60 | 1149 | 0 | - |

| | | | | | | |
|---|---|---|---|---|---|---|
| | phase state, % | | | | | |
| 7 | $E_{0\ominus}$ initial breakdown strength before aging, % | $\ln E_{0\ominus} = \dfrac{A}{T+C} + B$ | 0.0 | 2.451 | 0 | - |
| 8 | $\beta_1$ migration oscillation frequency coefficient, dimensionless | $\ln\beta_1 = \dfrac{A}{T+C} + B$ | -52172 | 41.57 | 850 | 0.9993 |
| 9 | $\beta_2$ volatilization oscillation frequency coefficient, dimensionless | $\ln\beta_2 = \dfrac{A}{T+C} + B$ | -8859 | 3.49 | 1350 | 0.9997 |
| 10 | $k_1$ migration rate constant, 1/h | $\ln k_1 = \dfrac{A}{T+C} + B$ | -1094 | 1.749 | 0 | 0.9999 |
| 11 | $k_2$ volatilization rate constant, 1/h | $\ln k_2 = \dfrac{A}{T+C} + B$ | -758.6 | -2.15 | 0 | 0.9999 |
| 12 | $k_3$ relaxation rate constant, 1/h | $\ln k_3 = \dfrac{A}{T+C} + B$ | -3180 | 6.808 | 150 | 0.9999 |
| 13 | k chemical reaction rate constant, 1/h | $\ln k = \dfrac{A}{T+C} + B$ | -23231 | 30.757 | 200 | 0.9998 |
| 14 | $\theta_1$ migration oscillation frequency index, dimensionless | $\ln\theta_1 = \dfrac{A}{T+C} + B$ | 0.0 | 0.0 | 0 | - |
| 15 | $\theta_2$ volatilization oscillation frequency index, dimensionless | $\ln\theta_2 = \dfrac{A}{T+C} + B$ | 0.0 | 0.0 | 0 | - |

"Q" in the equation (2.6) is replaced with the fifteen parameters in Table 38, plotting is performed by respectively using the logarithms of the fifteen parameters as vertical coordinates and using $1/(T+C)$ as abscissas, repeated iteration is performed by using a least square method electronic calculation program or a parallax method, and different "C" values are input, until $R^2$ automatically output by the system is $\geq 0.990$, it is considered that the line has been a straight line, and "A, B, C" and $R^2$ in one-to-one correspondence with the obtained fifteen parameters of the breakdown strength are optimal values thereof, which are listed in Table 38, respectively.

6.5 Prediction of changes in the breakdown strength of S20

As long as the constraint conditions under the actual working conditions are consistent with the accelerated aging test conditions, and only the temperatures are different, the equation (1.6) and the equation (2.6) are applied. When $R^2 \geq 0.999$, it is accurate to predict the aging trend of the breakdown strength under actual working conditions.

In this embodiment, it is only necessary to substitute the one-to-one corresponding three constants "A, B and C" in Table 38 and any service temperature below 245°C back into the "general expression formula" in Table 38, that is, the equation (2.6) or its shifted variant form, so as to respectively figure out new "Q" values of the one-to-one corresponding fifteen parameters, and then any service time and the new "Q" values of the fifteen parameters are substituted back into the equation (1.6), so as to predict the long-term change trend of the breakdown strength of S20 at any temperature and any time, when the compression ratio is 30%.

1) The equation (1.6) is used for predicting the time-varying changes of the breakdown strength under the compression ratio of 30% and at the service temperatures of 245°C, 218°C, 195°C, 150°C, 97°C and 85°C, which are listed in Table 37-1 to Table 37-6; and plotting is performed by using the breakdown strength as vertical coordinates and the service times as abscissas, and trend curves corresponding to Table 37-1 to Table 37-6 are shown in Fig. 67 to Fig. 72.

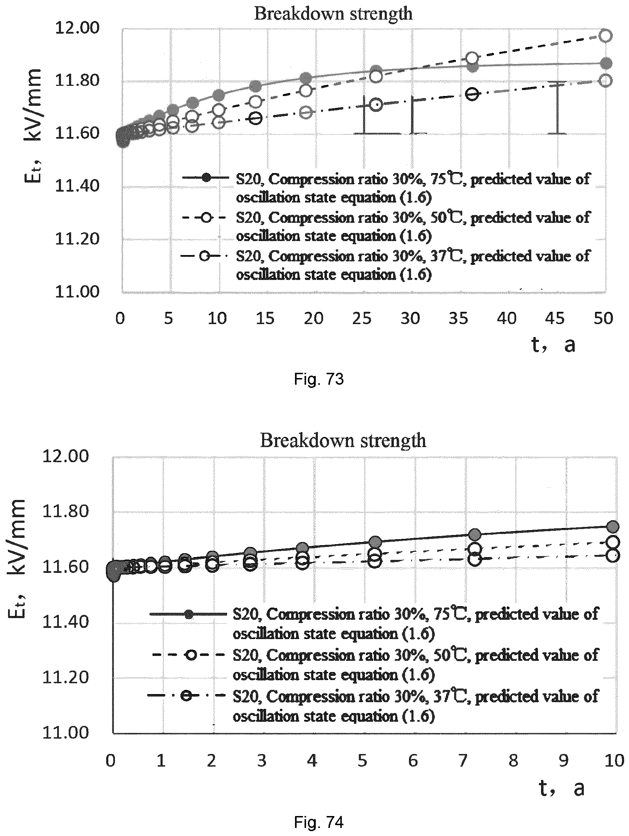

2) The equation (1.6) is used for predicting the change trends of the breakdown strength with the service time under the compression ratio of 30% and at the service temperatures of 75°C, 50°C and 37°C, which are listed in Table 39; and plotting is performed by using the breakdown strength as vertical coordinates and the service times as abscissas, and the trend curves corresponding to Table 39 are shown in Fig. 73 and Fig.74.

3) The standard deviation between the predicted result and the measured value of the breakdown strength is within the range of $\pm 1.96$) Th sigma.

Table 39   When the compression ratio is 30%, the long-term change trend of the

breakdown strength $E_t$, kV/mm of S20 predicted by using the equation (1.6)

| S20 | Aging time t, year | | | | | | | | | | |
|---|---|---|---|---|---|---|---|---|---|---|---|
| | 0.0⊖ | 0.0⊕ | 0.1 | 0.1 | 0.2 | 0.3 | 0.4 | 0.5 | 0.7 | 1 | 1 |
| 75°C | 11.6 | 12.0 | 11.6 | 11.6 | 11.6 | 11.6 | 11.6 | 11.6 | 11.6 | 11.6 | 11.6 |
| 50°C | 11.6 | 11.9 | 11.6 | 11.6 | 11.6 | 11.6 | 11.6 | 11.6 | 11.6 | 11.6 | 11.6 |
| 37°C | 11.6 | 11.9 | 11.6 | 11.6 | 11.6 | 11.6 | 11.6 | 11.6 | 11.6 | 11.6 | 11.6 |

(Continue) Table 39 When the compression ratio is 30%, the long-term change trend of the breakdown strength $E_t$, kV/mm of S20 predicted by using the equation (1.6)

| S20 | Aging time t, year | | | | | | | | | | |
|---|---|---|---|---|---|---|---|---|---|---|---|
| | 2 | 3 | 4 | 5 | 7 | 10 | 14 | 36 | | | 50 |
| 75°C | 11.6 | 11.7 | 11.7 | 11.7 | 11.7 | 11.7 | 11.8 | 11.9 | - | - | 11.9 |
| 50°C | 11.6 | 11.6 | 11.6 | 11.6 | 11.7 | 11.7 | 11.7 | 11.9 | - | - | 12.0 |
| 37°C | 11.6 | 11.6 | 11.6 | 11.6 | 11.6 | 11.6 | 11.7 | 11.8 | - | - | 11.8 |

7. Seventh embodiment: evaluation or prediction of the service life of volume resistivity

The seventh embodiment discloses another form of the test method and algorithm for the aging life of the new energy heat management composite, and the use thereof in the present invention. The long-term change trend of the volume resistivity of an interface target specimen during long-term service under actual working conditions is evaluated or predicted by using a short-term accelerated aging test method, including: using fixture compression specimens with a compression ratio of 30%; selecting six constant temperatures for the group of fixture compression specimens within a temperature range of (85-245)°C, and making the group of fixture compression specimens respectively undergo three aging conditions of damp and hot, high and low temperature, and high and low temperature alternating circle in each specified constant temperature environment for a specified time or an accumulative number of cycles; using the fixture compression specimen combined by a square clamping plate shown in Fig. 11 and Fig. 12 to test the volume resistivity of a target specimen 4.2 according to test procedures specified in GB/T 1410 or IEC 60093-1 or ASTM D 257; using measured values of the volume resistivity to fit corresponding fifteen parameters ($\rho_{v\infty}$, $\rho_{v0\ominus}$, $\rho_{v0\oplus}$, $\Delta\rho_{v1}$, $\Delta\rho_{v2}$, $\Delta\rho_{v3}$, $t_0$, $\beta_1$, $\beta_2$, $k_1$, $k_2$, $k_3$, $k$, $\theta_1$, $\theta_2$) in a micro-gasification expansion oscillation equation (1.7), and using each fitted parameter value to further fit three constants "A, B and C" contained in a dynamic correlation equation (2.7); substituting the three fitted constant values back into the dynamic correlation equation (2.7), so as to calculate new values of each parameter at the service temperatures of 75°C, 50°C and 37°C, respectively; and substituting the new values of this group of parameters back into the equation (1.7), so as to evaluate or predict the time-varying long-term change trend of the volume resistivity of the target specimen after a specified service time or an accumulative number of cycles under the conditions of damp and hot, high and low temperature, and high and low temperature alternating circle at 75°C, 50°C and 37°C. The details of the implementation steps will be further disclosed in the following five chapters 7.1 to 7.5-

7.1 Preparation of the fixture compression specimen

As shown in Fig. 18, the fixture compression specimen for the volume resistivity includes: a target specimen 4.2, bolts 8, an upper square clamping plate 13.10, a lower square clamping plate 13.20, a high-temperature insulation sheet 17 resistant to 245 °C or higher (such as a PTFE insulation sheet), a protected electrode 18, a protection electrode 19, a non-protected electrode 20, and positioning screws (electrode tips) 21; an annular gap between the protected electrode 18 and the protection electrode 19 is filled with a high temperature insulation ring 23 resistant to 245 °C or higher (for example, being bonded by a PTFE insulation ring or an insulating pouring sealant), so as to form an isolated and insulated entirety; the protection electrode 19 is isolated from the upper square clamping plate 13.10 by using the high-temperature insulation sheet 17 resistant to 245 °C or higher, so that the protected electrode 18 is isolated and insulated from the protection electrode 19; a pair of positioning screws (electrode tips) 21 is vertically and fixedly or welded with the centers of the upper square clamping plate 13.10 and the lower square clamping plate 13.20, respectively, and are conducted respectively to form a pair of independent electrode tip entireties; the pair of electrode tip entireties clamps the target specimen 4.2 up and down to form a "sandwich bread" structure; the upper square clamping plate 13.10 and the lower square clamping plate 13.20 are fastened and locked by metal screw rods 8, so that the thickness of the target specimen 4.2 is compressed to 70% of the initial value, that is, the compression ratio is 30%, and the fixture compression specimen for the volume resistivity is constituted; and when the aging process of the fixture compression specimen for the volume resistivity is completed, it is necessary to replace the metal screw rod 8 with an insulating bolt 8 and to ensure that there is no relative displacement between the electrode head entirety and the target specimen 4.2 before and after the aging process and during the test process of the volume resistivity.

The protected electrode 18, the protection electrode 19 and the non-protected electrode 20 are fabricated according to sizes specified in GB/T 1410 or IEC 60093-1 or ASTM D 257.

The upper square clamping plate 13.10 is consistent with Fig. 11, and the lower square clamping plate 13.20 is consistent with Fig. 12.

The target specimen 4.2 is selected to be flaky shape formed by solidifying solidified two-component organic silicone heat conducting adhesive, with a thickness of (0.7-1.0)mm and a nominal thermal conductivity of 2 W/(m.K), and the target specimen is referred to as S20 for short.

7.2 Three aging conditions

In this embodiment, the three aging conditions at six temperatures include: damp and hot, high and low temperature impact, and high and low temperature alternating cycle, and the specific details of the three aging conditions are completely the same as those disclosed in the second embodiment.

7.3 Test results

7.3.1 Damp and hot aging results

In the four temperature environments of 195°C, 150°C, 97°C and 85°C, the volume resistivity after damp and hot is listed in Table 40-3 to Table 40-6, respectively.

7.3.2 Aging results of high and low temperature impact

In the four temperature environments of 245°C, 195°C, 150°C and 97°C, the volume resistivity after high and low temperature impact is shown in Table 40-1, and Table 40-3 to Table 40-5, respectively.

7.3.3 Aging results of high and low temperature alternating cycle

In the four temperature environments of 218°C, 195°C, 150°C and 97°C, the volume resistivity after high and low temperature alternating cycle is shown in Table 40-2 to Table 40-5, respectively.

7.4 Establishment of a volume resistivity equation (1.7)

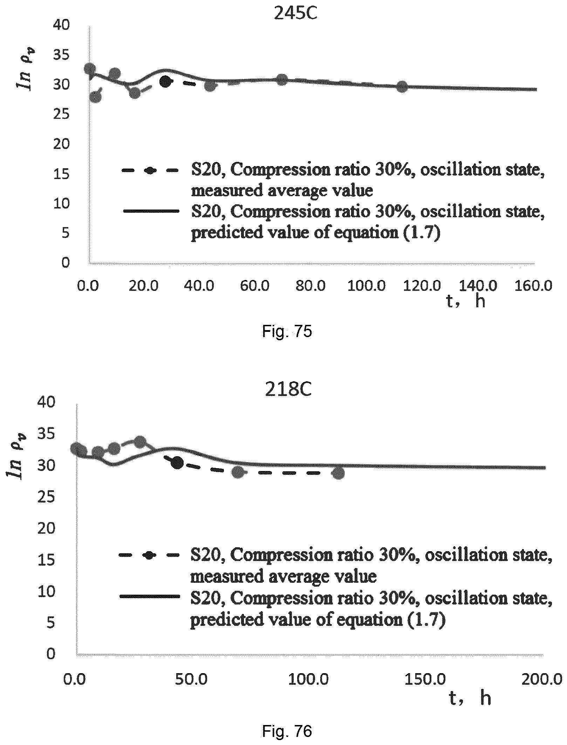

After undergoing the three aging conditions of damp and hot, high and low temperature impact, and high and low temperature alternating cycle in the six temperature environments of 245°C, 218°C, 195°C, 150°C, 97°C and 85°C, the aging trends of the volume resistivity corresponding to Table 40-1 to Table 40-6 are shown in Fig. 75 to Fig. 80.

Table 40-1　245℃, Rh<10%, the natural logarithm of the volume resistivity, the aging trend

| 245℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.08 | 2 | 9 | 16 | 27 | 43 |
| S20 | Damp and hot Measured | 3 | - | - | - | - | - | - | - | - |
| | High and low temperature impact Measured | 3 | - | - | - | 28.1 | 32 | 28.8 | 30.7 | 30 |
| | High and low temperature alternating cycle Measured | 3 | - | - | - | - | - | - | - | - |
| | Three aging conditions Measured average | 9 | - | - | - | 28.1 | 32.0 | 28.8 | 30.7 | 30.0 |
| | Three aging conditions | 9 | 32.8 | 34.7 | 31.1 | 31.8 | 30.7 | 30.3 | 32.5 | 30.8 |

| | Predicted according to the equation (1.7) | | | | | | | | | |
|---|---|---|---|---|---|---|---|---|---|---|

(Continue) Table 40-1    245℃, Rh<10%, the natural logarithm of the volume resistivity, the aging trend

| 245℃ Model number | Phase state, compression ratio | The number of sub-specimens | Aging time t, h | | | | | | | |
|---|---|---|---|---|---|---|---|---|---|---|
| | | | 69 | 112 | 192 | 192 | 311 | 503 | | 3400 |
| S20 | Damp and hot Measured | 3 | - | - | - | - | - | - | - | - |
| | High and low temperature impact Measured | 3 | 31 | 29.8 | - | - | - | - | - | - |
| | High and low temperature alternating cycle Measured | 3 | - | - | - | - | - | - | - | - |
| | Three aging conditions Measured average | 9 | 31.0 | 29.8 | - | - | - | - | - | - |
| | Three aging conditions Predicted according to the equation (1.7) | 9 | 30.9 | 29.8 | 29.2 | 28.9 | 28.9 | 28.9 | | 28.9 |

Table 40-2    218℃, Rh <13%, the natural logarithm of the volume resistivity, the aging trend

| 218℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.08 | 2 | 9 | 16 | 27 | 43 |
| S20 | Damp and hot Measured | 3 | - | - | - | - | - | - | - | - |
| | High and low temperature impact Measured | 3 | - | - | - | - | - | - | - | - |
| | High and low temperature alternating cycle Measured | 3 | - | - | - | 32.4 | 32.2 | 32.8 | 33.9 | 30.6 |
| | Three aging conditions Measured average | 9 | - | - | - | 32.4 | 32.2 | 32.8 | 33.9 | 30.6 |
| | Three aging conditions Predicted according to the equation (1.7) | 9 | 32.8 | 34.3 | 32.8 | 31.7 | 31.3 | 30.3 | 31.7 | 32.8 |

(Continue) Table 40-2　218℃, Rh <13%, the natural logarithm of the volume resistivity, the aging trend

| 218℃ Model number | Phase state, compression ratio | The number of sub-specimens | Aging time t, h | | | | | | |
|---|---|---|---|---|---|---|---|---|---|
| | | | 69 | 112 | 192 | 311 | 503 | 813 | 3400 |
| S20 | Damp and hot Measured | 3 | - | - | - | - | - | - | - |

| | | | | | | | | | | |
|---|---|---|---|---|---|---|---|---|---|---|
| | High and low temperature impact Measured | 3 | - | - | - | - | - | - | | - |
| | High and low temperature alternating cycle Measured | 3 | 29.1 | 28.9 | - | - | - | - | | - |
| | Three aging conditions Measured average | 9 | 29.1 | 28.9 | - | - | - | - | | - |
| | Three aging conditions Predicted according to the equation (1.7) | 9 | 30.5 | 30.1 | 29.8 | 29.4 | 29.2 | 29.2 | | 29.2 |

Table 40-3  195℃, Rh <15%, the natural logarithm of the volume resistivity, the aging trend

| 195℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.08 | 4 | 12 | 24 | 48 | 72 |
| S20 | Damp and hot Measured | 3 | 32.8 | 34.0 | 33.2 | - | 33 | 32.4 | 33.2 | 30 |
| | High and low temperature impact Measured | 3 | 32.8 | 34.0 | - | 31 | 32.2 | 32.6 | 34.1 | 31.5 |

| | | | | | | | | | | |
|---|---|---|---|---|---|---|---|---|---|---|
| | High and low temperature alternating cycle Measured | 3 | 32.8 | 34.0 | - | 32.5 | 31.3 | 31.4 | 32.5 | 31.3 |
| | Three aging conditions Measured average | 9 | 32.8 | 34.0 | 33.2 | 31.8 | 32.2 | 32.1 | 33.3 | 30.9 |
| | Three aging conditions Predicted according to the equation (1.7) | 9 | 32.8 | 34.0 | 31.2 | 32.2 | 31.2 | 30.5 | 33.3 | 30.7 |

(Continue) Table 40-3    195℃, Rh <15%, the natural logarithm of the volume resistivity, the aging trend

| 195℃ Model number | Phase state, compression ratio | The number of sub-specimens | Aging time t, h | | | | | | |
|---|---|---|---|---|---|---|---|---|---|
| | | | 120 | 192 | 312 | 505 | 817 | 1322 | 3400 |
| S20 | Damp and hot Measured | 3 | 29.9 | 29.8 | 29.4 | - | - | - | - |
| | High and low temperature impact Measured | 3 | 31.9 | 30.8 | - | - | - | - | - |
| | High and low temperature alternating cycle Measured | 3 | 30.7 | 29.1 | - | - | - | - | - |

| | Three aging conditions Measured average | 9 | 30.8 | 29.9 | - | - | - | - | | - |
|---|---|---|---|---|---|---|---|---|---|---|
| | Three aging conditions Predicted according to the equation (1.7) | 9 | 31.0 | 30.2 | 29.9 | 29.6 | 29.5 | 29.5 | | 29.5 |

Table 40-4  150℃, Rh <30%, the natural logarithm of the volume resistivity, the aging trend

| 150℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.13 | 6 | 24 | 48 | 72 | 120 |
| S20 | Damp and hot Measured | 3 | - | - | 34 | - | 31.9 | 32.3 | 32.7 | 31.1 |
| | High and low temperature impact Measured | 3 | - | - | - | 32.9 | 32.1 | 33.7 | 34.6 | 32 |
| | High and low temperature alternating cycle Measured | 3 | - | - | - | 32.3 | 32.5 | 31.9 | 32.8 | 32.1 |
| | Three aging conditions Measured average | 9 | - | - | 34.0 | 32.6 | 32.2 | 32.6 | 33.4 | 31.7 |
| | Three aging | 9 | 32.8 | 33.5 | 31.4 | 32.3 | 30.8 | 31.7 | 33.6 | 30.8 |

| | conditions Predicted according to the equation (1.7) | | | | | | | | | |
|---|---|---|---|---|---|---|---|---|---|---|

(Continue) Table 40-4    150℃, Rh <30%, the natural logarithm of the volume resistivity, the aging trend

| 150℃ Model number | Phase state, compression ratio | The number of sub-specimens | Aging time t, h | | | | | | |
|---|---|---|---|---|---|---|---|---|---|
| | | | 192 | 312 | 504 | 810 | 1322 | 2500 | 3400 |
| S20 | Damp and hot Measured | 3 | 32 | 30 | 30 | 29.5 | - | - | - |
| | High and low temperature impact Measured | 3 | 33.2 | 32 | - | - | - | - | - |
| | High and low temperature alternating cycle Measured | 3 | 31.4 | 30.8 | 31.3 | - | - | - | - |
| | Three aging conditions Measured average | 9 | 32.2 | 30.9 | 30.7 | - | - | - | - |
| | Three aging conditions Predicted according to the equation (1.7) | 9 | 31.3 | 30.8 | 30.6 | 30.3 | 30.1 | 30.1 | 30.1 |

Table 40-5  97℃, Rh 97%, the natural logarithm of the volume resistivity, the aging trend

| 97℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.22 | 10 | 48 | 72 | 120 | 192 |
| S20 | Damp and hot Measured | 3 | - | - | 35 | - | 30.8 | 30.9 | 36.2 | 35.5 |
| | High and low temperature impact Measured | 3 | - | - | - | 32.9 | 33.1 | 32.4 | 33.7 | 33.3 |
| | High and low temperature alternating cycle Measured | 3 | - | - | - | 32.8 | 32.5 | 33 | 33.5 | 33 |
| | Three aging conditions Measured average | 9 | - | - | 35.0 | 32.9 | 32.1 | 32.1 | 34.5 | 33.9 |
| | Three aging conditions Predicted according to the equation (1.7) | 9 | 32.8 | 33.1 | 31.6 | 32.3 | 31.0 | 30.9 | 33.2 | 32.7 |

(Continue) Table 40-5  97℃, Rh 97%, the natural logarithm of the volume resistivity, the aging trend

| 97℃ Model number | Phase state, compression ratio | The number of sub-specimens | Aging time t, h | | | | | | |
|---|---|---|---|---|---|---|---|---|---|
| | | | 312 | 504 | 810 | 1322 | 2100 | 2500 | 3400 |
| S20 | Damp and hot | 3 | 33 | 33.8 | 32.6 | 31 | - | - | - |

| | | | Aging time t, h | | | | | | | |
|---|---|---|---|---|---|---|---|---|---|---|
| Measured | | | | | | | | | | |
| High and low temperature impact Measured | 3 | 34.1 | 32.9 | - | - | - | - | | - | |
| High and low temperature alternating cycle Measured | 3 | 32.4 | 33 | - | - | - | - | | - | |
| Three aging conditions Measured average | 9 | 33.2 | 33.2 | - | - | - | - | | - | |
| Three aging conditions Predicted according to the equation (1.7) | 9 | 32.9 | 32.5 | 31.5 | 31.3 | 31.1 | 31.1 | | 31.1 | |

Table 40-6   85℃, Rh 85%, the natural logarithm of the volume resistivity, the aging trend

| 85℃ Model number | Phase state, compression ratio | The number of sub-specimens | Aging time t, h | | | | | | | |
|---|---|---|---|---|---|---|---|---|---|---|
| | | | 0.0⊖ | 0.0⊕ | 0.22 | 48 | 72 | 120 | 192 | 312 |
| S20 | Damp and hot Measured | 3 | - | - | 33.8 | 33.7 | 32.6 | 32.9 | 32.9 | 32.4 |
| | High and low temperature impact | 3 | - | - | - | - | - | - | - | - |

| | | | | | | | | | |
|---|---|---|---|---|---|---|---|---|---|
| Measured | | | | | | | | | |
| High and low temperature alternating cycle Measured | 3 | - | - | - | - | - | - | - | - |
| Three aging conditions Measured average | 9 | - | - | 33.8 | 33.7 | 32.6 | 32.9 | 32.9 | 32.4 |
| Three aging conditions Predicted according to the equation (1.7) | 9 | 32.8 | 33.1 | 31.6 | 31.3 | 30.9 | 32.2 | 33.9 | 31.5 |

(Continue) Table 40-6　85℃, Rh 85%, the natural logarithm of the volume resistivity, the aging trend

| 85℃ Model number | Phase state, compression ratio | The number of sub-specimens | Aging time t, h | | | | | | |
|---|---|---|---|---|---|---|---|---|---|
| | | | 504 | 810 | 1300 | 2100 | 2500 | 3400 | 5500 |
| S20 | Damp and hot Measured | 3 | 31.6 | 31.6 | 30.5 | 30.6 | - | - | - |
| | High and low temperature impact Measured | 3 | - | - | - | - | - | - | - |
| | High and low temperature alternating cycle | 3 | - | - | - | - | - | - | - |

| | Measured | | | | | | | | |
|---|---|---|---|---|---|---|---|---|---|
| | Three aging conditions Measured average | 9 | 31.6 | 31.6 | 30.5 | 30.6 | - | - | - |
| | Three aging conditions Predicted according to the equation (1.7) | 9 | 31.5 | 32.3 | 31.7 | 31.5 | 31.4 | 31.3 | 31.2 |

### 7.4.1 T inspection

The T inspection indicates that the volume resistivity under the three aging conditions of damp and hot, high and low temperature impact, and high and low temperature alternating cycle shows similar aging change trends, and this indicates that the difference in the influence of the two factors, that is, high and low temperature impact, and high and low temperature alternating cycle, on the aging volume resistivity of S20 is negligible.

Therefore, when processing the aging data of S20 that has been in service for a long time under actual working conditions, the three data specimen groups obtained under the three aging conditions of damp and hot, high and low temperature impact, and high and low temperature alternating cycle should be combined into a larger data specimen, and then data processing is carried out.

### 7.4.2 Parameter fitting in equation (1.7)

In another use of the calculation method of the micro-gasification expansion oscillation equation (1) of the present invention, a general symbol (P) of the physical, chemical and electrical properties in the equation (1) is replaced with a specific symbol ($\rho_V$) of the natural logarithm of the volume resistivity, so as to convert the equation (1) into an equation (1.7):

$$\rho_{vt} = \rho_\infty + \left\{ \rho_{v0\ominus} + \left[ \Delta\rho_{v1}e^{-k_1t} \times \mathrm{Sin}\beta_1 \left( \frac{t}{t_0} - 1 \right)^{\theta_1} \pi + \Delta\rho_{v2}e^{-k_2t} \times \mathrm{Sin}\beta_2 \left( \frac{t}{t_{0\lambda}} - 1 \right)^{\theta_2} \pi + \right.$$

$$\left. \Delta\rho_{v3}e^{-k_3t} \right] - \rho_{V\infty} \right\} e^{-kt} \qquad (1.7)$$

in the equation (1.7)

$\rho_V$-the natural logarithm of the resistivity of the target specimen, and the measured values are listed in Table 40-1 to Table 40-6 for fitting and verification;

$\rho_{vt}$-the natural logarithm of the volume resistivity of the target specimen at any specified constant temperature for any service time, a predicted value;

$\rho_{V\infty}$-the natural logarithm of the volume resistivity after weathering for more than 100 years, determined by numerical simulation of a material formula, or a fitted value;

$\rho_{v0\ominus}$-the natural logarithm of initial volume resistivity before aging, a measured value;

$\rho_{v0\oplus}$-the natural logarithm of the volume resistivity during micro-gasification expansion; a fitted value;

Sin-micro-gasification oscillation trigonometric function;

$\Delta\rho_{v1}$-micro-gasification internal influence parameter of the natural logarithm of the volume resistivity, $\Delta\rho_{v1} = (\rho_{v0\oplus} - \rho_{v0\ominus})$;

$\Delta\rho_{v2}$-micro-gasification interface influence parameter of the natural logarithm of the volume resistivity, a fitted value;

$\Delta\rho_{v3}$-mechanical stress influence parameter of the natural logarithm of the volume resistivity, a fitted value;

t-aging time or service time, determined by a specified time or an accumulative number of cycles;

$t_0$-migration lag time of low molecular substances, a fitted value;

$\beta_1$-migration oscillation frequency coefficient, a fitted value;

$\beta_2$-volatilization oscillation frequency coefficient, a fitted value;

$k_1$-migration rate parameter, a fitted value;

$k_2$-volatilization rate parameter, a fitted value;

$k_3$-relaxation rate parameter, a fitted value;

k-chemical reaction rate parameter, a fitted value;

$\theta_1$-migration oscillation frequency index, a fitted value; and

$\theta_2$-volatilization oscillation frequency index, a fitted value.

Table 41 Parameter values of the natural logarithm of the volume resistivity of S20 in the equation (1.7)

| Serial number | S20, parameters in the micro-gasification expansion oscillation equation (1) | Parameter values of various temperatures | | | | | |
|---|---|---|---|---|---|---|---|
| | | 245°C | 218°C | 195°C | 150°C | 97°C | 85°C |
| 1 | $\rho_{v\infty}$ logarithm of the volume resistivity after weathering for more than 100 years, % | 28.90 | 29.20 | 29.50 | 30.10 | 31.00 | 31.25 |
| 2 | $t_0$ migration lag time of low molecular substances, h | 4.00 | 4.90 | 5.90 | 8.90 | 17.0 | 20.0 |
| 3 | $\Delta\rho_1$ micro-gasification internal influence constant, % | 0.00 | 0.00 | 0.00 | 0.00 | 0.00 | 0.00 |
| 4 | $\Delta\rho_2$ micro-gasification interface influence constant, % | -2.85 | -2.72 | -2.64 | -2.46 | -2.20 | -2.15 |
| 5 | $\Delta\rho_3$ mechanical stress influence constant, % | 0.00 | 0.00 | 0.00 | 0.00 | 0.00 | 0.00 |
| 6 | $\rho_{0\oplus}$ logarithm of the | 34.70 | 34.29 | 34.00 | 33.50 | 33.10 | 33.06 |

| | | | | | | | |
|---|---|---|---|---|---|---|---|
| | volume resistivity in micro-gasification phase state, % | | | | | | |
| 7 | $\rho_{0\ominus}$ logarithm of the initial volume resistivity before aging, % | 32.80 | 32.80 | 32.80 | 32.80 | 32.80 | 32.80 |
| 8 | $\beta_1$ migration oscillation frequency coefficient, dimensionless | 29.60 | 14.60 | 7.20 | 2.00 | 0.300 | 0.190 |
| 9 | $\beta_2$ volatilization oscillation frequency coefficient, dimensionless | 0.213 | 0.209 | 0.205 | 0.198 | 0.188 | 0.185 |
| 10 | $k_1$ migration rate constant, 1/h | 0.70 | 0.62 | 0.56 | 0.43 | 0.30 | 0.27 |
| 11 | $k_2$ volatilization rate constant, 1/h | 1.2E-02 | 8.5E-03 | 6.5E-03 | 3.7E-03 | 1.5E-03 | 1.2E-03 |
| 12 | $k_3$ relaxation rate constant, 1/h | 7.80 | 6.30 | 5.25 | 3.55 | 2.00 | 1.73 |
| 13 | k chemical reaction rate constant, 1/h | 1.4E-02 | 9.5E-03 | 6.8E-03 | 3.5E-03 | 1.2E-03 | 9.8E-04 |
| 14 | $\theta_1$ migration oscillation frequency index, dimensionless | 1.0 | 1.0 | 1.0 | 1.0 | 1.0 | 1.0 |
| 15 | $\theta_2$ volatilization oscillation frequency index, dimensionless | 1.0 | 1.0 | 1.0 | 1.0 | 1.0 | 1.0 |

(Continue) Table 41   Parameter and constant values of the natural logarithm of the volume resistivity of S20 in the equation (1.7)

| Serial number | P40, parameters in the micro-gasification expansion oscillation equation (1) | Constant values of various parameters | | | | |
|---|---|---|---|---|---|---|
| | | General expression formula | A | B | C | $R^2$ |
| 1 | $\rho_{V\infty}$ logarithm of the volume resistivity after weathering for more than 100 years, % | $\ln \rho_{V\infty} = \dfrac{A}{T+C} + B$ | 165.1 | 3.117 | 150 | 0.9999 |
| 2 | $t_0$ migration lag time of low molecular substances, h | $\ln t_0 = \dfrac{A}{T+C} + B$ | 1867 | -2.215 | 0 | 0.9999 |
| 3 | $\Delta\rho_1$ micro-gasification internal influence constant, % | $\Delta\rho_1 = \Delta\sigma_{0\oplus} - \Delta\sigma_{0\ominus}$ | 0.0 | -13.82 | 0 | - |
| 4 | $\Delta\rho_2$ micro-gasification interface influence constant, % | $\ln(\Delta\rho_2) = \dfrac{A}{T+C} + B$ | -592.7 | 1.93 | 150 | 0.9990 |
| 5 | $\Delta\rho_3$ mechanical stress influence constant, % | $\ln(\Delta\rho_3) = \dfrac{A}{T+C} + B$ | 0.0 | -13.82 | 0 | - |
| 6 | $\rho_{0\oplus}$ logarithm of the volume resistivity in micro-gasification phase state, % | $\rho_{0\oplus} = A(1 - e^{\frac{-B}{T+C}})$ | 32.80 | 1583 | -10 | 0.9992 |
| 7 | $\rho_{0\ominus}$ logarithm of the initial volume resistivity before aging, % | $\ln \rho_{0\ominus} = \dfrac{A}{T+C} + B$ | 0.0 | 3.490 | 0 | - |
| 8 | $\beta_1$ migration oscillation frequency coefficient, dimensionless | $\ln\beta_1 = \dfrac{A}{T+C} + B$ | -52172 | 41.57 | 850 | 0.9993 |
| 9 | $\beta_2$ volatilization oscillation | $\ln\beta_2 = \dfrac{A}{T+C} + B$ | -172 | -1.23 | 15 | 0.9991 |

| | | | | | | |
|---|---|---|---|---|---|---|
| | frequency coefficient, dimensionless | | | | | |
| 10 | $k_1$ migration rate constant, 1/h | $\ln k_1 = \dfrac{A}{T + C} + B$ | -1094 | 1.749 | 0 | 0.9999 |
| 11 | $k_2$ volatilization rate constant, 1/h | $\ln k_2 = \dfrac{A}{T + C} + B$ | -3583 | 1.64 | 70 | 0.9995 |
| 12 | $k_3$ relaxation rate constant, 1/h | $\ln k_3 = \dfrac{A}{T + C} + B$ | -3180 | 6.808 | 150 | 0.9999 |
| 13 | k chemical reaction rate constant, 1/h | $\ln k = \dfrac{A}{T + C} + B$ | -5651 | 4.173 | 150 | 0.9994 |
| 14 | $\theta_1$ migration oscillation frequency index, dimensionless | $\ln \theta_1 = \dfrac{A}{T + C} + B$ | 0.0 | 0.0 | 0 | - |
| 15 | $\theta_2$ volatilization oscillation frequency index, dimensionless | $\ln \theta_2 = \dfrac{A}{T + C} + B$ | 0.0 | 0.0 | 0 | - |

In the formula (1.7) in this embodiment, some of the fifteen parameters contained in the natural logarithm of the volume resistivity are negligible, and thus are assigned as "0"; although all the parameters are difficult to be obtained directly through linear fitting, the average values of the measured values in Table 40-1 to Table 40-6 are taken as specimens, starting with the "0" assignment, the assignment is tentatively increased step by step with a step pitch as small as possible, the parameter is repeatedly and iteratively input into the equation (1.7) by using an electronic calculation program or a parallax method, after each parameter is iterated for more than 50 times, the standard deviation of a difference value between a calculated value ($\rho_{vt}$) and a measured value ($\rho_v$) converges to the minimum, optimal values of the fifteen parameters "Q" of the thermal conductivity at each temperature are obtained, and the results of iterative optimization are listed in Table 41. Because of the mathematical frequency doubling effect, when there is more than one optimal value among the fitted values of the fifteen parameters, only the smaller group of fifteen "Q" values closest to "1 time" is selected as optimal parameters.

7.4.3 Constant fitting in equation (2.7)

In the seventh embodiment, for the fifteen parameters in the equation (1.7) of the natural logarithm of the volume resistivity contained in Table 41, on mechanism, each parameter does not change with time, but only changes with temperature. Each parameter that changes with temperature further includes the constants expressed by the corresponding three symbols "A, B and C" in the equation (2), and during the fitting process, the constants, which are corresponding to the parameters in the equation (1.7) of the natural logarithm of the volume resistivity, in the equation (2) need to be replaced with corresponding symbols, so as to convert the equation (2) into an equation (2.7):

$$\ln Q = \frac{A}{T+C} + B \dots\dots\dots\dots\dots\dots\dots\dots\dots\dots\dots\dots\dots\dots\dots\dots\dots\dots\dots\dots\dots\dots (2.7)$$

in the equation (2.7),

Q-corresponding to one of the fifteen parameters in the equation (1.7) at any temperature;

A-empirical constant of each parameter associated with the reaction activation energy and the diffusion activation energy of multiple components, a fitted value, K;

B-empirical constant of each parameter associated with the chemical reaction rate and the diffusion rate of multiple components, a fitted value, dimensionless;

C-conformal constant of Fourier series transformation of each parameter associated with the activation energy of multiple components, a fitted value, K;

T-absolute temperature, specified constant temperature +273.15, K;

"Q" in the equation (2.7) is replaced with the fifteen parameters in Table 41, plotting is performed by respectively using the logarithms of the fifteen parameters as vertical coordinates and using 1/(T+C) as abscissas, repeated iteration is performed by using a least square method electronic calculation program or a parallax method, and different "C" values are input, until $R^2$ automatically output by the system is $\geq 0.990$, it is considered that the line has been a straight line, and "A, B, C" and $R^2$ in one-to-one correspondence with the obtained fifteen parameters of the natural logarithm of the volume resistivity are optimal values thereof, which are listed in Table 41, respectively.

7.5 Prediction of changes in the volume resistivity of S20

As long as the constraint conditions under the actual working conditions are consistent with the accelerated aging test conditions, and only the temperatures are different, the equation (1.7) and the equation (2.7) are applied. When $R^2 \geq 0.999$, it is accurate to predict the aging trend of the natural logarithm of the volume resistivity under actual working conditions.

In this embodiment, it is only necessary to substitute the one-to-one corresponding three constants "A, B and C" in Table 41 and any service temperature below 245°C back into the "general expression formula" in Table 41, that is, the equation (2.7) or its shifted variant form, so as to respectively figure out new "Q" values of the one-to-one corresponding fifteen parameters, and then any service time and the new "Q" values of the fifteen parameters are substituted back into the equation (1.7), so as to predict the long-term change trend of the natural logarithm of the volume resistivity of S20 at any temperature and any time, when the compression ratio is 30%.

1) The equation (1.7) is used for predicting the time-varying changes of the natural logarithm of the volume resistivity under the compression ratio of 30% and at the service temperatures of 245°C, 218°C, 195°C, 150°C, 97°C and 85°C, which are listed in Table 40-1 to Table 40-6; and plotting is performed by using the natural logarithm of the volume resistivity as vertical coordinates and the service times as abscissas, and trend curves corresponding to Table 40-1 to Table 40-6 are shown in Fig. 75 to Fig. 80.

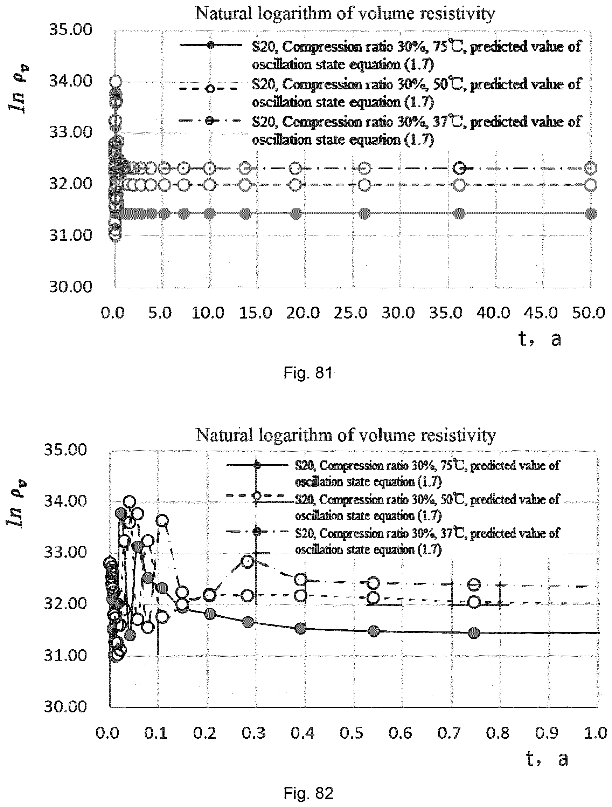

2) The equation (1.7) is used for predicting the change trends of the compression set rate with the service time under the compression ratio of 30% and at the service temperatures of 75°C, 50°C and 37°C, which are listed in Table 42; and plotting is performed by using the natural logarithm of the volume resistivity as vertical coordinates and the service times as abscissas, and the trend curves corresponding to Table 42 are shown in Fig. 81 and Fig.82.

3) The standard deviation between the predicted result and the measured value of the natural logarithm of the volume resistivity is within the range of $\pm 1.96$) Th sigma.

Table 42   When the compression ratio is 30%, the long-term change trend of the natural logarithm of the volume resistivity of S20 predicted by using the equation (1.7)

| S20 | Aging time t, year | | | | | | | | | | |
|---|---|---|---|---|---|---|---|---|---|---|---|
| | 0.0⊖ | 0.0⊕ | 0.11 | 0.15 | 0.20 | 0.28 | 0.39 | 0.54 | 0.75 | 1.03 | 1.42 |
| 75°C | 32.8 | 33.1 | 32.3 | 31.9 | 31.8 | 31.6 | 31.5 | 31.4 | 31.4 | 31.4 | 31.4 |
| 50°C | 32.8 | 33.0 | 31.7 | 32.0 | 32.1 | 32.1 | 32.1 | 32.1 | 32.0 | 32.0 | 32.0 |
| 37°C | 32.8 | 33.0 | 33.6 | 32.2 | 32.2 | 32.8 | 32.4 | 32.4 | 32.3 | 32.3 | 32.3 |

(Continue) Table 42 When the compression ratio is 30%, the long-term change trend of the natural logarithm of the volume resistivity of S20 predicted by using the equation (1.7)

| S20 | Aging time t, year | | | | | | | | | | |
|---|---|---|---|---|---|---|---|---|---|---|---|
| | 2.0 | 2.7 | 3.8 | 5.2 | 7.2 | 9.9 | 13.7 | 36 | 50 | 2.0 | 2.7 |
| 75°C | 31.4 | 31.4 | 31.4 | 31.4 | 31.4 | 31.4 | 31.4 | 31.4 | 31.4 | 31.4 | 31.4 |
| 50°C | 32.0 | 32.0 | 32.0 | 32.0 | 32.0 | 32.0 | 32.0 | 32.0 | 32.0 | 32.0 | 32.0 |
| 37°C | 32.3 | 32.3 | 32.3 | 32.3 | 32.3 | 32.3 | 32.3 | 32.3 | 32.3 | 32.3 | 32.3 |

8. Eighth embodiment: evaluation or prediction of half-life period and rated temperature

Another form of the test method and algorithm for the aging life of the new energy heat management composite, and the use thereof in the present invention is to evaluate or predict rated indicators of an interface target specimen corresponding to physical, chemical and electrical properties during long-term service under actual working conditions, by using a short-term accelerated aging test method, including: determining a half-life period of any one of the physical, chemical and electrical properties in a specified service temperature environment, or determining a rate temperature of any one of the physical, chemical and electrical properties at a specified service time of 20,000 hours, by using a micro-gasification expansion oscillation equation (1) and a dynamic correlation equation (2).

In the eighth embodiment, a method for determining a half-life period and a rated temperature of a target specimen 4.2 under the thermal conductivity P40 is disclosed, including: using a micro-gasification expansion oscillation equation (1.1), a temperature correlation equation (2.1) and 15 groups of three "A, B, C" constant values in Table 11; substituting the 15 groups of three "A, B, C" constant values and different service temperatures ($T_i$) back into the dynamic correlation equation (2.1), so as to respectively calculate new values of the 15 parameters ($\lambda_\infty$, $t_0$, $\Delta\lambda_1$, $\Delta\lambda_2$, $\Delta\lambda_3$, $\Delta\lambda_{0\oplus}$, $\lambda_{0\ominus}$, $\beta_1$, $\beta_2$, $k_1$, $k_2$, $k_3$, $k$, $\theta_1$, $\theta_2$) in the environment of the service temperature ($T_i$) by using a software program or an electronic calculation form; and substituting the new values of this group of 15 parameters back into the micro-gasification expansion oscillation equation (1.1) one by one, continuing to use the software program or the electronic calculation form to repeatedly and tentatively input the time required for dropping the thermal conductivity by half in the environment of the service temperature ($T_i$), that is, the half-life period ($\tau_i$) of the thermal conductivity, for example, the initial thermal conductivity of the target specimen P40 is 4.18 W/(m.K), when the input time is t, the thermal conductivity output by the software program or the electronic calculation form is 2.09 W/(m.K), the time value t is the half-life period ($\tau_i$) of the thermal conductivity in the environment of the service temperature ($T_i$), see Table 43.

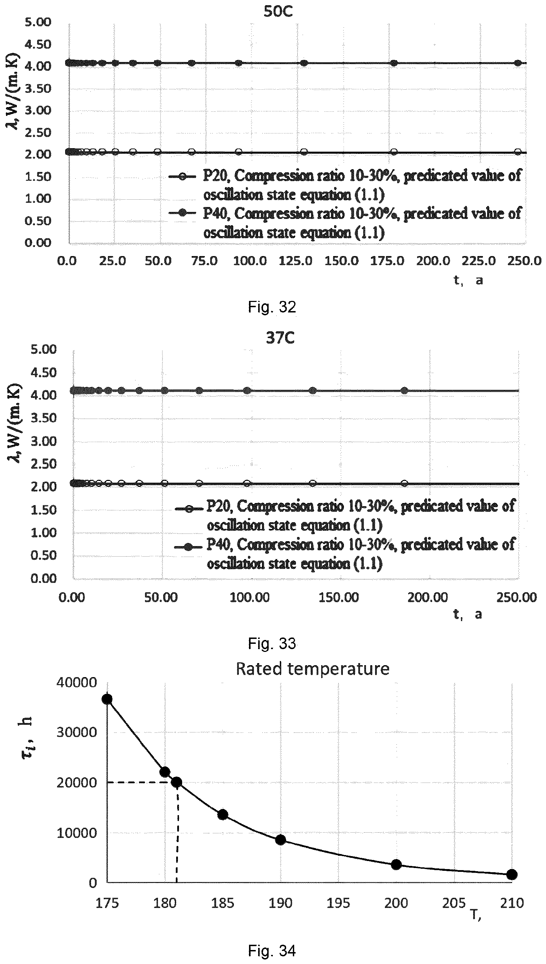

As shown in Fig. 34, plotting is performed by using the half-life periods ($\tau_i$) of the thermal conductivity in Table 43 as vertical coordinates and using the service temperatures ($T_i$) as abscissas, various points are connected into a smooth curve, the curve has an intersection with a 20,000-hour horizontal line of the vertical coordinates, an intersection of a vertical line and the abscissas is plotted by passing through the intersection, so as to obtain the rated temperature of the target specimen P40, and the rated temperature of the target specimen P40 under the thermal conductivity is 181°C.

Table 43 When the compression ratio is (10-30)%, the half-life period of the thermal conductivity of P40 determined by using the equation (1.1)

| Service temperature,$T_i$,°C | 210 | 200 | 190 | 185 | 180 | 175 |
|---|---|---|---|---|---|---|
| Half-life period of the $\Delta\lambda_1$ heat conductivity coefficient $\tau_i$, h | 1586 | 3565 | 8497 | 22675 | 22035 | 36550 |

By means of the above eight embodiments of the test method and algorithm for the aging life of the new energy heat management composite, and the use thereof in the present invention, it can be found that the beneficial technical effects are as follows:

(1) The highest aging test temperature can reach 298°C, which shortens the laboratory aging test time by 90% from over 1,000 hours;

(2) it is suitable for predicting the aging lives of materials with the coexistence of three-phase material state of solid, liquid and gas, which breaks through the limitation that the "width of an extended prediction temperature is less than 0.8 times of the difference between the highest test temperature and the lowest temperature" in the GB/T 20028, ASTM G 166, ASTM G 169, ISO 2578 and UL 746B standards;

(3) it is suitable for evaluating or predicting the long-term service lives of all polymer matrix composites; and

(4) the linear correlation coefficient $R^2$ is two "9" accuracy levels higher than GB/T 20028, ASTM G 166, ASTM G 169, ISO 2578, and UL 746B, so that the prediction is more accurate.

**Claims**

1.    A test method and algorithm for an aging life of a new energy heat management composite, comprising: preparing a target specimen into any one or a combined specimen of any two of an open specimen, a closed specimen and a fixture compression specimen, so as to serve as a standard specimen for an aging life test; respectively placing the standard specimens in at least four specified constant temperature environments, and making the standard specimens respectively undergo at least one condition of damp and hot, high and low temperature impact and high and low temperature alternating cycle for a specified time or an accumulative number of cycles in each temperature environment; testing the physical, chemical and electrical properties of the target specimen by using the standard specimens or laminated combined test pieces; fitting fifteen parameters in a micro-gasification expansion oscillation equation (1) by using measured values of the physical, chemical and electrical properties; fitting three constants in a kinetic correlation equation (2) of the fifteen parameters; substituting the fitted constants back into the kinetic correlation equation (2) one by one, so as to calculate new values of the fifteen parameters in any specified constant temperature environment; and substituting the new values of the fifteen parameters back into the equation (1), so as to evaluate or predict the physical, chemical and electrical properties of the target specimen at any specified time under the at least one condition of damp and hot, high and low temperature impact and high and low temperature alternating cycle for the specified time or the accumulative number of cycles.

2.    A use of the test method and algorithm for the aging life of the new energy heat management composite, comprising: by using the test method and algorithm for the aging life, evaluating or predicting the physical, chemical and electrical properties of the target specimen in any specified constant temperature environment for the specified time or the accumulative number of cycles; or evaluating or predicting a half-life period of any one of the physical, chemical and electrical properties in the specified constant temperature environment, or evaluating or predicting a rated temperature of any one of the physical, chemical and electrical properties at a specified service time of 20,000 hours; wherein the physical, chemical and electrical properties further comprise at least one of color, density, thermal conductivity, oil separation rate, compression set rate, specific heat, hardness, tensile strength, elongation at break, butt joint tension bonding strength, lap joint shear bonding strength, glass transition temperature, linear expansion coefficient, breakdown strength, DC or AC electric leakage resistance, volume resistivity, dielectric constant, loss factor, oxygen index, flame retardancy, vacuum volatiles, hydroscopicity, mold resistance, fumes density, fumes index, and toxicity index of burned gas.

3.  The test method and algorithm for the aging life according to claim 1, **characterized in that** the composite comprises: any one of solid, fluid and melt of a polymer matrix composite; or a mixture of any two states of solid, fluid and melt; or any one or a compound of rubber, plastic, fibers and thermosetting materials; or any one or a compound of elastomers, adhesives, sealants and foam materials.

4.  The test method and algorithm for the aging life according to claim 1, **characterized in that** the target specimen comprises: the composite is made into a specimen that conforms to a shape specified by corresponding test standards for physical, chemical and electrical properties.

5.  The test method and algorithm for the aging life according to claim 1, **characterized in that** the open specimen comprises: the target specimen is not coated, wrapped, clamped or closed by using materials, wraps or containers that are different from the chemical components of the target specimen, but the target specimen is exposed to an aging environment.

6.  The test method and algorithm for the aging life according to claim 1, **characterized in that** the closed specimen comprises: a part or all of the superficial area of the target specimen is isolated from the aging environment by using materials, wraps or containers that are different from the chemical components of the target specimen, in any manner of coating, wrapping, clamping or closing.

7.  The test method and algorithm for the aging life according to claim 1, **characterized in that** the fixture compression specimen comprises: the target specimen is clamped into a "sandwich biscuit" structure by using at least two rigid plates, and the distance between the two rigid plates is adjusted to a specified thickness or compression ratio or pressure by using fasteners; the shape of the edge contour line of the rigid plate comprises any one of a camber line, a straight line and a broken line, or the edge contour line is formed by connecting and enclosing any two of the camber line, the straight line and the broken line end to end; and the size of the rigid plate is correspondingly set according to the size of the target specimen required by the test requirements of the physical, chemical and electrical properties, and when the rigid plate is liable to generate warping deformation under stress, any one or a combined body of any two of "+, ×, =, ⋈, , ⊕, #"-shaped stiffeners are arranged on one surface of the rigid plate to resist the warping deform.

8.  The test method and algorithm for the aging life according to claim 1, **characterized in that** the combined specimen comprises: on the superficial area of the target specimen, a part of the superficial area is in the state of the open specimen, and the other part of the superficial area is in the state of the closed specimen; or the fixture compression specimen is made into the state of the closed specimen again.

9.  The test method and algorithm for the aging life and the use thereof according to claim 1 and claim 2, **characterized in that** the specified constant temperature comprises: within an allowable temperature measurement error range, a constant temperature required for the experiment is set at a temperature below 400°C at least in an oven or a drying room or a warehouse; or a temperature curve is taken as a vertical coordinate, the time is taken as an abscissa, and an average temperature of ratios of areas below the temperature curve to corresponding times is taken as the constant temperature.

10. The test method and algorithm for the aging life according to claim 1, **characterized in that** the damp and hot comprises: in the specified constant temperature environment, the moisture content of any one or a mixed medium of an air atmosphere, an oxidizing atmosphere, a reducing atmosphere and an inert gas atmosphere is controlled in the oven or the drying room or the warehouse, so as to control the relative humidity to (5-100)%.

11. The test method and algorithm for the aging life according to claim 1, **characterized in that** the high and low temperature impact comprises: after a specified time in a specified higher temperature environment, the target specimen is transitioned to a lower temperature environment for the specified time according to a specified cooling rate; or, after a specified time in a specified lower temperature environment, the target specimen is transitioned to a higher temperature environment for the specified time according to a specified heating rate.

12. The test method and algorithm for the aging life according to claim 1, **characterized in that** the high and low temperature alternating cycle comprises: according to a specified cooling rate and a heating rate, the target specimen is alternately transitioned for a specified time or an accumulative number of cycles between a higher specified constant temperature and a lower specified constant temperature environment; and the alternating transition is that the temperature curve is taken as the vertical coordinate, the time is taken as the abscissa, and the contour shape of the temperature curve comprises any one of a straight line, a broken line and a cambered line, or a cyclic reciprocating and high-low undulating wave state formed by connecting any two lines end to end.

**13.** The test method and algorithm for the aging life or the use thereof according to claim 1 or claim 2, **characterized in that** the specified time or the accumulative number of cycles comprises: the standard specimen is placed in the temperature-controlled oven or the drying room or the warehouse, is taken out from the oven or the drying room or the warehouse after a certain period of time or an accumulative number of times in accordance with established test procedures, and is placed in another specified constant temperature environment.

**14.** The test method and algorithm for the aging life according to claim 1, **characterized in that** the laminated combined test piece comprises: during a constant temperature process, or when the physical, chemical and electrical properties are tested, at least one layer of materials or parts with known performance indicators and known dimensions is attached to the upper surface and the lower surface of the rigid plate of the fixture compression specimen, so that the instrument can accurately measure the physical, chemical and electrical properties.

**15.** The test method and algorithm for the aging life according to claim 1, **characterized in that** the measured value comprises: data of the physical, chemical and electrical properties measured by an instrument or equipment that meets the requirements of test standards for the physical, chemical and electrical properties, in accordance with actions and conditions specified by corresponding standards.

**16.** The test method and algorithm for the aging life according to claim 1, **characterized in that** the micro-gasification expansion oscillation formula (1) comprises:

$$P_t = P_\infty + \left\{ P_{0\ominus} + \left[ \Delta P_1 e^{-k_1 t} \times Sin\, \beta_1 \left( \frac{t}{t_0} - 1 \right)^{\theta_1} \pi + \Delta P_2 e^{-k_2 t} \times Sin\, \beta_2 \left( \frac{t}{t_0} - 1 \right)^{\theta_2} \pi + \right.\right.$$

$$\left.\left. \Delta P_3 e^{-k_3 t} \right] - P_\infty \right\} e^{-kt} \ldots\ldots (1)$$

in the equation (1),

P-any one of the physical, chemical and electrical properties, a measured value, used for parameter fitting or verification;

$P_t$-the physical, chemical and electrical properties at any specified constant temperature for any service time, an evaluated or predicted value;

$P_\infty$-the physical, chemical and electrical properties at the aging end point, determined by numerical simulation of a material formula, or a fitted value;

$P_{0\ominus}$-initial physical, chemical and electrical properties before aging, a measured value;

$P_{0\oplus}$-the physical, chemical and electrical properties at the beginning of micro-gasification expansion, a fitted value;

Sin-micro-gasification oscillation trigonometric function;

$\Delta P_1$-micro-gasification internal influence parameter, $\Delta P_1 = (P_{0\oplus} - P_{0\ominus})$;

$\Delta P_2$-micro-gasification interface influence parameter, a fitted value;

$\Delta P_3$-mechanical stress influence parameter, a fitted value;

t-aging time or service time, determined by a specified time or an accumulative number of cycles;

$t_0$-migration lag time of low molecular substances, a fitted value;

$\beta_1$-migration oscillation frequency coefficient, a fitted value;

$\beta_2$-volatilization oscillation frequency coefficient, a fitted value;

$k_1$-migration rate parameter, a fitted value;

$k_2$-volatilization rate parameter, a fitted value;

$k_3$-relaxation rate parameter, a fitted value;

k-chemical reaction rate parameter, a fitted value;

$\theta_1$-migration oscillation frequency index, a fitted value;

$\theta_2$-volatilization oscillation frequency index, a fitted value;

wherein, the equation (1) covers all the physical, chemical and electrical properties, for the sake of brevity, it is expressed as a general expression containing fifteen parameters that do not change with time, and it is not just a relational expression expressing one property; and when any one of the physical, chemical and electrical properties is evaluated or predicted, the corresponding parameters and symbols of the physical, chemical and electrical properties in the equation (1) need to be replaced one by one.

17. The test method and algorithm for the aging life according to claim 1 or claim 16, **characterized in that** the parameters comprise: a total of fifteen parameters $P_\infty$, $P_{0\ominus}$, $P_{0\oplus}$, $\Delta P_1$, $\Delta P_2$, $\Delta P_3$, $t_0$, $\beta_1$, $\beta_2$, $k_1$, $k_2$, $k_3$, $k$, $\theta_1$, $\theta_2$ in the equation (1), fourteen of which are independent parameters, the other $\Delta P_1$ is a linear correlation parameter, and the parameters do not change with time but change with temperature; and for the sake of brevity, a symbol "Q" is used for representing any one of the fifteen parameters.

18. The test method and algorithm for the aging life according to claim 1, **characterized in that** the constants further comprise: each parameter "Q" in the micro-gasification expansion oscillation equation (1) contains three constants, which neither change with time nor with temperature, and only change with the chemical components of the target specimen; for the sake of brevity, the three letters "A, B and C" are used for representing the three constants under each parameter; when any of the physical and chemical electrical properties is evaluated or predicted, each parameter and its corresponding constants in the dynamic correlation equation (2) are replaced one by one;

$$\ln Q = \frac{A}{T+C} + B \dotsb \quad (2)$$

in the equation (2),

Q-any one of the fifteen parameters in the equation (1) at any temperature;
A-empirical constant associated with superposed reaction activation energy and diffusion activation energy of multiple components, a fitted value, K;
B-empirical constant associated with superposed chemical reaction rate and diffusion rate of multiple components, a fitted value, dimensionless;
C-conformal constant after Fourier series transformation associated with the activation energy of multiple components, a fitted value, K; and
T-absolute temperature, specified constant temperature +273.15, K.

19. The test method and algorithm for the aging life according to claim 1, **characterized in that** the parameter fitting comprises: the measured value (P) of the physical, chemical and electrical properties is used as a verification specimen; an electronic calculation program or a parallax method is utilized to perform respective increase or decrease with a step pitch as small as possible, the measured value is input into the equation (1), and the respective "Q" values of the fifteen different parameters are repeatedly iterated and cycled to output calculated values ($P_t$); when a standard deviation of a difference value between the calculated value ($P_t$) and the measured value (P) converges to the minimum, the "Q" values corresponding to the fifteen parameters are used as optimal values; due to a mathematical frequency doubling effect, if there is more than one optimal value among the fitted values of the fifteen parameters, only the group of fifteen smaller "Q" values closest to "1 time" is selected as the optimal parameters.

20. The test method and algorithm for the aging life according to claim 1, **characterized in that** the constant fitting comprises: different "C" values are tentatively input, and are repeatedly iterated in the equation (2), plotting is performed by using the logarithms of the "Q" values of the fifteen optimal parameters as vertical coordinates, and using $1/(T+C)$ as abscissas, the points are connected into a line, and when the line is close to a straight line, "A, B and C" become the optimal fitted values; or a least square method electronic calculation program or a parallax method is utilized to perform increase or decrease with a step pitch as small as possible, different "C" values are input and are repeatedly iterated in the equation (2), when $R^2$ output by a calculation program system is $\geq 0.990$, it is considered that the line has been a straight line; the "A, B and C" in one-to-one correspondence with the obtained fifteen parameters become the optimal constants, wherein the minimum boundary of the value "C" is -273.

# DRAWINGS

Fig. 1

Fig. 2

F

-F

Fig. 3

F

-F

Fig. 4

Fig. 5

Fig. 6

Fig. 7

Fig. 8

9a

9b

Fig. 9

Fig. 10

Fig. 11

13. 20

13. 21

13. 22

13. 23

Fig. 12

14. 10    4. 2    14. 20

-F

F

Fig. 13

14. 10    15. 1    13. 10    10

15. 4    15. 2    4. 2    15. 3    14. 2    13. 20    8

Fig. 14

16. 10

16. 11

16. 12

Fig. 15

16. 20

16. 22

16. 21

Fig. 16

16. 11    16. 12    13. 10    4. 2    22

16. 21        16. 22        13. 20

Fig. 17

Fig. 18

Fig. 19

Fig. 20

Fig. 21

Fig. 22

Fig. 23

Fig. 24

Fig. 25

Fig. 26

Fig. 27

Fig. 28

Fig. 29

Fig. 30

Fig. 31

Fig. 32

Fig. 33

Fig. 34

Fig. 35

Fig. 36

Fig. 37

Fig. 38

Fig. 39

Fig. 40

Fig. 41

Fig. 42

245C

Fig. 43

218C

Fig. 44

Fig. 45

Fig. 46

97C

Fig. 47

85C

Fig. 48

Fig. 49

Fig. 50

Fig. 51

Fig. 52

Fig. 53

Fig. 54

Fig. 55

Fig. 56

**Tensile strength**

Fig. 57

**Tensile strength**

Fig. 58

Fig. 59

Fig. 60

Fig. 61

Fig. 62

Fig. 63

Fig. 64

## Shear bonding strength

Fig. 65

## Shear bonding strength

Fig. 66

Fig. 67

Fig. 68

Fig. 69

Fig. 70

Fig. 71

Fig. 72

Fig. 73

Fig. 74

Fig. 75

Fig. 76

Fig. 77

Fig. 78

## 97C

Fig. 79

## 85C

Fig. 80

Natural logarithm of volume resistivity

Fig. 81

Natural logarithm of volume resistivity

Fig. 82

| | |
|---|---|
| **INTERNATIONAL SEARCH REPORT** | International application No. |
| | **PCT/CN2021/129423** |

**A. CLASSIFICATION OF SUBJECT MATTER**

G01N 25/00(2006.01)i;  G01N 3/32(2006.01)i;  G01N 3/54(2006.01)i;  G01N 3/18(2006.01)i;  G06F 30/20(2020.01)i;
G06F 119/04(2020.01)i

According to International Patent Classification (IPC) or to both national classification and IPC

**B. FIELDS SEARCHED**

Minimum documentation searched (classification system followed by classification symbols)

G01N25; G01N3; G01N33; G06F30; G06F119

Documentation searched other than minimum documentation to the extent that such documents are included in the fields searched

Electronic data base consulted during the international search (name of data base and, where practicable, search terms used)

CNTXT, ENTXTC, VEN, CNKI, ScienceDirect: 复合材料, 橡胶, 塑料, 老化, 寿命, 方程, 模型, 拟合, 参数, 振荡, 气化膨胀, compound, combined, complex, material, rubber, plastic, aging, life, equation, model, fitting, parameter, oscillation, gasification expansion

**C. DOCUMENTS CONSIDERED TO BE RELEVANT**

| Category* | Citation of document, with indication, where appropriate, of the relevant passages | Relevant to claim No. |
|---|---|---|
| PX | CN 113189130 A (SHENZHEN GLPOLY ELECTRONIC CO., LTD. et al.) 30 July 2021 (2021-07-30)<br>claims 1-20 | 1-20 |
| A | CN 111458225 A (TONGJI UNIVERSITY) 28 July 2020 (2020-07-28)<br>description, paragraphs 39-139 and figure 1 | 1-20 |
| A | CN 109490077 A (BEIJING INSTITUTE OF STRUCTURE & ENVIRONMENT ENGINEERING) 19 March 2019 (2019-03-19)<br>entire document | 1-20 |
| A | CN 106404531 A (CRRC QINGDAO SIFANG CO., LTD.) 15 February 2017 (2017-02-15)<br>entire document | 1-20 |
| A | WO 2018107730 A1 (HAIKOU INSTITUTE OF FUTURE TECHNOLOGY) 21 June 2018 (2018-06-21)<br>entire document | 1-20 |
| A | KR 20160050114 A (HYUNDAI MOBIS CO., LTD.) 11 May 2016 (2016-05-11)<br>entire document | 1-20 |

☐ Further documents are listed in the continuation of Box C.   ☑ See patent family annex.

| | |
|---|---|
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **07 February 2022** | **11 February 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

INTERNATIONAL SEARCH REPORT
Information on patent family members

International application No.

**PCT/CN2021/129423**

| Patent document cited in search report | | | Publication date (day/month/year) | Patent family member(s) | | | Publication date (day/month/year) |
|---|---|---|---|---|---|---|---|
| CN | 113189130 | A | 30 July 2021 | None | | | |
| CN | 111458225 | A | 28 July 2020 | CN | 111458225 | B | 03 September 2021 |
| CN | 109490077 | A | 19 March 2019 | None | | | |
| CN | 106404531 | A | 15 February 2017 | CN | 106404531 | B | 21 June 2019 |
| WO | 2018107730 | A1 | 21 June 2018 | CN | 108204925 | A | 26 June 2018 |
| | | | | CN | 108204925 | B | 20 March 2020 |
| KR | 20160050114 | A | 11 May 2016 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Propulsion Technology. *Chinese Astronautical Society (North Sea Fleet Command) Conference Paper,* September 1984, vol. 6 (2), 49-60